# EUROPEAN PATENT SPECIFICATION

(11) **EP 2 504 448 B1**
(45) Date of publication and mention of the grant of the patent: **19.10.2016**
(21) Application number: 10833991.2
(22) Date of filing: 25.11.2010
(51) Int. Cl.: C12P 19/34

(54) **METHODS AND COMPOSITIONS FOR DETECTING GENETIC MATERIAL**
VERFAHREN UND ZUSAMMENSETZUNG ZUR ERKENNUNG VON GENETISCHEM MATERIAL
PROCÉDÉS ET COMPOSITIONS DESTINÉS À DÉTECTER UN MATÉRIEL GÉNÉTIQUE

(30) Priority: 02.03.2010 US 309837 P; 25.11.2009 US 264591 P; 02.03.2010 US 309845 P; 25.03.2010 US 317635 P; 25.03.2010 US 317639 P; 25.03.2010 US 317684 P; 25.03.2010 US 341065 P; 25.03.2010 US 341218 P; 08.09.2010 US 380981 P; 01.11.2010 US 409106 P; 02.11.2010 US 409473 P; 05.11.2010 US 410769 P
(43) Date of publication of application: 03.10.2012
(73) Proprietor: Bio-Rad Laboratories, Inc., Hercules, CA 94547 (US)
(72) Inventor: HINDSON, Benjamin, Livermore CA 94551 (US); SAXONOV, Serge, San Mateo CA 94402 (US); BELGRADER, Philip, Sevena Park MD 21146 (US); NESS, Kevin, Pleasanton, CA 94566 (US); LUCERO, Michael, South San Francisco CA 94080 (US); COLSTON, Billy, San Ramon CA 94583 (US)
(74) Representative: Cole, William Gwyn
(86) International application number: PCT/US2010/058124
(87) International publication number: WO 2011/066476

(56) References cited:
- WO-A2-2007/092473
- US-A1- 2007 172 873
- US-A1- 2007 172 873
- US-A1- 2008 014 589
- FAN H C ET AL: "Microfluidic digital PCR enables rapid prenatal diagnosis of fetal aneuploidy", AMERICAN JOURNAL OF OBSTETRICS & GYNECOLOGY, MOSBY, ST LOUIS, MO, US, vol. 200, no. 5, 1 May 2009 (2009-05-01), pages 543.e1-543.e7, XP026056021, ISSN: 0002-9378, DOI: 10.1016/J.AJOG.2009.03.002 [retrieved on 2009-04-16]
- LINAS MAZUTIS ET AL: "Droplet-Based Microfluidic Systems for High-Throughput Single DNA Molecule Isothermal Amplification and Analysis", ANALYTICAL CHEMISTRY, AMERICAN CHEMICAL SOCIETY, US, vol. 81, no. 12, 15 June 2009 (2009-06-15) , pages 4813-4821, XP008148031, ISSN: 0003-2700, DOI: 10.1021/AC900403Z [retrieved on 2009-05-18]
- WANG ET AL.: 'Analysis of molecular inversion probe performance for allele copy number determination.' GENOME BIOL vol. 8, no. 11, 2007, page R246, XP021041512

## Description

### BACKGROUND OF THE INVENTION

Fetal aneuploidies are aberrations in chromosome number and commonly arise as a result of a meiotic nondisjunction during oogenesis or spermatogenesis; however, certain aneuploidies, such as trisomy 8, result more often from postzygotic mitotic disjunction (Nicolaidis & Petersen (1998) Human Reproduction 13:313-319). Such aberrations include both reductions and increases in the normal chromosome number and can involve autosomes as well as the sex chromosomes. An example of a reduction aneuploidy is Turner's syndrome, which is typified by the presence of a single X sex chromosome. Examples of increases in chromosome number include Down's syndrome (trisomy of chromosome 21), Patau syndrome (trisomy of chromosome 13), Edwards syndrome (trisomy of chromosome 18), and Kleinfelter's syndrome (an XXY trisomy of the sex chromosomes). Aneuploidies commonly lead to significant physical and neurological impairments which result in a large percentage of affected individuals failing to reach adulthood. In fact, fetuses having an autosomal aneuploidy involving a chromosome other than 13, 18, or 21 generally die in utero. However, certain aneuploidies, such as Kleinfelter's syndrome, present far less pronounced phenotypes and those affected with other trisomies, such as XXY & XXX, often will mature to be fertile adults. In some cases, partial aneuploidy resulting in an abnormal copy number of a portion of a chromosome may result from an imbalanced nondisjunction.

Prenatal diagnosis of fetal aneuploidies using invasive testing by amniocentesis or Chorionic Villus Sampling (CVS), are associated with a 0.5% to 2% procedure-related risk of pregnancy loss (D'Alton, M. E., (1994) Semin Perinatol 18:140-62; Caughey AB (2006) Obstet Gynecol 108:612-6).

Another barrier to accurately screening fetal aneuploidy is the low concentration of fetal DNA in maternal plasma, particularly at earlier gestational ages. Single or low multiplex assay approaches are unlikely to provide enough target counts to differentiate between an aneupoloid fetus (e.g., trisomy of chromosome 21) from a euploid fetus. There is also, generally, a need in the art for methods and compositions for detecting copy number variations in biological samples, not necessarily from maternal blood.

Fan, H. C. et al. (Microfluidic digital PCR enables rapid prenatal diagnosis of fetal aneuploidy, American Journal of Obstetrics and Gynecology, 2009, 200, pp543e1-543e7) describes a method of detecting the copy number of a target polynucleotide within a population of genetic material. The method comprises the steps of isolating the target nucleic acids (chromosomes), partitioning the sample such that of three diluted samples only one contains a target nucleic acid, PCR amplifying a marker on the said target nucleic acid, detecting amplification using a probe, calculating the copy number of each target in all diluted samples and from this calculating the copy number of each target in the sample at the outset of the experiment.

U.S. Patent Application Publication US2007/0172873 describes a digital PCR method wherein the target nucleic acid is fragmented followed by ligation of adaptors to each 5' and 3' end of each fragment. The fragments are then diluted, amplified and detected according to the principles of digital PCR.

Mazutis, L. et al. (Droplet-Based Microfluidic Systems for High-Throughput Single DNA Molecule Isothermal Amplification and Analysis. Analytical Chemistry, 2009, 81(12) pp4813-4821) describes an alternative digital PCR method involving partitioning of target nucleic acid by dilution followed by amplification by a "hyperbranched rolling circle amplification" reaction (HRCA). In this reaction, amplification proceeds by extension of random primers using polymerase having strand displacement activity thereby generating a large number of detectable nucleic acids.

### SUMMARY OF THE INVENTION

The scope of the present invention is defined in the appended claims and any subject-matter provided below that does not fall within the scope of the claims is provided for information purposes only.

The present disclosure provides methods and compositions for detecting copy number of a target polynucleotide within a population of genetic material. Partitioning may be used to subdivide the target polynucleotide into a plurality of reaction volumes. In some cases, a probe to the target polynucleotide is subdivided into a plurality of reaction volumes.

In some cases, the methods comprise the following steps: a. binding a first ligation probe to a first target polynucleotide; b. binding a second ligation probe to a second target polynucleotide; c. subjecting said first and second ligation probes to a ligation reaction in order to obtain one or more ligated products; d. partitioning said one or more ligated products into two or more partitions; e. amplifying a sequence within said one or more ligated products to obtain amplified products; f. determining a number of said partitions that contain said amplified products; and g. calculating a copy number of said first target polynucleotide. In some cases, the target polynucleotide is not partitioned into said two or more partitions.

Partitions can include a wide variety of types of partitions, including solid partitions (e.g., wells, tubes, etc.) and fluid partitions (e.g., aqueous droplets within an oil phase, such as a continuous oil phase, or aqueous droplets within a mixture of at least two immiscible fluids). The partitions may also be stable or unstable. For example, in some cases, during the amplification process said two or more partitions remain substantially intact. In some cases, the partitions are aqueous droplets within an oil phase and said aqueous droplets remain substantially intact during the amplification reaction of the instant methods. The partitions (e.g., said aqueous droplets) may also remain substantially intact during the determination steps, when partitions are evaluated for the presence of one or more target polynucleotides (or probes to said polynucleotides). The partitions may comprise an amplification reaction that is initiated from said ligated product.

The first and second ligation probes may bind (or be designed to bind) a variety of target polynucleotides; often a first ligation probe binds a first target polynucleotide and a second ligation probe binds a second polynucleotide. In some cases, the first and second ligation probes are each designed to bind to said first target polynucleotide. In other cases, said first ligation probe binds a first target polynucleotide that has a sequence that differs from the sequence of said second target poynucleotide. In some cases, first ligation probe is designed to bind to a polynucleotide sequence that is conserved between individuals within a species. In some cases, first ligation probe is designed to bind to a polynucleotide sequence that is conserved across two or more different species. In some cases, a ligation probe binds to a nonpolymorphic region of a chromosome.

The methods may comprise ligating multiple ligation probes to said first target polynucleotide. For example, the method may comprise binding at least four ligation probes to said first target polynucleotide. In other cases, at least 1, 2, 3, 4, 5, 6, 7, 8, 9, 10, 15, 20, 25, 30, 35, 40, 45, 50, 100, 200, 500, 1000, 5000, 10,000, 20,000, 30,000, 40,000, 50,000, 60,000, 70,000, 100,000, 2,000,000, 3, 000,000, 4,000,000, 5,000,000, 6,000,000, 7,000,000, 8,000,000, 9,000,000 or 10,000,000 ligation probes are used in the methods provided herein. Often, one or more of said ligation probes bind to a different polynucleotide (e.g., different chromosomes, different regions within the same chromosome). In some cases, a plurality of first ligation probes (e.g., target ligation probes) are used and a plurality of second ligation probes (e.g., reference ligation probes) are used in the present methods and compositions. The methods may further comprise binding at least four ligation probes to said first target polynucleotide and at least four ligation probes to said second target polynucleotide. In some cases, at least 1, 2, 3, 4, 5, 6, 7, 8, 9, 10, 15, 20, 25, 30, 35, 40, 45, 50, 100, 200, 500, 1000, 5000, 10,000, 20,000, 30,000, 40,000, 50,000, 60,000, 70,000, 100,000, 2,000,000, 3, 000,000, 4,000,000, 5,000,000, 6,000,000, 7,000,000, 8,000,000, 9,000,000 or 10,000,000 ligation probes are bound to said first or said second target polynucleotide.

The first ligation probe may bind (or be designed to bind) to a first region within said first target polynucleotide and said second ligation probe may bind (or be designed to bind) to a second region within said first target polynucleotide, wherein said first and second regions do not have identical sequences.

Often the first target polynucleotide is not identical to said second target polynucleotide. In some cases the first target polynucleotide is identical to the second target polynucleotide. In some examples, said first target polynucleotide is a test chromosome and said second target polynucleotide is a reference chromosome. Examples of test chromosomes include but are not limited to: chromosome 21, chromosome 13, chromosome 18, and the X chromosome. Said test chromosome may also be from the group consisting of chromosome 1, 2, 3, 4, 5, 6, 7, 8, 9, 10, 11, 12, 13, 14, 15, 16, 17, 18, 19, 20, 21, 22, X, and Y. A first target polynucleotide may be a segment of a chromosome, such as a segment of a chromosome that is associated with fetal aneuploidy (either the chromosome or the segment may be associated with fetal aneuploidy).

The methods and compositions provided herein often relate to ligating a probe to itself, ligating two probes together, and/or ligation products of said ligation reactions. Said ligation reactions may result in the ligation of a 5' region of said first ligation probe to a 3' region of said first ligation probe to obtain a circular ligated product. In some cases, a ligation reaction results in the ligation of the 5' region of said first ligation probe to the 3' region of said second ligation probe, in order to obtain a linear ligated product comprising at least a portion of said first and second ligation probes. Said 5' region and said 3' region of said first ligation probe may each bind (or be designed to bind) adjacent sequences within said first target polynucleotide. Said adjacent sequences are separated by 0 nucleotides. Said 5' region and said 3' region of said first ligation probe may bind, or be designed to bind, neighboring sequences within said first target polynucleotide. Said neighboring sequences may be separated by at least one nucleotide. In some cases, the neighboring sequences are separated by a gap of at least 5, 10, 20, 30, 40 ,50, 100, 200, 300, 400, or 500 nucleotides.

The ligation reaction may further comprise a template-driven gap fill reaction to incorporate nucleotides in the gap between said 5' region and said 3' region of said first ligation probe (or of said second ligation probe).

The ligation probes may comprise a site cleavable by an enzyme. For example, the site cleavable by an enzyme may comprise one or more uracils. The uracils may be separated by other nucleotides in some cases. The site cleavable by an enzyme may comprise a restriction site. The first ligation probe may be of a specific type, such as a molecular inversion probe, a padlock probe, a linear ligation probe, etc..

The methods provided herein may further comprise performing an enzymatic reaction to remove linear polynucleotides or single-stranded polynucleotides or double-stranded polynucleotides. For example, an exonuclease (e.g., Exo I, II, and/or III) may be used in the methods described herein. Often, exonuclease treatment removes all, or a substantial amount, of unbound ligation probes from a sample volume.

The probes provided herein may be conjugated to signaling agent. Said first ligation probe may be conjugated to a first signaling agent and a second ligation probe is conjugated a second signaling agent. Often, a plurality of such first and second ligation probes are used in the methods and compositions herein, wherein said probes are conjugated to the same signaling agent (e.g., identical fluorophore) or to different signaling agents (e.g., fluorophores of different colors). Said first signaling agent may be a fluorescent marker of a first color and said second signaling agent may be a fluorescent marker of a second color.

Detection of ligation probes is also often a step in the methods provided herein. The methods may comprise detecting said first ligation probe with a first signaling agent and detecting said second ligation probe with a second signaling agent. Said first ligation probe may comprise a first plurality of ligation probes, wherein each probe within said plurality is directed to a different region of a first chromosome, and wherein said second ligation probe comprises a second plurality of ligation probes, wherein each probe within said plurality is directed to a different region of a second chromosome. In some cases, said first target polynucleotide is a test chromosome and said second target polynucleotide is a reference chromosome. In some cases, said first and second ligation probes are conjugated to the same color.

The methods and compositions provided herein may also involve a method of detecting copy number of a target polynucleotide within a population of genetic material comprising: a. binding a first ligation probe to a first target polynucleotide; b. binding a second ligation probe to a second target polynucleotide; c. subjecting said first and second ligation probes to a ligation reaction in order to obtain one or more ligated products; d. partitioning said one or more ligated products into two or more aqueous droplets within a continuous oil phase; amplifying a sequence within said one or more ligated products to obtain amplified products; determining a number of said two or more aqueous droplets that contain said amplified products; and g. calculating a copy number of said target polynucleotide based on said number. In some cases, said target polynucleotide is not partitioned into said two or more aqueous droplets. In some cases, said target polynucleotide is not amplified. In some cases, or during said amplifying or determining steps, said two or more aqueous droplets remain substantially intact.

In some cases, said two or more aqueous droplets comprise on average more than one ligated probe and said method further comprises using an algorithm to calculate an average number of target ligated probes per aqueous droplet. Said two or more aqueous droplets may be greater than 4,000 droplets. In some cases, said two or more aqueous droplets may be greater than 1,000, 10,000, 20,000, 50,000, 100,000, 200,000, 500,000, 1,000,000, or 5,000,000 droplets.

In some cases, said droplets are present in a single chamber at a high droplet/ml density. The density may be greater than 100,000 aqueous droplets/ml. Examples of densities of droplets in a single chamber include: 10,000 droplets/mL, 100,000droplets/mL, 200,000droplets/mL, 300,000droplets/mL, 400,000droplets/mL, 500,000droplets/mL, 600,000droplets/mL, 700,000droplets/mL, 800,000droplets/mL, 900,000droplets/mL or 1,000,000droplets/mL. The droplets used in any of the methods or compositions provided herein may be monodisperse droplets. The droplets may have, on average, a diameter of between 50 nm and 300 µm. The droplet diameter may be, on average, about .001, .01, .05, .1, 1, 5, 10, 20, 30, 40, 50, 60, 70, 80, 100, 120, 130, 140, 150, 160, 180, 200, 300, 400, or 500 microns In some cases, the droplets do not comprise a substantial number of beads conjugated to oligonucleotides.

The aqueous droplets may be present within an oil fluid or phase. The oil phase may comprise an anionic flourosurfactant an ammonium salt of an anionic fluorosurfactant, such as KrytoxTM. Krytox may be selected from a group consisting of Krytox AS, Krytox FSH, and morpholino derivative of Krytox FSH. The oil phase may comprise a fluorinated oil.

The methods provided herein (e.g., detecting copy number using droplets) can be used to detect said first target polynucleotide within a population of genetic material comprising less than 1,000 copies of said first target polynucleotide. In some cases, said two or more aqueous droplets comprise on average more than one ligated probe and said method further comprises using an algorithm to calculate an average number of target ligated probes per aqueous droplet.

The droplets may comprise a first target polynucleotide that is a chromosomal segment associated with a genetic disorder. The droplets may comprise a specific type of ligation probe (e.g., padlock probe, molecular inversion probe, ligation detection reaction (LDR) probe, etc.). The ligation probe may be subjected to a ligation reaction that ligates the 5' region of the ligation probe to the 3' region of the ligation probe.

The methods and compositions provided herein may also relate to a method of detecting a fetal genetic condition comprising: a. obtaining a mixture of maternal and fetal genetic material comprising target polynucleotides; b. combining said mixture with targeting oligonucleotides that bind said target polynucleotides;c. subdividing said targeting oligonucleotides into reaction volumes, wherein at least one of said reaction volumes comprises no target polynucleotide and no targeting oligonucleotide; d. performing an amplification reaction within said reaction volumes; e. detecting the presence of said target polynucleotide or said targeting oligonucleotide within said reaction volumes; and
f. determining the relative level of said target polynucleotide in said mixture in order to detect a fetal genetic condition.

The reaction volumes may be aqueous droplets within a continuous oil phase. The targeting oligonucleotides may comprise one or more primer pairs; ligation probes; molecular inversion probes; ligation detection reaction (LDR) probes; padlock probes; and any combination thereof. The reaction volumes may comprise, on average, greater than one copy of targeting oligonucleotid, and/or, on average, greater than one copy of target polynucleotide. Said reaction volumes may further comprise primers to a reference polynucleotide. In some cases, said reaction volumes further comprise a ligation probe to a reference polynucleotide.

The ligation probes may be amplified within said reaction volumes.

The fetal genetic material used in the methods for detecting a fetal genetic condition may be derived from a cellular sample that was selectively pre-enriched for fetal genetic material. But, fetal genetic material used in the methods for detecting a fetal genetic condition is not derived from a cellular sample that was selectively preenriched for fetal genetic material

The target polynucleotide may be within a chromosome selected from the group consisting of chromosome 18, 13, 21, and X; or from the group consisting of chromosome 1, 2, 3, 4, 5, 6, 7, 8, 9, 10,11, 12, 13, 14, 15, 16, 17, 18, 19,20, 21, 22, X, or Y.

In some cases, said reaction volumes are aqueous droplets within an oil phase, said targeting oligonucleotides are ligation probes, and said determining step comprises comparing a number of droplets comprising an amplified product of said ligation probes with a number of droplets comprising an amplified product of ligation probes directed to a reference polynucleotide. In some cases, said reference polynucleotide is a region of a chromosome that is not associated with a fetal genetic abnormality.

As used in the methods and compositions provided herein, said targeting oligonucleotides may be ligation probes that become circular upon ligation following hybridization to a target polynucleotide.

This disclosure also provides compositions, such as microcapsule compositions, as well as methods for using said microcapsule compositions. In some cases, the composition is a microcapsule comprising a ligated probe wherein said microcapsule is obtained by: a. selectively binding a plurality of ligation probes to target polynucleotides within a genetic sample; b. ligating a 5' end of at least one of said bound ligation probes to a 3' end of the same or different bound ligation probe, thereby obtaining at least one ligation product; c. introducing an aqueous solution comprising said at least one ligation product into a device for generating droplets; d. using said device to produce an aqueous droplet comprising said at least one ligation product, wherein said aqueous droplet is within an immiscible fluid; and
e. converting said droplet into a microcapsule comprising a solid-phase exterior. In some cases, said converting comprises heating above 50 ° C, or heating above 70 ° C. The immiscible liquid (e.g., oil) may comprise a fluorinated surfactant. In some cases, the aqueous phase comprises a fluorinated surfactant. The oil may be a fluorocarbon oil. The oil phase may comprise an anionic surfactant. The oil phase may comprises ammonium Krytox. In some cases, said microcapsule does not comprise a bead bound to an oligonucleotide. Said microcapsule may remain substantially intact at temperatures above 70° C. The microcapsule may comprise ligation probes capable of selectively binding to a target polynucleotide associated with a genetic disorder. In some cases, said ligation probes are capable of selectively binding to a target polynucleotide associated with fetal aneuploidy. In some cases, said genetic target is within a chromosome selected from the group consisting of chromosome 21, chromosome 13, chromosome 18, and the X chromosome. In some cases, the genetic target is within chromosome 1, 2, 3, 4, 5, 6, 7, 8, 9, 10,11, 12, 13, 14, 15, 16, 17, 18, 19,20, 21, 22, X, or Y.

The microcapsule may comprise one or more of said ligation probes (e.g., padlock probe, molecular inversion probe, ligation detection reaction (LDR) probe, circular probe, etc.). The microcapsules may comprise a linear probe obtained by linearizing a probe previously circularized after a ligation reaction. In some cases, the microcapsules comprise circularized ligation probe. In some cases, the microcapsules contain linear products of a ligation detection reaction (LDR).

The compositions and method provided herein may also relate to a water-in-oil mixture comprising two or more aqueous droplets, wherein at least one of said two or more aqueous droplets comprises a first ligation probe directed to a first target polynucleotide and at least one of said two or more aqueous droplets comprises a second ligation probe directed to a second target polynucleotide. Said first target polynucleotide and said second target polynucleotide may be the same molecule, or different molecules with identical sequences or structures, or different molecules with different sequences or structures. In some cases, said first target polynucleotide has a different sequence than that of said second target polynucleotide. In some instances, said first target polynucleotide has an identical sequence to said second target polynucleotide. Said first target polynucleotide may comprise a first region within a genomic segment and said second target polynucleotide may comprise a second region within said genomic segment, wherein said first region does not have the same sequence as said second region.

In some cases, said water-in-oil mixture further comprises an ammonium krytox surfactant. Said krytox surfactant may be present in the oil phase of said mixture at a concentration of at least 0.01 %.

In some cases, said mixture comprises a ligation probe that is the linearized product of a circular probe that was subjected to enzymatic cleavage. In some cases, said mixture comprises the circularized probe itself. In some cases, the ligation probe may comprise an enzymatic cleavage site, such as where enzymatic cleavage is catalyzed by uracil-N-glycosylase or a restriction enzyme.

The present disclosure includes a method of differential detection of target sequences in a mixture of maternal and fetal genetic material, comprising the steps of: a) obtaining maternal tissue containing both maternal and fetal genetic material; b) distributing the genetic material into discrete samples, each sample containing on average not more than about one target sequence per sample, wherein the discrete sample contains a set of primers to a known target sequence and/or a set of reference primers to a known reference sequence; c) performing an amplification reaction; d) detecting the presence of the target or reference sequence in the discrete samples; and e) comparing the ratio of target sequences detected to reference sequences detected to determine a differential amount of target sequence. Said method may further comprise a step of comparing the ratio of target sequences detected to reference sequences detected to determine a differential amount of target sequence, wherein a difference in target sequences detected to reference sequences detected indicates a fetal genetic abnormality. The method may be a method of detecting fetal aneuploidy. In some cases, the target sequence is a marker for aneuploidy and the reference sequence is diploid in maternal and fetal genetic material. The maternal tissue may be maternal peripheral blood, blood plasma or serum, or other tissue described herein. The reaction samples may be in aqueous phases in an emulsion. In some cases, detecting the presence of the target or reference sequence further includes hybridizing it in situ with a nucleic acid having a fluorescent label. In some cases, the number of reaction samples is at least about 10,000. In some cases, steps b) to e) are repeated with a primer set to a different target sequence. In some cases, the reaction volume comprises more than one primer set with each primer set to a particular target sequence. In some cases, the reaction volume comprises more than one reference primer set with each primer set to a particular reference sequence. Examples of primer sets that can be used include primer sets specific for human chromosome 21, human chromosome 18, human chromosome 13, or human chromosome X. In some cases, aneuploidy is detected where the ratio of target to reference sequence detected is greater than 1. In some cases, an aneuploidy is detected where the ratio of target to reference sequence detected is less than 1. In some cases, the target sequence is at least a portion of a CFTR, Factor VIII (F8 gene), beta globin, hemachromatosis, G6PD, neurofibromatosis, GAPDH, beta amyloid, or pyruvate kinase gene.

### BRIEF DESCRIPTION OF THE FIGURES

FIG. 1 is a schematic overview illustrating the steps that can be taken to detect copy number variations in a patient sample through the use of droplet digital PCR and ligation probes.
FIG. 2 is a schematic illustration of an example of steps that can be followed to detect changes in the number of chromosomes (or portions thereof) in a sample.
FIG. 3 depicts a workflow of an exemplary method for diagnosing fetal aneuploidy.
FIG. 4 is a schematic illustration of the use of Molecular Inversion Probes (MIPs) to detect two genetic targets.
FIG. 5 shows multiplexing of the MIP approach to increase sensitivity of detection of genetic targets.
FIG. 6 shows a two-color system for detection of nucleic acids in droplets using universal primers and universal probes without cleavage.
FIG. 7 shows a scheme for detecting two genetic targets with two colors using a ligation-detection reaction (LDR) followed by PCR in droplets.
FIG. 8 depicts the use of multiplexed oligonucleotides for LDR-PCR in droplets to enhance sensitivity of detection.
FIG. 9 depicts a computer useful for displaying, storing, retrieving, or calculating data or results obtained by the methods and compositions described herein.
FIG. 10 shows a correlation between number of input copies of template DNA and number of positive droplets (or counts), and increased sensitivity when using 12-plex MIPs (lower panels) compared to 3-plex MIPs (upper panels), for a test sample.
FIG. 11 shows number of positive droplets (or counts) versus number of template copies, for a reference sample.
FIG. 12 shows hybridization efficiency for different template copy numbers and at different levels of MIP multiplexing.

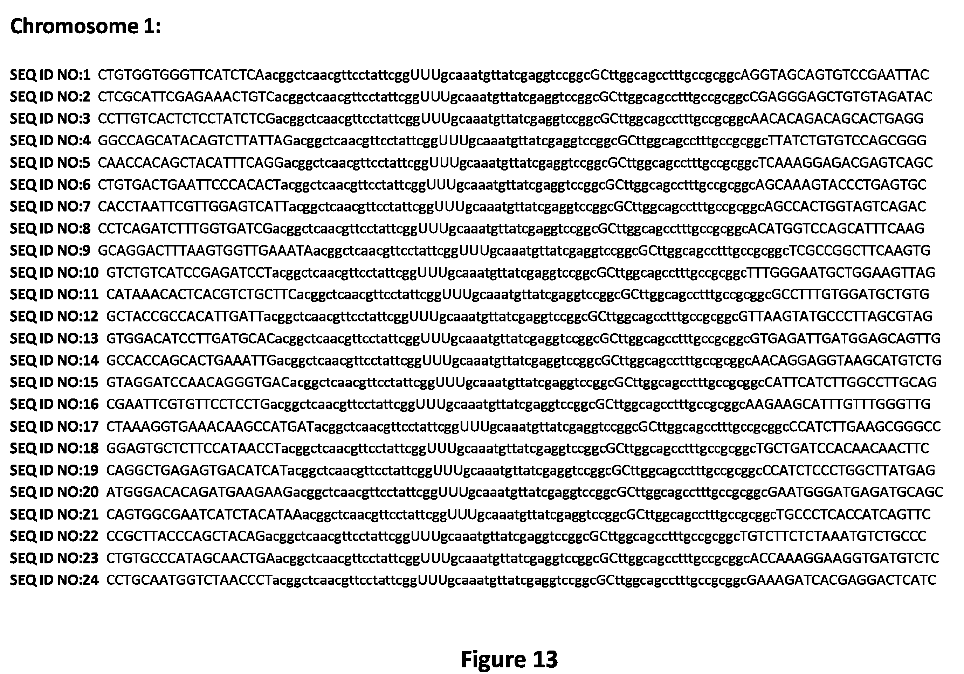

FIG. 13 shows 24 MIPs directed to different regions within Chromosome 1 (SEQ ID NOS: 1-24).

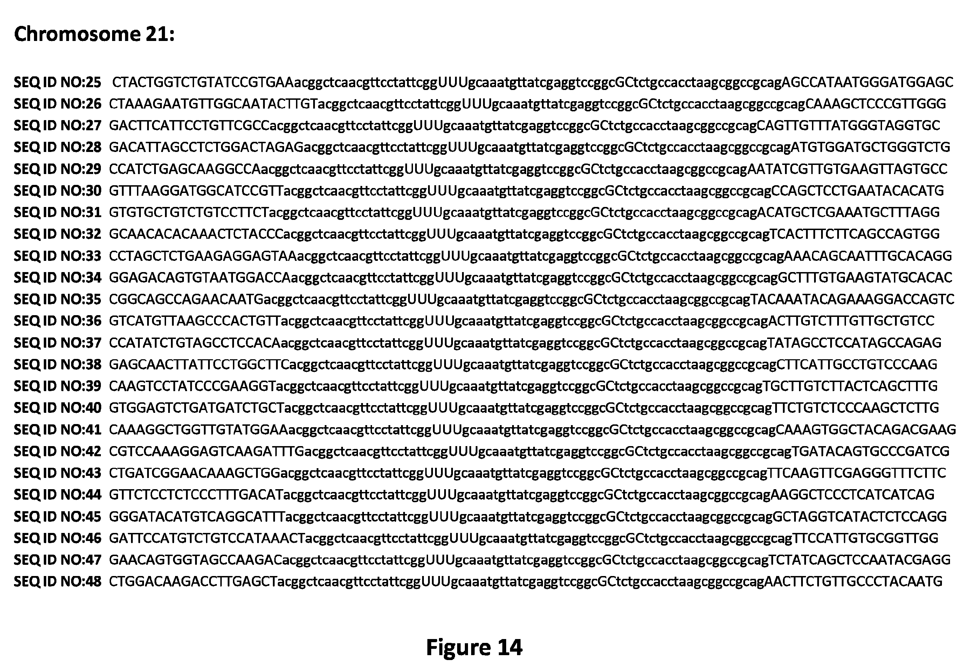

FIG. 14 shows 24 MIPs directed to different regions within Chromosome 21 (SEQ ID NOS: 25-48).

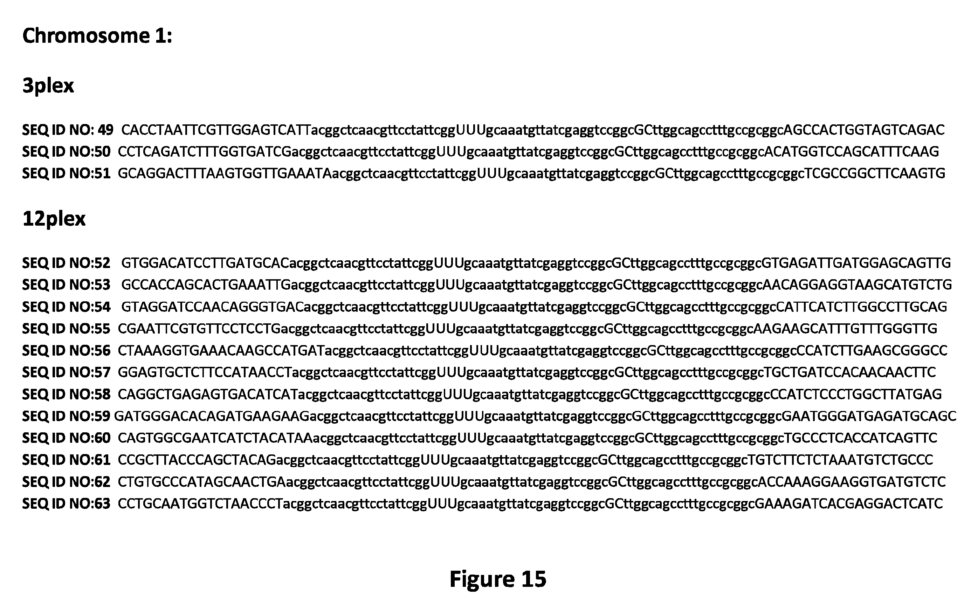

FIG. 15 shows a 3-plex set of MIPs directed to different regions within Chromosome 1 (SEQ ID NOS: 49-51) and a 12-plex set of MIPs directed to different regions within Chromosome 1 (SEQ ID NOS: 52-63).

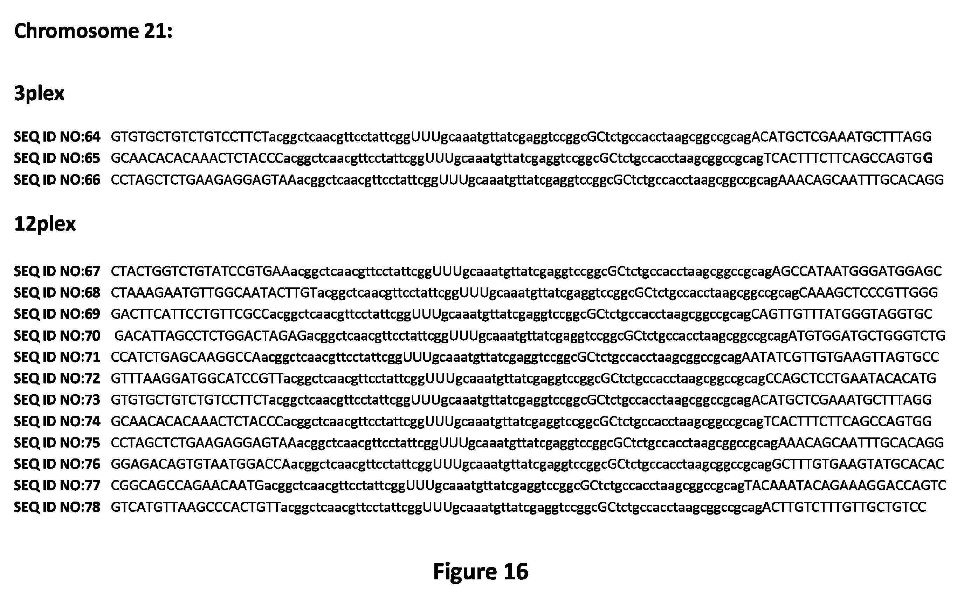

FIG. 16 shows a 3-plex set of MIPs directed to different regions within Chromosome 21 (SEQ ID NOS: 64-66) and a 12-plex set of MIPs directed to different regions within Chromosome 21 (SEQ ID NOS: 67-78).
FIG. 17 shows exemplary universal primers and probes for the detection of a MIP (SEQ ID NOS: 79-82).

### DETAILED DESCRIPTION OF THE INVENTION

### General Overview

As noted above, the scope of the present invention is defined in the appended claims and any subject-matter or specific embodiment described below that does not fall within the scope of the claims is provided for information purposes only.

This disclosure provides methods and compositions for detecting genetic variations in a biological sample. In some cases, this disclosure provides methods and compositions for detecting the number of copies of a target polynucleotide (e.g., chromosome, chromosome fragment, gene, etc.) within a biological sample. In some cases, methods and compositions for detecting genetic mutations and/or single nucleotide polymorphisms (SNPs) within a biological sample are also provided.

This disclosure also provides compositions and methods for detecting fetal aneuploidy, or other genetic abnormality, in a biological sample derived from maternal tissue. Often such a biological sample comprises a mixture of maternal and fetal nucleic acids (e.g., DNA, RNA). Aneuploidy is a chromosomal abnormality, and refers to an aberration in the copy number of a chromosome, or fragment thereof, or portion thereof. The methods and materials described herein apply techniques for analyzing numerous nucleic acids contained in a tissue sample, such as blood (whole blood or peripheral blood), serum or plasma, containing a mixture of DNA (and/or DNA fragments) from both the mother and the fetus, and allowing detection of small differences between target and reference DNA levels that may indicate fetal aneuploidy.

As used herein, copy number variations (CNVs) refer to gains or losses of segments of genetic material. There are large numbers of CNV regions in humans and a broad range of genetic diversity among the general population. CNVs also play a role in many human genetic disorders. The method is especially useful for detection of a translocation, addition, amplification, transversion, inversion, aneuploidy, polyploidy, monosomy, trisomy), trisomy 21, trisomy 13, trisomy 14, trisomy 15, trisomy 16, trisomy 18, trisomy 22, triploidy, tetraploidy, and sex chromosome abnormalities including but not limited to XO, XXY, XYY, and XXX. The method also provides a non-invasive technique for determining the sequence of fetal DNA and identifying mutations within the fetal DNA.

This disclosure provides means for the detection of CNV, genetic variations, and/or fetal aneuploidy, for example, by the use of digital PCR (e.g., droplet digital PCR), as well as specialized probes, often referred to herein as ligation probes (e.g., molecular inversion probes and other probes) capable of being ligated together directly or indirectly when hybridized to a target polynucleotide. In the methods provided herein, a sample comprising a target nucleotide, or probes to said target nucleotide is partitioned into a plurality of compartments (e.g., droplets). The compartments (e.g., droplets) are then subjected to a thermocyling reaction to encourage PCR reactions within compartments that contain either a target nucleotide, or a probe to said target nucleotide, resulting in amplified products (e.g., amplified DNA, RNA or other nucleic acid).

In some embodiments, a single probe is ligated at its ends following hybridization to a target polynucleotide (e.g., molecular inversion probe). In other embodiments, the ligation probes comprise two separate molecules that can be ligated together following hybridization to a target polynucleotide.

The compositions described herein include compositions comprising mixtures of two or more immiscible fluids such as oil and water that contain a type of nucleic acid probe (e.g., molecular inversion probe, ligation probe, etc.). In other cases, the compositions described herein comprise microcapsules that contain a type of nucleic acid probe (e.g., molecular inversion probe, ligation probe, etc.). Such microcapsules may resist coalescence, particularly at high temperatures, and therefore enable amplification reactions to occur at a very high density (e.g., number of reactions per unit volume).

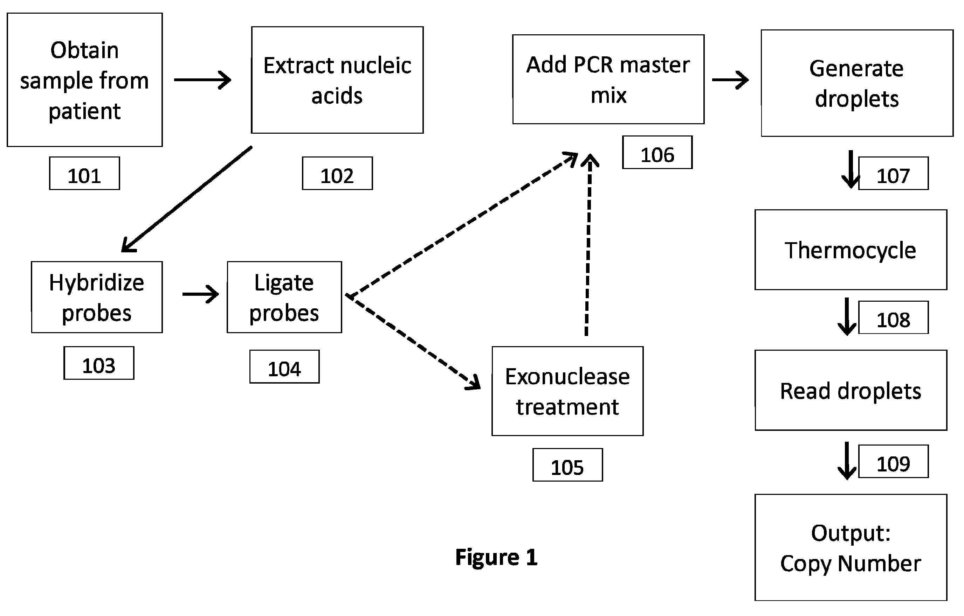

FIG 1 provides a schematic overview illustrating the steps that can be taken to detect copy number variations in a sample from a patient through the use of droplet digital PCR (ddPCR) and ligation probes. A sample of genomic nucleic acids (e.g., genomic DNA or RNA) is extracted (101) from a sample obtained from a patient (101). Probes (such as the ligation probes described herein) are allowed to hybridize to a target nucleotide sequence within the patient sample (103); following hybridization, the probes are ligated together (104) and then the sample is, optionally, subjected to an enzymatic treatment (e.g., exonuclease) to breakdown genomic nucleic acids and residual unligated probes (105). PCR reaction components (e.g., primers, fluorescence detection probes, polymerase, dNTPs, etc.) are then added to the sample (106), which is then partitioned into multiple droplets (107). After droplet formation, the droplets are subjected to thermocycling to amplify the probes within the sample (108). The number of positive and negative droplets are then determined (109), which is used to determine relative copy number of a target polynucleotide. Although Figure 1 depicts droplets as being the means of partitions, other means of partitioning known in the art can be used as well, e.g., partitioning among wells within a nano- or Microfluidic device, etc. Also, although Figure 1 depicts detecting copy number variations, other genetic conditions can be detected as well.

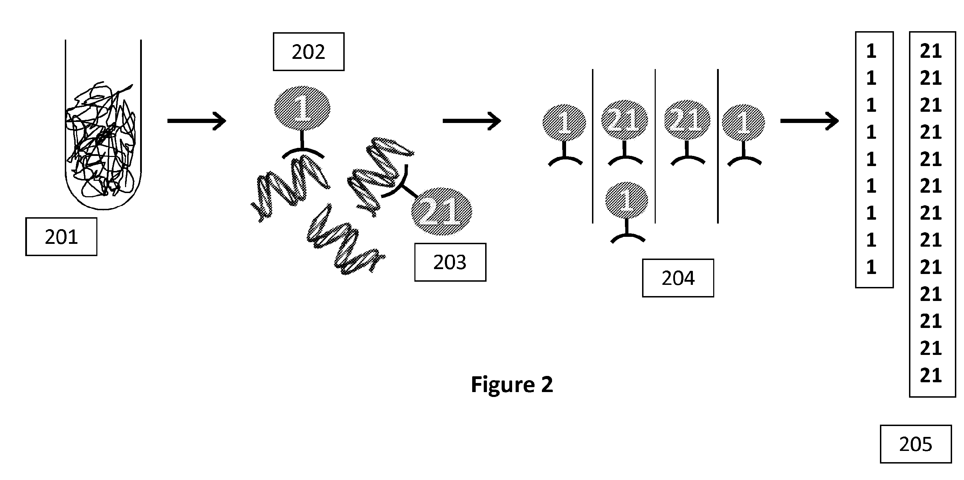

The detection of copy number within a sample may involve the detection of chromosomal abnormalities, including aneuploidy. FIG. 2 is a general overview of steps that can be taken to identify fetal aneuploidy in a maternal sample. A starting tissue sample (201) contains a mixture of maternal and fetal DNA. The DNA is extracted, and mixed with probes for chromosome 1 (202) (a reference chromosome) and chromosome 21 (a test chromosome) (203). Probes are bound to a genetic target and then partitioned into multiple compartments (204). Probes are detected within the compartments, and the number of compartments containing the test chromosome (e.g., chr. 21) is compared to the number of compartments containing the reference chromosome (e.g., chr. 1)(205), followed by calculation of the relative copy number of chromosome 21.

The present disclosure provides for the analysis of maternal tissue (e.g., blood, serum or plasma) for a genetic condition, wherein the mixed fetal and maternal DNA in the maternal tissue is analyzed to distinguish a fetal mutation or genetic abnormality from the background of the maternal DNA. Using a combination of steps, a DNA sample containing DNA (or RNA) from a mother and a fetus can be analyzed to measure relative concentrations of cell-free, peripherally circulating DNA sequences. Such concentration differences can be used to distinguish a genetic condition present in a minor fraction of the DNA, which represents the fetal DNA.

The method may employ digital analysis, in which the DNA in the sample is translated into a plurality of ligated probes that are partitioned to a nominal single ligated probe molecule in a reaction volume to create a sample mixture. For example, the reaction volume can be a droplet, such as a droplet of an aqueous phase dispersed in an immiscible liquid, such as described in U.S. Patent No. 7,041,481. Each reaction volume has a possibility of having distributed in it less than 1 target (e.g., target polynucleotide, targeting probe, or other target molecule) or one or more targets (e.g., target polynucleotide, targeting probe or other targeting molecule). The target molecules can be detected in each reaction volume, preferably as target sequences which are amplified, which can include a quantization (or quantification) of starting copy number of the target sequence, that is, 0, 1, 2, 3, etc. A reference sequence can be used to distinguish an abnormal increase in the target sequence, e.g., a trisomy. Thus there can be a differential detection of target sequence to reference sequence that indicates the presence of a fetal aneuploidy. It is not necessary that the reference sequence be maternal sequence.

In addition, the method may employ a wide range of approaches to capture and detect fetal genetic material, either directly or indirectly. One method described herein involves a combination of using a molecular inversion probe (MIP) (or other oligonucleotide probe) instead of a pair of primers to bind to genomic DNA, followed by steps comprising a hybridization step to bind MIP probes to a complementary sequence within a target polynucleotide, a ligation reaction step to circularize bound probes, an exonuclease treatment step to digest residual non-circularized MIP probes, an optional treatment step, where an enzyme such as uracil-N-glycosylase is used to linearize circularized probes; a partitioning step, where the circularized probes, or linearized probes (that were previously circular) are partitioned or subdivided into two or more partitions (e.g., droplets); followed by an amplification step involving amplification of a sequence unique to the oligonucleotide probe through droplet digital PCR.

In some cases, multiplexed MIPs (or other oligonucleotide) are used herein in order to improve sensitivity of detection. For example, a group of two or more MIPs can be used, wherein each of such MIPs binds to a different sequence on the same chromosome (e.g., chromosome 21). In some cases, multiple MIPs recognizing, for example, a target and reference sequence, can be differentially detected during amplification using fluorophores of different colors. In some cases, binding of a single linear probe to genomic DNA and a subsequent ligation reaction produces a circular molecule. In other cases, two linear probes bind to adjacent regions of genomic DNA, and a subsequent ligation reaction produces a ligation-dependent molecule that can be detected in a ligation-detection reaction (LDR).

As used herein, the term ligation refers to a covalent bond or linkage between two or more nucleic acids, e.g. oligonucleotides and/or polynucleotides. Often, a ligation may comprise ligating the 5' terminus of a polynucleotide (e.g., ligation probe) to the 3' terminus of another polynucleotide (e.g., ligation probe), or to the same polynucleotide. The nature of the bond or linkage may vary widely and the ligation may be carried out enzymatically or chemically. Ligations are usually carried out enzymatically to form a phosphodiester linkage between a 5' carbon of a terminal nucleotide of one oligonucleotide with 3' carbon of another oligonucleotide. A variety of binding-driven ligation reactions are described in the following references: Whitely et al, U.S. Pat. No. 4,883,750; Letsinger et al, U.S. Pat. No. 5,476,930; Fung et al, U.S. Pat. No. 5,593,826; Kool, U.S. Pat. No. 5,426,180.

The present disclosure provides methods and compositions for the removal of undesired material, including unbound genomic DNA and unligated probe, and the selection or isolation of desired material, including ligation product. In some cases where the product of ligation is circular, such as in reactions involving a MIP, unbound genomic DNA and unligated probe is removed using exonuclease treatment. In some cases, the circular ligation product is then released using treatment with an enzyme such as uracil-N-glycosylase, which depurinates uracil residues in the probes. In these cases, the abasic site is cleaved upon heating, resulting in a linearized ligation product.

Detection can occur using a variety of methods. In some cases, a product of ligation is detected using a droplet digital PCR reaction in which DNA synthesis proceeds by the extension of at least one detection probe containing a fluorescer-quencher pair within a single molecule. Fluorescer refers to a molecule that emits detectable light after absorbing light or other electromagnetic radiation (e.g. a fluorophore). Quencher refers to a molecule that decreases the fluorescence intensity of a substance, and in the case of a fluorescer-quencher pair, the quencher may reduce detection of a covalently-attached fluorescer by absorbing the detectable light it emits. During the process of DNA synthesis, the 5'→ 3' exonuclease activity of a polymerase enzyme such as Taq polymerase cleaves the detection probe, resulting in release of the fluorescer from the quencher. A variety of fluorescence detection methods can detect the released fluorescer, but not the fluorescer-quencher pair. In some embodiments, detection of a product of ligation provides a quantitative measurement of the presence of a specific sequence, such as a target or reference sequence in fetal or maternal genetic material.

The present disclosure may further provide compositions and methods for the detection of a nucleic acid molecule of interest, where the sample may comprise DNA, RNA, or cDNA from any organism that is detected using droplet digital PCR. In some cases, the sample is isolated using a ligation reaction which is followed in some cases by exonuclease treatment to remove unwanted material. In some embodiments, detection occurs by fluorescence monitoring of droplet digital PCR, where a droplet comprises reagents for PCR and one or more ligation products detectable by PCR reaction, suspended in aqueous phases in an emulsion.

### Tissue Acquisition and Preparation

The methods and compositions of the present disclosure provide a means for obtaining fetal or maternal genetic material. The methods and compositions provide for detecting a difference in copy number of a target polynucleotide without the need of an invasive surgical procedure, amniocentesis, chorionic villus sampling, etc. In other cases, the methods and compositions provide for detecting a difference in copy number of a target polynucleotide from a sample (e.g., blood sample), to be used in addition to, supplementary to, preliminary step to, or as an adjunct to a more invasive test such as a surgical procedure. Often, the fetal/maternal genetic material is obtained via a blood draw, or other method provided herein. In some preferred embodiments, the starting material is maternal plasma or peripheral blood, such as maternal peripheral venous blood. The peripheral blood cells may be enriched for a particular cell type (e.g., mononuclear cells; red blood cells; CD4+ cells; CD8+ cells; B cells; T cells, NK cells, or the like). The peripheral blood cells may also be selectively depleted of a particular cell type (e.g., mononuclear cells; red blood cells; CD4+ cells; CD8+ cells; B cells; T cells, NK cells, or the like). The starting material may also be bone marrow-derived mononuclear cells. The starting material may also include tissue extracted directly from a placenta (e.g., placental cells) or umbilical cord (e.g., umbilical vein endothelial cells, umbilical artery smooth muscle cell, umbilical cord blood cells). The starting material may also derive directly from the fetus in the form, e.g., of fetal tissue, e.g., fetal fibroblasts or blood cells. The starting material may also be from an infant or child, including neonatal tissue.

This starting material may be obtained in some cases from a hospital, laboratory, clinical or medical laboratory. In some embodiments, the sample is taken from a subject (e.g., an expectant mother) at at least 4, 5, 6, 7, 8, 9, 10, 11, 12, 13, 14, 15, 16, 17, 18, 19, 20, 21, 22, 23, 24, 25, or 26 weeks of gestation. In some embodiments, the subject is affected by a genetic disease, a carrier for a genetic disease or at risk for developing or passing down a genetic disease, where a genetic disease is any disease that can be linked to a genetic variation such as mutations, insertions, additions, deletions, translocation, point mutation, trinucleotide repeat disorders and/or single nucleotide polymorphisms (SNPs). In other embodiments, the sample is taken from a female patient of child-bearing age and, in some cases, the female patient is not pregnant or of unknown pregnancy status. In still other cases, the subject is a male patient, a male expectant father, or a male patient at risk of, diagnosed with, or having a specific genetic abnormality. In some cases, the female patient is known to be affected by, or is a carrier of, a genetic disease or genetic variation, or is at risk of, diagnosed with, or has a specific genetic abnormality. In some cases, the status of the female patient with respect to a genetic disease or genetic variation may not be known. In further embodiments, the sample is taken from any child or adult patient of known or unknown status with respect to copy number variation of a genetic sequence. In some cases, the child or adult patient is known to be affected by, or is a carrier of, a genetic disease or genetic variation.

An advantage of the methods and compositions provided herein is that they can enable detection of fetal nucleic acids (e.g., DNA, RNA) at a relatively early stage of gestation and at stages when the total concentration of fetal nucleic acids (e.g., DNA, RNA) in the maternal plasma is low. The starting material may have a fetal concentration that is at least 0.1%, 0.2%, 0.5%, 1%, 1.5%, 2%, 2.5%, 3%, 3.5%, 4%, 4.5%, 5%, 5.5%, 6%, 6.5%, 7%, 7.5%, 8%, 8.5%, 9%, 9.5%, 10%, 10.5%, 11%, 11.5%, 12%, 12.5%, 13%, 13.5%, 14%, 14.5%, 15%, 15.5%, 16%, 16.5%, 17%, 17.5%, 18%, 18.5%, 19%, 19.5%, 20%, 20.5%, 21%, 21.5%, 22%, 22.5%, 23%, 23.5%, 24%, 24.5%, or 25% of the total maternal genomic DNA load in a maternal sample, and preferably at least 3% of the total maternal genomic DNA load. In some cases, the fetal DNA concentration may be less than about 0.1 %, 0.2%, 0.5%, 1%, 1.5%, 2%, 2.5%, 3%, 3.5%, 4%, 4.5%, 5%, 5.5%, 6%, 6.5%, 7%, 7.5%, 8%, 8.5%, 9%, 9.5%, 10%, 10.5%, 11%, 11.5%, 12%, 12.5%, 13%, 13.5%, 14%, 14.5%, 15%, 15.5%, 16%, 16.5%, 17%, 17.5%, 18%, 18.5%, 19%, 19.5%, 20%, 20.5%, 21%, 21.5%, 22%, 22.5%, 23%, 23.5%, 24%, 24.5%, or 25% of the total maternal genomic DNA load in a maternal sample. In cases where the starting material comprises a type of polynucleotide (e.g., DNA, RNA) present in one quantity (H) and a type of polynucleotide (e.g., DNA, RNA, etc.) present at a lower quantity compared to H, the starting material may have a concentration of L that is at least 0.1%, 0.2%, 0.5%, 1%, 1.5%, 2%, 2.5%, 3%, 3.5%, 4%, 4.5%, 5%, 5.5%, 6%, 6.5%, 7%, 7.5%, 8%, 8.5%, 9%, 9.5%, 10%, 10.5%, 11%, 11.5%, 12%, 12.5%, 13%, 13.5%, 14%, 14.5%, 15%, 15.5%, 16%, 16.5%, 17%, 17.5%, 18%, 18.5%, 19%, 19.5%, 20%, 20.5%, 21%, 21.5%, 22%, 22.5%, 23%, 23.5%, 24%, 24.5%, or 25% of the total concentration of H in the sample, and preferably at least 3% of the H. In some cases, the L may be less than about 0.1%, 0.2%, 0.5%, 1%, 1.5%, 2%, 2.5%, 3%, 3.5%, 4%, 4.5%, 5%, 5.5%, 6%, 6.5%, 7%, 7.5%, 8%, 8.5%, 9%, 9.5%, 10%, 10.5%, 11%, 11.5%, 12%, 12.5%, 13%, 13.5%, 14%, 14.5%, 15%, 15.5%, 16%, 16.5%, 17%, 17.5%, 18%, 18.5%, 19%, 19.5%, 20%, 20.5%, 21%, 21.5%, 22%, 22.5%, 23%, 23.5%, 24%, 24.5%, or 25% of the total quantity of H in the sample.

In some cases, in order to obtain sufficient nucleic acid for testing, a blood volume of at least 1, 2, 3, 4, 5, 10, 20, 25, 30, 35, 40, 45, or 50 mL is drawn. This blood volume can provide at least 1,000 genome equivalents (GE) of total DNA. Total DNA is present at roughly 1,000 GE/mL of maternal plasma in early pregnancy, and a fetal DNA concentration of about 3.5% of total plasma DNA. However, less blood can be drawn for a genetic screen where less statistical significance is required, or the DNA sample is enriched for fetal DNA. Also, the fetal DNA concentration may vary according to the gestational age of the fetus. In some embodiments, fetal DNA or RNA may be enriched by isolating red blood cells, in particular fetal nucleated red blood cells, which differ from anucleated adult red blood cells, as described below. In other embodiments, red blood cells may be removed from a maternal blood sample, and genetic material may be obtained from maternal plasma.

In some embodiments, the starting material can be a tissue sample comprising a solid tissue, with non-limiting examples including brain, liver, lung, kidney, prostate, ovary, spleen, lymph node (including tonsil), thyroid, pancreas, heart, skeletal muscle, intestine, larynx, esophagus, and stomach. In other embodiments, the starting material can be cells containing nucleic acids, including connective tissue, muscle tissue, nervous tissue, and epithelial cells, and in particular exposed epithelial cells such as skin cells and hair cells. In yet other embodiments, the starting material can be a sample containing nucleic acids, from any organism, from which genetic material can be obtained and detected by droplet digital PCR, as outlined herein.

### Enrichment of Fetal Material

Fetal cells may be enriched from a maternal sample containing a mixture of fetal and maternal cells. Such enrichment may occur, in some cases, where fetal concentration is at least 0.1%, 0.2%, 0.5%, 1%, 1.5%, 2%, 2.5%, 3%, 3.5%, 4%, 4.5%, 5%, 5.5%, 6%, 6.5%, 7%, 7.5%, 8%, 8.5%, 9%, 9.5%, 10%, 10.5%, 11%, 11.5%, 12%, 12.5%, 13%, 13.5%, 14%, 14.5%, 15%, 15.5%, 16%, 16.5%, 17%, 17.5%, 18%, 18.5%, 19%, 19.5%, 20%, 20.5%, 21%, 21.5%, 22%, 22.5%, 23%, 23.5%, 24%, 24.5%, or 25% of the total maternal genomic DNA (or RNA) load. Such enrichment may occur, in some cases, where fetal concentration is more than 0.1%, 0.2%, 0.5%, 1%, 1.5%, 2%, 2.5%, 3%, 3.5%, 4%, 4.5%, 5%, 5.5%, 6%, 6.5%, 7%, 7.5%, 8%, 8.5%, 9%, 9.5%, 10%, 10.5%, 11%, 11.5%, 12%, 12.5%, 13%, 13.5%, 14%, 14.5%, 15%, 15.5%, 16%, 16.5%, 17%, 17.5%, 18%, 18.5%, 19%, 19.5%, 20%, 20.5%, 21%, 21.5%, 22%, 22.5%, 23%, 23.5%, 24%, 24.5%, or 25% of the total maternal genomic DNA (or RNA) load.

In some embodiments, fetal cells are enriched by affinity methods, which may include collection of fetal cells on a solid structure conjugated with molecules with greater affinity for fetal cells compared to non-fetal cells, such as fetal-specific antibodies. Non-limiting examples of a solid structure include a polymer surface, magnetic beads, polymer beads, and surface of a microfluidic channel. In some embodiments, a biological sample is not enriched for fetal cells prior to, or as part of, the methods or compositions described herein. In some embodiments, the fetal cells are not enriched by affinity methods. In some embodiments, the fetal cells are not enriched by the use of fetal-specific antibodies. In some cases, the fetal cells are not enriched via the introduction of the sample to a microfluidic device.

Flow cytometry techniques can also be used to enrich fetal cells (Herzenberg et al., PNAS 76: 1453-1455 (1979); Bianchi et al., PNAS 87: 3279-3283 (1990); Bruch et al., Prenatal Diagnosis 11: 787-798 (1991)). U.S. Pat. No. 5.432.054 also describes a technique for separation of fetal nucleated red blood cells, using a tube having a wide top and a narrow, capillary bottom made of polyethylene. In some cases, flow cytometry is not used to enrich fetal cells in samples analyzed using the present methods or compositions. Centrifugation using a variable speed program results in a stacking of red blood cells in the capillary based on the density of the molecules. The density fraction containing low-density red blood cells, including fetal red blood cells, is recovered and then differentially hemolyzed to preferentially destroy maternal red blood cells. A density gradient in a hypertonic medium is used to separate red blood cells, now enriched in the fetal red blood cells from lymphocytes and ruptured maternal cells. The use of a hypertonic solution shrinks the red blood cells, which increases their density, and facilitates purification from the more dense lymphocytes. After the fetal cells have been isolated, fetal DNA can be purified using standard techniques in the art, detailed herein.

In some embodiments, the maternal blood can be processed to enrich the fetal DNA concentration in the total DNA, as described in Li et al., (2005) J. Amer. Med. Assoc. 293:843-849. Briefly, circulatory DNA can be extracted from 5- to 10-mL maternal plasma using commercial column technology (e.g., Roche High Pure Template DNA Purification Kit; Roche) in combination with a vacuum pump. After extraction, the DNA can be separated by agarose gel (1%) electrophoresis (Invitrogen), and the gel fraction containing circulatory DNA with a size of approximately 300 nucleotides can be carefully excised. The DNA can be extracted from this gel slice by using an extraction kit (QIAEX II Gel Extraction Kit; Qiagen) and eluted into a final volume of 40-µL sterile 10-mM TRIS-hydrochloric acid, pH 8.0 (Roche).

In some embodiments, free fetal DNA is isolated from a maternal blood sample containing whole cells. In preferred embodiments, free fetal DNA is isolated from a sample of maternal plasma. In some embodiments, the plasma sample is at least 50%, 75%, or 95% free of intact cells. In some embodiments, the plasma is completely free of intact cells.

United States Patent Application 20040137470 to Dhallan, Ravinder S, published Jul. 15, 2004, entitled "Methods for detection of genetic disorders," describes an enrichment procedure for fetal DNA," in which blood is collected into 9 ml EDTA Vacuette tubes (catalog number NC9897284) and 0.225 ml of 10% neutral buffered solution containing formaldehyde (4% w/v), is added to each tube, and each tube gently is inverted. The tubes are stored at 4°C. until ready for processing. Agents that impede cell lysis or stabilize cell membranes can be added to the tubes including but not limited to formaldehyde, and derivatives of formaldehyde, formalin, glutaraldehyde, and derivatives of glutaraldehyde, crosslinkers, primary amine reactive crosslinkers, sulfhydryl reactive crosslinkers, sulfhydryl addition or disulfide reduction, carbohydrate reactive crosslinkers, carboxyl reactive crosslinkers, photoreactive crosslinkers, cleavable crosslinkers, etc. Any concentration of agent that stabilizes cell membranes or impedes cell lysis can be added. In a preferred embodiment, the agent that stabilizes cell membranes or impedes cell lysis is added at a concentration that does not impede or hinder subsequent reactions.

In another embodiment, the DNA is isolated using techniques and/or protocols that substantially reduce the amount of maternal DNA in the sample including but not limited to centrifuging the samples, with the braking power for the centrifuge set to zero (the brake on the centrifuge is not used), transferring the supernatant to a new tube with minimal or no disturbance of the "buffy-coat," and transferring only a portion of the supernatant to a new tube. In a preferred embodiment, both acceleration power and braking power for the centrifuge are set to zero. In another embodiment, the DNA is isolated using techniques and/or protocols that substantially reduce the amount of maternal DNA in the sample including but not limited to centrifuging the samples, with the acceleration power for the centrifuge set to zero, transferring the supernatant to a new tube with minimal or no disturbance of the "buffy-coat," and transferring only a portion of the supernatant to a new tube. In another embodiment, the "buffy-coat" is removed from the tube prior to removal of the supernatant using any applicable method including but not limited to using a syringe or needle to withdraw the "buffy-coat." In another embodiment, the braking power for the centrifuge is set at a percentage including but not limited to 1-5%, 5-10%, 10-20%, 20-30%, 30-40%, 40-50%, 50-60%, 60-70%, 70-80%, 80-90%, 90-95%, 95-99% of maximum braking power.

In another embodiment, the acceleration power for the centrifuge is set at a percentage including but not limited to 1-5%, 5-10%, 10-20%, 20-30%, 30-40%, 40-50%, 50-60%, 60-70%, 70-80%, 80-90%, 90-95%, 95-99% of maximum acceleration power. In another embodiment, the present disclosure provides a composition comprising free fetal DNA and free maternal DNA, wherein the composition comprises a relationship of free fetal DNA to free maternal DNA including but not limited to at least about 15% free fetal DNA, at least about 20% free fetal DNA, at least about 30% free fetal DNA, at least about 40% free fetal DNA, at least about 50% free fetal DNA, at least about 60% free fetal DNA, at least about 70% free fetal DNA, at least about 80% free fetal DNA, at least about 90% free fetal DNA, at least about 91% free fetal DNA, at least about 92% free fetal DNA, at least about 93% free fetal DNA, at least about 94% free fetal DNA, at least about 95% free fetal DNA, at least about 96% free fetal DNA, at least about 97% free fetal DNA, at least about 98% free fetal DNA, at least about 99% free fetal DNA, and at least about 99.5% free fetal DNA.

Further, an agent that stabilizes cell membranes can be added to the maternal blood to reduce maternal cell lysis including but not limited to aldehydes, urea formaldehyde, phenol formaldehyde, DMAE (dimethylaminoethanol), cholesterol, cholesterol derivatives, high concentrations of magnesium, vitamin E, and vitamin E derivatives, calcium, calcium gluconate, taurine, niacin, hydroxylamine derivatives, bimoclomol, sucrose, astaxanthin, glucose, amitriptyline, isomer A hopane tetral phenylacetate, isomer B hopane tetral phenylacetate, citicoline, inositol, vitamin B, vitamin B complex, cholesterol hemisuccinate, sorbitol, calcium, coenzyme Q, ubiquinone, vitamin K, vitamin K complex, menaquinone, zonegran, zinc, ginkgo biloba extract, diphenylhydantoin, perftoran, polyvinylpyrrolidone, phosphatidylserine, tegretol, PABA, disodium cromglycate, nedocromil sodium, phenyloin, zinc citrate, mexitil, dilantin, sodium hyaluronate, or polaxamer 188. In another embodiment, an agent that preserves or stabilizes the structural integrity of cells can be used to reduce the amount of cell lysis. In another embodiment, any protocol that reduces the amount of free maternal DNA in the maternal blood can be used prior to obtaining the sample. In another embodiment, prior to obtaining the sample, the pregnant female rests with- out physical activity for a period of time including but not limited to 0-5, 5-10, 10-15, 15-20, 20-25, 25-30, 30-35, 35-40, 40-45, 45-50, 50-55, 55-60, 60-120, 120-180, 180- 240, 240-300, 300-360, 360-420, 420-480, 480-540, 540- 600, 600-660, 660-720, 720-780, 780-840, 840-900, 900- 1200, 1200-1500, 1500-1800, 1800-2100, 2100-2400, 2400- 2700, 2700-3000, 3000-3300, 3000-3600, 3600-3900, 3900- 4200, 4200-4500, and greater than 4500 minutes. In another embodiment, the sample is obtained from the pregnant female after her body has reached a relaxed state. The period of rest prior to obtaining the sample may reduce the amount of maternal nucleic acid in the sample. In another embodiment, the sample is obtained from the pregnant female in the a.m., including but not limited to 4-5 am, 5-6 am, 6-7 am, 7-8 am, 8-9 am, 9-10 am, 10-11 am, and 11-12 am. In another embodiment, the sample is obtained from the pregnant female after she has slept for a period of time including but not limited to 0-1, 1-2, 2-3, 3-4, 4-5, 5-6, 6-7, 7-8, 8-9, 9-10, 10-11, 11-12, or greater than 12 hours. In another embodiment, prior to obtaining the sample, the pregnant female exercises for a period of time followed by a period of rest. In another embodiment, the period of exercise includes but is not limited to 0-15, 15-30, 30-45, 45-60, 60-120, 120-240, or greater than 240 minutes. In another embodiment, agents that prevent the destruction of DNA, including but not limited to a DNase inhibitor, zinc chloride, ethylenediaminetetraacetic acid, guanidine-HC1, guanidine isothiocyanate, N-lauroylsarcosine, and Na-dodecylsulphate, can be added to the blood sample. In another embodiment, fetal DNA is obtained from a fetal cell, wherein said fetal cell can be isolated from sources including but not limited to maternal blood, umbilical cord blood, chorionic villi, amniotic fluid, embryonic tissues and mucous obtained from the cervix or vagina of the mother.

In another embodiment, any blood drawing technique, method, protocol, or equipment that reduce the amount of cell lysis can be used, including but not limited to a large boar needle, a shorter length needle, a needle coating that increases laminar flow, e.g., teflon, a modification of the bevel of the needle to increase laminar flow, or techniques that reduce the rate of blood flow. The fetal cells likely are destroyed in the maternal blood by the mother's immune system. However, it is likely that a large portion of the maternal cell lysis occurs as a result of the blood draw or processing of the blood sample. Thus, methods that prevent or reduce cell lysis will reduce the amount of maternal DNA in the sample, and increase the relative percentage of free fetal DNA.

An example of a protocol for using this agent is as follows: The blood is stored at 4°C. until processing. The tubes are spun at 1000 rpm for ten minutes in a centrifuge with braking power set at zero. The tubes are spun a second time at 1000 rpm for ten minutes. The supernatant (the plasma) of each sample is transferred to a new tube and spun at 3000 rpm for ten minutes with the brake set at zero. The supernatant is transferred to a new tube and stored at -80°C. Approximately two milliliters of the buffy coat, which contains maternal cells, is placed into a separate tube and stored at -80°C.

### Extraction of DNA or RNA

Genomic DNA may be isolated from plasma (e.g., maternal plasma) using techniques known in the art, such as using the Qiagen Midi Kit for purification of DNA from blood cells. DNA can be eluted in 100 µl of distilled water. The Qiagen Midi Kit also is used to isolate DNA from the maternal cells contained in the buffy coat. A QIAamp Circulating Nucleic Acid Kit may also be used for such purposes, see, e.g., http://www.qiagen.com/products/qiaampcirculatingnucleicacidkit.aspx.

Methods of extracting polynucleotides (e.g., DNA) may also include the use of liquid extraction (e.g, Trizol, DNAzol) techniques.

For example, the starting sample (e.g., blood or plasma) may have a starting volume of 15-30 ml, from which about 100-200 ul of DNA or other polynucleotide may be extracted. The 200 ul of DNA of the extracted sample may then be converted (or concentrated) into a final sample with a smaller volume, e.g., 5 ul, 10 ul. In some cases, the volume of the starting sample may be greater than 2-, 5-, 10-, 20-, 30-, 40-, 50-, 75-, 100-, 500-, 1000-, 5000-, 10,000-, 50,000-, 100,000-, 500,000-, or 1,000,000- fold the volume of the final sample. The final sample may also be a sample that is introduced into a device for droplet generation.

The final sample may be from 1 to 20 ul in volume. In some embodiments, the final sample is greater than 1, 5, 10, 15, 20, 25, 30, 35, 40, 45, 50, 75, or 100 ul. In some embodiments, the final sample is less than 1, 5, 10, 15, 20, 25, 30, 35, 40, 45, 50, 75, or 100 ul. In some embodiments, the final sample is greater than 1, 5, 10, 15, 20, 25, 30, 35, 40, 45, 50, 75, or 100 nl. In some embodiments, the final sample is less than 1, 5, 10, 15, 20, 25, 30, 35, 40, 45, 50, 75, or 100 nl. In some embodiments, the final sample is greater than 1, 5, 10, 15, 20, 25, 30, 35, 40, 45, 50, 75, or 100 pl. In some embodiments, the final sample is less than 1, 5, 10, 15, 20, 25, 30, 35, 40, 45, 50, 75, or 100 pl.

In some embodiments, DNA can be concentrated by known methods, including centrifugation and the use of various enzyme inhibitors (e.g. for DNase). The DNA can be bound to a selective membrane (e.g., silica) to separate it from contaminants. The DNA can also be enriched for fragments circulating in the plasma which are less than 1000, 500, 400, 300, 200 or 100 base pairs in length. This size selection can be done on a DNA size separation medium, such as an electrophoretic gel or chromatography material (Huber et al. (1993) Nucleic Acids Res. 21:1061-6), gel filtration chromatography, TSK gel (Kato et al. (1984) J. Biochem, 95:83-86). In some cases, the polynucleotide (e.g., DNA, RNA) may be selectively precipitated, concentrated (e.g., sample may be subjected to evaporation), or selectively captured using a solid-phase medium. Following precipitation, DNA or other polynucleotide may be reconstituted or dissolved into a small volume. A small volume may enable hybridization, or enable improved hybridization, of a probe with target polynucleotide.

In some embodiments, the starting material may comprise cells or tissue, including connective tissue, muscle tissue, nervous tissue, blood cells, or epithelial cells. In some cases, non-nucleic acid materials can be removed from the starting material using enzymatic treatments (such as protease digestion). Other non-nucleic acid materials can be removed in some cases by treatment with membrane-disrupting detergents and/or lysis methods (e.g. sonication, French press, freeze/thaw, dounce), which may be followed by centrifugation to separate nucleic acid-containing fractions from non-nucleic acid-containing fractions. The extracted nucleic acid can be from any appropriate sample including but not limited to, nucleic acid-containing samples of tissue, bodily fluid (for example, blood, serum, plasma, saliva, urine, tears, peritoneal fluid, ascitic fluid, vaginal secretion, breast fluid, breast milk, lymph fluid, cerebrospinal fluid or mucosa secretion), umbilical cord blood, chorionic villi, amniotic fluid, embryonic tissues, lymph fluid, cerebrospinal fluid, mucosa secretion, or other body exudate, fecal matter, an individual cell or extract of the such sources that contain the nucleic acid of the same, and subcellular structures such as mitochondria, using protocols well established within the art.

In a preferred embodiment, blood can be collected into an apparatus containing a magnesium chelator including but not limited to EDTA, and is stored at 4° C. Optionally, a calcium chelator, including but not limited to EGTA, can be added. In another embodiment, a cell lysis inhibitor is added to the maternal blood including but not limited to formaldehyde, formaldehyde derivatives, formalin, glutaraldehyde, glutaral- dehyde derivatives, a protein cross-linker, a nucleic acid cross-linker, a protein and nucleic acid cross-linker, primary amine reactive crosslinkers, sulfhydryl reactive crosslinkers, sultydryl addition or disulfide reduction, carbohydrate reac- tive crosslinkers, carboxyl reactive crosslinkers, photoreac- tive crosslinkers, cleavable crosslinkers

Plasma RNA extraction is described in Enders et al. (2003), Clinical Chemistry 49:727-731. Briefly, plasma harvested after centrifugation steps can be mixed with Trizol LS reagent (Invitrogen) and chloroform. The mixture can be centrifuged, and the aqueous layer transferred to new tubes. Ethanol is added to the aqueous layer. The mixture is then applied to an RNeasy mini column (Qiagen) and processed according to the manufactures's recommendations.

In some cases when the extracted material comprises single-stranded RNA, double-stranded RNA, or DNA-RNA hybrid, these molecules may be converted to double-stranded DNA using techniques known in the field. For example, reverse transcriptase may be employed to synthesize DNA from RNA molecules. In some cases, conversion of RNA to DNA may require a prior ligation step, to ligate a linker fragment to the RNA, thereby permitting use of universal primers to initiate reverse transcription. In other cases, the poly-A tail of an mRNA molecule, for example, may be used to initiate reverse transcription. Following conversion to DNA, the methods detailed herein may be used, in some cases, to further capture, select, tag, or isolate a desired sequence.

While the present description refers throughout to fetal DNA, fetal RNA found in maternal blood (as well as RNA in general) can be analyzed as well. As described previously, "mRNA of placental origin is readily detectable in maternal plasma," (Ng et al. (2003) Proc. Nat. Acad. Sci. 100:4748-4753), hPL (human placental lactogen) and hCG (human chorionic gonadotropin) mRNA transcripts are detectable in maternal plasma, as analyzed using the respective real-time RT-PCR assays. In the present method, mRNA encoding genes expressed in the placenta and present on a chromosome of interest can be used. For example, DSCR4 (Down syndrome critical region 4) is found on chromosome 21 and is mainly expressed in the placenta. Its mRNA sequence can be found at GenBank NM_005867. In this case, it is preferred to use RNase H minus (RNase ^{H-}) reverse transcriptases (RTs) to prepare cDNA for detection. RNase ^{H-} RTs are available from several manufacturers, such as SuperScript(TM) II (Invitrogen). Reverse transcriptase PCR can be used as described herein for chromosomal DNA. The RNA may include siRNA, mRNA cRNA, tRNA, rRNA, mRNA, or any other type of RNA.

### Ligation Probes

In some preferred embodiments, target polynucleotides are tagged, selected, captured, isolated and/or processed through the use of one or more ligation probes (also, at times, referred to herein as "ligatable probes"). A ligation probe comprises either: (1) a "circularizable probe", wherein each end (5' and 3') of a single polynucleotide (or oligonucleotide) binds to adjacent or neighboring regions of a target polynucleotide, and where following such binding, a ligation reaction can join the 5' terminus to the 3' terminus of the probe, thereby circularizing the probe; or (2) two polynucleotide (or oligonucleotide) probes wherein, after two probes bind to regions within a target polynucleotide, the 5' end of one probe can be ligated to the 3' end of a different probe. After two of such probes hybridize to neighboring or adjacent sequences of a target polynucleotide, a ligation reaction results in joining the two probes together into one linear probe.

In some embodiments, a ligation probe may also comprise: an enzymatic cleavage site, a universal primer site, and/or a universal probe-binding site. In some embodiments, the ligation probe is phosphorylated at its 5' terminus. In other embodiments, the ligation probe is not phosphorylated at it 5' terminus. Such phosphorylation at the 5' terminus may enable ligation of the 5' terminus to the 3' terminus of the same (or different) ligation probe that is bound to an adjacent region of target polynucleotide, without the need of a gap-fill reaction. In other cases, a probe is synthesized without phosphorylation at the 5' end. In such cases, the probe is designed so that the 5' end binds to a region neighboring, but not directly adjacent to, the binding site of the 3' end of the same (or different) probe. Ligation of such probe may additionally require a gap-fill, or extension reaction.

In some embodiments, a ligation probe is a molecular inversion probe. US patent 7,368,242 describes a molecular inversion probe and how it can be used to generate an amplicon after interacting with a target polynucleotide in a sample. A linear version of the probe is combined with a sample containing target polynucleotide under conditions that permit neighboring regions in the genetic target to form stable duplexes with complementary regions of the molecular inversion probe (or other ligation probe). In general, the 5' terminus of the probe binds to one of the target sequences, and the 3' terminus of the probe binds to the adjacent sequence, thereby forming a loop structure. The ends of the target-specific regions may abut one another (being separated by a nick) or there may be a gap of several (e.g. 1-10 nucleotides) between them. In some embodiments, the gap is greater than 5, 10, 20, 30, 40 ,50, 100, 200, 300, 400, or 500 nucelotides. In some preferred embodiments, the target-specific regions are directly adjacent (e.g., separated by 0 nucleotides). In either case, after hybridization of the target-specific regions, the ends of the two target specific regions are covalently linked by way of a ligation reaction or a multiextension reaction followed by a ligation reaction, using a gap-filling reaction.

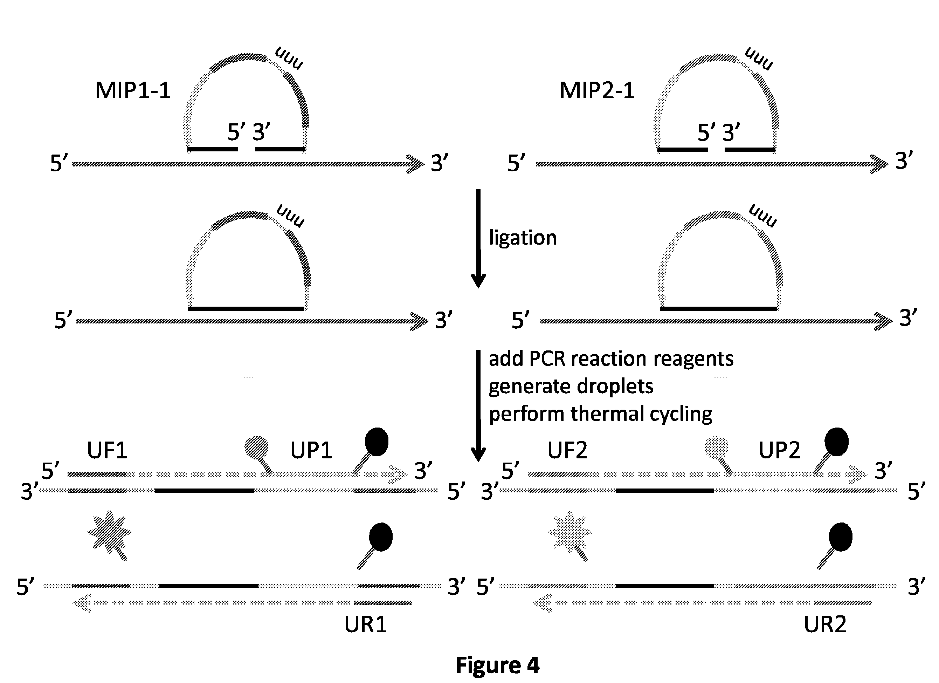

FIG. 4 is a schematic illustration of the use of Molecular Inversion Probes (MIPs) to detect two genetic targets. One genetic target is recognized by one probe (MIP1-1), and a second genetic target is recognized by a second probe (MIP2-1). After binding of a MIP to a genetic target, a ligation reaction is conducted to ligate the 5-terminus of a bound MIP probe to the 3-terminus, thereby forming a circular MIP. In some cases, a MIP probe binds two sequences of neighboring DNA that are separated by one or more nucleotides. In such cases, a gap-fill (or extension) reaction is performed to fill in the gap using the target DNA as a template. After a MIP binds its target sequences, the MIP forms a loop, and the sequence of the probe may be inverted (see FIG. 4). This inversion may be followed by a ligation reaction, in which the ends of the inverted molecule are ligated to form a circularized probe.

Following the binding of the MIP probe to the DNA (and, optionally the gap-fill reaction), a ligation reaction is conducted with a ligase enzyme to circularize the MIP probe. The circular MIP probe is then retained during exonuclease digestion, which digests unused, linear, single-stranded probe and single-stranded linear genomic DNA and double-stranded linear genomic DNA. The circular MIPs are then combined with PCR reagents into droplets for analysis by droplet digital PCR. In some embodiments, the circular probes are linearized prior to, or during the PCR reaction. As shown in FIG. 4, a probe may contain a site that comprises an enzymatic cleavate site (e.g., a series of uracil residues that are susceptible to enzymatic cleavage by uracil N-glycosylase enzyme). In some cases, there is an enzymatic cleavage step, wherein the polynucleotide is cleaved and forms a linear molecule. In other cases, there is no enzymatic cleavage step at this step, and the polynucleotide remains in a circular state. Next, the ligated MIP probes (either circularized, linear, or a mixture of both) are subdivided among one of more partitions. Preferably, the partitions are droplets (e.g., aqueous droplets within an oil phase). The droplets are then subjected to a thermal cycling reaction. During the thermal cycling reaction, a linearized MIP (or in some cases, a circular MIP) serves as the template for a reaction primed by a universal forward primer (UF1 or UF2) and a universal reverse primer (UR1 or UR2). During amplification, a universal probe that hybridizes to a sequence in each MIP (UP1 or UP2) is cleaved such that the fluorescent side of the probe is separated from the quencher side of the probe. As a result of this cleavage, fluorescence from the fluorescer side of the probe increases.

In some embodiments, a gap-fill reaction is performed by a polymerase with a 5'->3' polymerization activity. Polymerases useful in this method include those that will initiate 5'-3' polymerization at a nick site. The polymerase may also displace the polymerized strand downstream from the nick. In some embodiments, the polymerase used for the gap-fill reaction lacks any 5' ->3'exonuclease activity. A polymerase ordinarily having such exonuclease activity may lack such activity if that activity is blocked by the addition of a blocking agent; if a domain or fragment of the polymerase where such domain or fragment performs 5' ->3'exonuclease activity is deleted, mutated, or otherwise modified; if the polymerase is chemically modified; or any other method known in the art.

In some embodiments, the polymerase used for the gap-fill reaction comprises a 3'->5' editing exonuclease activity. Examples of suitable polymerases include the klenow fragment of DNA polymerase I and the exonuclease deficient klenow fragment of DNA polymerase I and a similar fragment from the Bst polymerase (Bio-Rad, Richmond, Calif.). SEQUENASE 1.0 and SEQUENASE 2.0 (US Biochemical), T5 DNA polymerase and Phi29 DNA polymerases also work, as does Stoffel Fragment of AmpliTaq DNA Polymerase (Life Technologies, Carlsbad, CA).

Although the present disclosure describes ligation probes (e.g., MIP probes) comprising DNA, the ligation probes described herein may contain any other nucleic acid (e.g., RNA, mRNA, cDNA, rRNA, tRNA, siRNA, miRNA, etc.), polypeptide, synthetic nucleic acid, or synthetic polypeptide. In some cases, the ligation probes may comprise a two or more different types of polynucleotides (e.g., comprising both RNA and DNA) or the ligation probe may comprise a polynucleotide and a polypeptide (e.g., RNA plus polypeptide; DNA plus polypeptide). In certain other applications, the ligation probe (e.g., MIP probe) may be conjugated to a fluorescent dye, solid support, or bead in the methods described herein.

Nucleic acid refers to naturally occurring and non-naturally occurring nucleic acids, as well as nucleic acid analogs that function in a manner similar to the naturally occurring nucleic acids. The nucleic acids may be selected from RNA, DNA or nucleic acid analog molecules, such as sugar- or backbone-modified ribonucleotides or deoxyribonucleotides. Other nucleic analogs, such as peptide nucleic acids (PNA) or locked nucleic acids (LNA), are also suitable. Examples of non-naturally occurring nucleic acids include: halogen-substituted bases, alkylsubstituted bases, hydroxy-substituted bases, and thiol-substituted bases, as well as 5-propynyl-uracil, 2-thio-5-propynyl-uracil, 5-methylcytosine, isoguanine, isocytosine, pseudoisocytosine, 4-thiouracil, 2-thiouracil and 2-thiothymine, inosine, 2-aminopurine, N9-(2-amino-6-chloropurine), N9-(2,6-diaminopurine), hypoxanthine, N9-(7-deaza-guanine), N9-(7-deaza-8-aza-guanine) and N8-(7-deaza-8-aza-adenine), 2-amino-6-"h"-purines, 6-amino-2-"h"-purines, 6-oxo-2-"h"-purines, 2-oxo-4-"h"-pyrimidines, 2-oxo 6-"h"-purines, 4-oxo-2-"h"-pyrimidines. Those will form two hydrogen bond base pairs with non-thiolated and thiolated bases; respectively, 2,4 dioxo and 4-oxo-2-thioxo pyrimidines, 2,4 dioxo and 2-oxo-4-thioxo pyrimidines, 4-amino-2-oxo and 4-amino-2-thioxo pyrimidines, 6-oxo-2-amino and 6-thioxo-2-amino purines, 2-amino-4-oxo and 2-amino-4-thioxo pyrimidines, and 6-oxo-2-amino and 6-thioxo-2-amino purines.

In some preferred embodiments, the method comprises selection, tagging, capture and/or isolation of a desired sequence from genomic DNA by selectively protecting the desired sequence from enzymatic digestion (from enzymes such as endonucleases and exonucleases). For example, circularization of a MIP probe (after it has bound its target) protects the probe from digestion by certain enzymes (e.g., exo I, exo III). Other methods of protecting the probe after it has bound its target may also be used.

In some cases, the ligation reaction may then be followed by enzymatic digestion, such as exonuclease treatment (e.g., exonuclease I, exonuclease III), to digest unbound genomic DNA and unbound probe but not circular DNA, thereby isolating the circular MIP representing the desired sequence. In some cases, MIPs allow for multiplexing, when more than one probe binds a desired genetic target and undergoes ligation to form a circular MIP. Multiple MIPs may thereby represent a given genetic target, enhancing the sensitivity of detection.

In some cases wherein circular MIPs are generated to represent sequences of interest, these circular MIPs may be linearized prior to (or during) detection by PCR reaction. In some cases, the MIPs contain uracil bases that may be depurinated by treatment with an enzyme such as uracil-N-glycosylase, and the circular molecule may become linearized at the abasic sites upon heating. In other cases, the MIPs may contain restriction enzyme sites that are targeted by site-specific restriction enzymes, cleaving the circular probes to form linear DNA molecules. In some embodiments in which circular MIPs are linearized, enzymes that occupy the solution containing MIPs, including exonucleases, are inactivated by such methods as heat-inactivation, pH denaturation, or physical separation prior to MIP linearization. In some cases, DNA may be purified from proteins using gel purification or ethanol precipitation, or proteins may be removed from the solution using precipitation with organic solutions such as trichloroacetic acid.

Examples of restriction enzymes include AatII, Acc65I, AccI, AciI, AcII, AcuI, AfeI, AflII, AflIII, AgeI, AhdI, AleI, AluI, AlwI, AlwNI. ApaI, ApaLI, ApeKI, ApoI, AscI, AseI, AsiSI, AvaI, AvaII, AvrII, BaeGI, BaeI, BamHI, BanI, BanII, BbsI, BbvCI, BbvI, BccI, BceAI, BcgI, BciVI. BclI, BfaI, BfuAI, BfuCI, BglI, BglII. BlpI, BmgBI, BmrI, BmtI, BpmI, Bpu10I. BpuEI, BsaAI, BsaBI, BsaHI, BsaI, BsaJI, BsaWI, BsaXI, BseRI, BseYI, BsgI, BsiEI. BsiHKAI, BsiWI, BsII, BsmAI, BsmBI, BsmFI, BsmI, BsoBI, Bsp1286I, BspCNI, BspDI, BspEI, BspHI, BspMI, BspQI, BsrBI, BsrDI, BsrFI, BsrGI, BsrI, BssHII, BssKI, BssSI, BstAPI, BstBI, BstEII, BstNI, BstUI, BstXI, BstYI, BstZ17I, Bsu36I, BtgI, BtgZI, BtsCI, BtsI, Cac8I, ClaI, CspCI, CviAII, CviKI-1, CviQI, DdeI, DpnI, DpnII, DraI, DraIII, DrdI, EaeI, EagI, EarI, EciI, Eco53kI, EcoNI, EcoO109I, EcoP15I. EcoRI, EcoRV, FatI, FauI, Fnu4HI, FokI, FseI, FspI, HaeII, HaeIII, HgaI, HhaI, HincII, HindIII, HinfI, HinP1I, HpaI, HpaII, HphI, Hpy166II, Hpy188I, Hpy188III, Hpy99I, HpyAV, HpyCH4III, HpyCH4IV, HpyCH4V, KasI, KpnI, MboI, MboII, MfeI, MluI, MlyI, MmeI, MnlI, MscI, MseI, MslI, MspA1I, MspI, MwoI, NaeI, NarI, Nb.BbvCI, Nb.BsmI, Nb.BsrDI, Nb.BtsI, NciI, NcoI, NdeI, NgoMIV, NheI, NlaIII, NlaIV, NmeAIII, NotI, NruI, NsiI, NspI, Nt.AlwI, Nt.BbvCI, Nt.BsmAI, Nt.BspQI, Nt.BstNBI, Nt.CviPII, PacI, PaeR7I, PciI, PflFI, PflMI, PhoI, PleI, PmeI, PmlI, PpuMI, PshAI, PsiI, PspGI, PspOMI, PspXI, PstI, PvuI, PvuII, RsaI, RsrII, SacI, SacII, SalI, SapI, Sau3AI, Sau96I, Sbfl, ScaI, ScrFI, SexAI, SfaNI, SfcI, SfiI, SfoI, SgrAI, SmaI, SmII, SnaBI, SpeI, SphI, SspI, StuI, StyD4I, StyI, SwaI, T, TaqαI, TfiI, TliI, TseI, Tsp45I, Tsp509I, TspMI, TspRI, Tth111I, XbaI, XcmI, XhoI, XmaI, XnmI, and ZraI.

Other types of probes, and other methods of selecting a genetic probe, may also be used in the methods and compositions described herein. For example, although use of MIP probes generally involves circularization of a single ligation probe; a circularization step is not always necessary. For example, ligation detection PCR techniques can be used, where two different probes, each of which hybridizes to neighboring DNA (or adjacent DNA), are ligated together followed by addition of universal primers and probes to detect the ligated fragments.

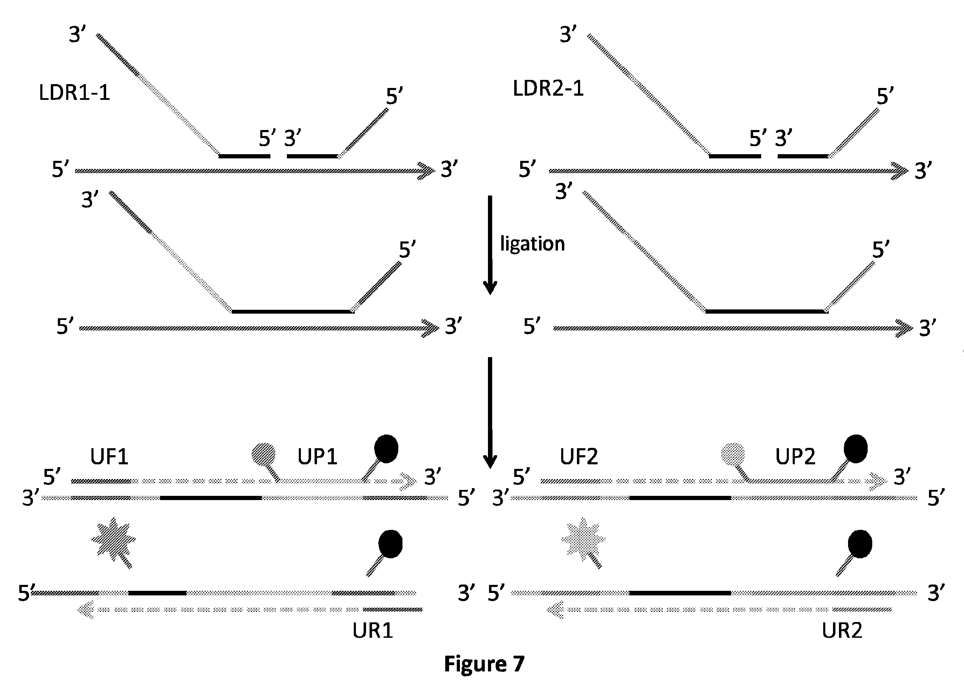

FIG. 7 shows a scheme for detecting two genetic targets with two colors using a ligation-detection reaction (LDR) followed by PCR in droplets. Two linear oligonucleotides bind to adjacent or neighboring regions on a genetic target. These regions may be directly adjacent or separated by a gap. Alternatively, the regions can be separated by a gap that can be filled-in using a polymerase reaction, that extends the length of the 3' end of the first probe so that its 3' end is directly adjacent to the 5' end of the second probe. The two probes are then ligated to each other (as depicted in FIG. 7). During ligation, the two linear oligonucleotides are ligated to form a single template oligonucleotide (LDR1-1 or LDR2-1). This single template oligonucleotide, but not the pairs of oligonucleotides from which it was formed, can produce a product in a PCR reaction using universal forward (UF1 or UF2) and reverse (UR1 or UR2) primers. Additionally, the PCR reaction contains a universal probe (UP1 or UP2) comprising a fluorescer-quencher pair that hybridizes to a portion of the template oligonucleotide. During the PCR reaction, a 5'-->3' exonuclease activity of a DNA polymerase (such as Taq) cleaves the probe, resulting in detachment of the fluorescer end from the quencher end of the molecule. As a result of this separation between fluorescer probe and quencher probe, fluorescence intensity will increase in the reaction, and can be detected in following steps. This analysis can be performed using two universal probes (UP1 and UP2) containing fluorescers of two different colors that can be distinguished during detection. For example, LDR1-1 may recognize a target sequence such as a suspected aneuploid chromosome, while LDR2-1 recognizes a reference sequence such as a presumed diploid chromosome, allowing detection of aneuploidy.

The ligation probes used in ligation detection reactions described herein may be protected from exonuclease treatment once they are bound to a target polynucleotide. For example, addition of a protective group, a chemical blocking unit, or a phosphorothiate modification may protect a hybridized ligation probe from being digested by certain exonucleases capable of digesting unbound probe and/or unbound target polynucleotides (e.g., genomic DNA). Phosphorothioate-modification may protect a ligation probe from the activity of exo III, a 3' to 5' exonuclease. Similarly, phosphorothioate-modification may protect a ligation probe from the activity exo T7, a 5' to 3' exonuclease. In some cases, exo T, a 3' to 5' exonuclease, and RecJf, a 5' to 3' exonuclease can be used. Disclosure of phosphorothioate providing protection against exo T activity is provided in Putney et al. (1981) PNAS 78(12):7350-54. For RecJf, see alsoTosch et al, (2007) J. of Plysics: Conference Series 61 (2007) 1241-1245; doi: 10.1088/1742-6596/61/1/245 International Conference on Nanoscience and Technology (ICN&T 2006). Both exo T and RecJF digest ssDNA and are blocked by phosphorothioates. The phosphothiorate modification may be located at the ends of the universal PCR primer sequences in the probes, or at tails upstream of the unversal PCR primer sequences.

In some embodiments, the probes comprise a mixture of different linear oligonucleotides, wherein the 5'region of one of the linear oligonucleotides is able to be ligated to the 3' region of a different linear oligonucleotide, after each probe hybridizes to a target polynucleotide. In some embodiments, two identical (or substantially identical) oligonucleotides can each bind to a region of adjacent or neighboring target polynucleotide in a manner such that the 5' end of one such probe can then be ligated to the 3' end of another such probe. Such ligation occurs following hybridization of each probe to the target polynucleotide.

In other embodiments, the method comprises capture of a desired sequence without subsequent isolation. In some cases, more than one linear probe recognizes the desired sequence and binds to it. Following the binding of probe, a ligation reaction may be performed to ligate one or more probes to one another. In some cases, the desired sequence is captured as a result of the ligation, which may allow PCR detection of ligated probe (known as ligase detection reaction-PCR, or LDR-PCR) in subsequent steps, while unligated probe is not detectable by PCR. In some cases, multiple probes may bind a genetic target and undergo ligation, enhancing the sensitivity of detection of the genetic target by LDR-PCR.

Ligation probes (e.g., MIP probes) can be designed to satisfy certain criteria in order to minimize sample to sample variation in assay performance, or to otherwise optimize an assay. Some criteria of use in the design of a ligation probe include: (1) target sequences that do not contain any known SNPs (eg all the SNPs in dbSNP); 2) target sequences within conserved regions of genomic DNA; (3) target sequences that do not overlap any known CNV regions (e.g. all the CNVs present in CNV tracks in the UCSC genome database); 4) target sequences within in regions of a target polynucleotide (e.g., genomic DNA, RNA) that are conserved across species (e.g. as assessed by conservation tracks in the UCSC genome database). Additionally, to optimize universal and consistent performance of the probes, several criteria can be applied to the selection of the target sequences. Target sequences can be chosen so that they are unique in the human genome. Target sequences can be chosen so that both termini of the MIP probes contain G/C nucleotides, so that they are near 40 nucleotides in length, so that combined homer arms have similar melting temperatures (e.g. within 2 of 67 degrees using default parameters from Primer3 software) and so that individual homer arms have similar melting temperatures (e.g. within 2 degrees of 50 degrees using default parameters from Primer3 software). (The 5'- and 3'- ends of the probe, which are complementary to genomic DNA are called homer arms: H2 and H1, respectively.)

The MIPs and the targets can also be screened to discard MIPs and targets that form secondary structures because they may not bind well to their counterparts. Additionally, MIPs can be compared to each other to reduce the possibility of reactions between MIP probes in solution. Some generic rules for avoiding secondary structure can be found in Hyman et al. (2010), Applied and Environmental Microbiology 76: 3904-3910. Secondary structure screening can be aided by building distributions of dG scores and removing outliers.

The methods provided herein include methods for assessing multiple abnormalities simultaneously, for example on chromosomes 13, 18, and 21. For such studies, the chromosomes can be used as references for each other, and therefore an extra reference sample or reference probe (e.g., to Chromosome 1) may be unnecessary

The sample containing the genetic target may comprise genomic DNA in the form of whole chromosomes, chromosomal fragments, or non-chromosomal fragments. In some cases, the average length of the genomic DNA fragment may be less than about 100, 200, 300, 400, 500, or 800 base pairs, or less than about 1, 2, 5, 10, 20, 30, 40, 50, 60, 70, 80, 90, 100, 110, 120, 130, 140, 150, 160, 170, 180, 190, or 200 nucleotides, or less than about 1, 2, 5, 10, 20, 30, 40, 50, 60, 70, 80, 90, 100 kilobases. In some cases, the fragments range from 10 to 500, 10-1000, or 100-150 bases (or nucleotides) in length, and, in some embodiments, preferably between 100-150 bases.

In some cases in which fetal genomic DNA is enriched compared to maternal DNA, the fragment size may be an average of about 300 base pair or 100 or 150 base pairs. In some cases, the sample will comprise at least one genome equivalent. In other cases, the sample will comprise less than one genome equivalent, but include enough genomic DNA to make a determination of the ratios of target and reference sequences in fetal or maternal samples. In still other cases, the sample will comprise about half of one genome equivalent. The term genome equivalent is used to refer to the calculated distribution of sample DNA based on a calculated genome size and DNA weight, wherein the haploid genome weighs about 3.3 pg, and the genomic content of a diploid normal cell (46 chromosomes) weighs about 6.6 pg and corresponds to two genomic equivalents (GE)( "genomic equivalent" and "genome equivalent" are used interchangeably herein). In practice, there may be some variation in DNA sample size. Also, due to random fragment distribution, a given genome equivalent may not contain exactly the DNA fragments corresponding only to a single complete diploid genome.

### Multiplexing

The amplification methods (e.g., PCR) described herein, and known in the art, can be multiplexed, that is, run with multiple primers and probes in each reaction volume. In some embodiments of the methods and compositions provided herein, there are at least 1, 2, 3, 4, 5, 6, 7, 8, 9, 10, 15, 20, 25, 30, 35, 40, 45, 50, 100, 200, 500, 1000, 5000, 10,000, 20,000, 30,000, 40,000, 50,000, 60,000, 70,000, 100,000, 2,000,000, 3, 000,000, 4,000,000, 5,000,000, 6,000,000, 7,000,000, 8,000,000, 9,000,000 or 10,000,000 or more different probes in a given sample volume. In some embodiments, there are at least 1, 2, 3, 4, 5, 6, 7, 8, 9, 10, 15, 20, 25, 30, 35, 40, 45, 50, 100, 200, 500, 1000, 5000, 10,000, 20,000, 30,000, 40,000, 50,000, 60,000, 70,000, 100,000, 2,000,000, 3, 000,000, 4,000,000, 5,000,000, 6,000,000, 7,000,000, 8,000,000, 9,000,000 or 10,000,000 or more primers in a given sample volume.

In some embodiments, a plurality of probes (or primer sets) are used, and the probes (or primer sets) differ with respect to one or more aspects. The probes may bind identical target polynucleotides; or different target polynucleotides (e.g., different chromosomes; or identical chromosomes, but different regions within said chromosomes). For example, greater than 1, 2, 3, 4, 5, 6, 7, 8, 9, 10, 11, 12, 13, 14, 15, 16, 17, 18, 19, or 20 probes directed to different targets may be used. In some cases, greater than 20, 30, 40, 50, 100, 500, 1000, 5000, 10000, 50000, 100000, 500000, 1000000, or more probes directed to different targets are used. In other cases, the probes differ as to the type of cleavage site that is present within said probe. In some cases, the plurality of probes comprises a plurality of different types of probes (e.g., ligation probes, MIPs, padlock probes, sets of PCR primers, universal primers, universal probes, and any combination thereof). In some cases, said plurality of different types of probes differ in that each probe is conjugated to a different signaling agent (e.g., green fluorophore vs. red fluorophore, etc.). In some cases, the probes differ in that they comprise different primer binding sites. In other cases, the same set of universal primers may be used to bind all, or most of, the probes within a plurality of probes.

Multiplexing in reaction volumes, such as droplets, allows for detection of small changes in DNA ratio between a target and reference sequence from the expected ratio of 1:1 for diploid sequences. Multiplexing allows for a large number of sequences to be counted for any set of target and reference sequences, despite samples where the GE/mL is low (e.g. 1000 GE/mL), such as in maternal plasma. The intact target and reference chromosomes are large molecules and have multiple conserved and unique regions that can be recognized and amplified by specific primer sets. In plasma, the circulating DNA can be present as small fragments (-300 bp). By designing multiplexed primers that produce small products (e.g., 100 base pairs), small fragments (e.g. 300 base pairs) of the target or reference sequence can be efficiently amplified.

Multiplexing may increase the likelihood that a target isolated in a reaction volume, such as a droplet, can be recognized by one of the multiplexed primers. Multiplexing may also increase the likelihood that amplification will occur and may permit a positive measurement of a target sequence that would be counted as negative in a single-plex assay. The same can be done for a reference sequence. In some embodiments, the degree of multiplexing can include more than one primer set to a target sequence, such as at least 2, 3, 4, 5, 10, 15, 20 or 25 primer sets, each to a particular target sequence. In some embodiments, the degree of multiplexing can include more than one reference primer set to a reference sequence, such as at least 2, 3, 4, 5, 10, 15, 20 or 25 primer sets, each to a particular reference sequence. In some embodiments, the degree of multiplexing can include more than one primer set to a target sequence and more than one reference primer set to a reference sequence, such as at least 2, 3, 4, 5, 10, 15, 20 or 25 primer sets to particular target or reference sequences. In some embodiments, the number of primer sets to a target sequence is not the same as the number of primer sets to a reference sequence. In some embodiments, the degree of multiplexing can be less than 500, 250, 200, 150, or 100 primer sets for each target and reference sequence. The target and reference sequence multiplexes can be combined into a single reaction volume.

The different primer pair amplified sequences can be differentiated based on spectrally distinguishable probes (e.g. 2 different dye-labeled probes such as Taqman or Locked Nucleic Acid Probes (Universal Probe Library, Roche)). In such approach, all probes are combined into a single reaction volume and distinguished based on the differences in the color emitted by each probe. For example, the probes targeting one polynucleotide (e.g., a test chromosome, chr. 21) may be conjugated to a dye with a first color and the probes targeting a second polynucleotide (e.g., a reference chromosome, chr. 1) in the reaction may be conjugated to a dye of a second color. The ratio of the colors then reflects the ratio between the test and the reference chromosome.

In some cases a set of probes (e.g., a set of probes targeting a test chromosome, e.g., Chromosome 21), may target different regions of a target polynucleotide, yet each probe within the set has the same universal primer binding sites. In some cases, each probe has the same probe-binding site. In some cases, two or more probes in the reaction may have different probe-binding sites. In some cases, the probes added to such reactions are conjugated to the identical signal agent (e.g., fluorophore of same color). In some cases, different signal agents (e.g., two different colors) are conjugated to one or more probes.

Alternatively the set of reaction volumes (e.g. droplets) can be split into two sample sets, with amplification of target sequence in one set and reference sequence in the other set. The target and reference genes are then measured independently of each other. This would allow the use of a single fluorescence probe such as SYBR Green. In some instances, this requires splitting the sample and potentially doubling the number of primers in each multiplex set to achieve an equivalent sensitivity. In some cases, the sample is split and a plurality of ligation probes to a test chromosome is added to one half of the sample, and a plurality of ligation probes to a reference chromosome is added to the second half of the sample. In such examples, the ligation probes may then be hybridized to a universal probe conjugated to the same signaling agent (e.g., fluorophore of the same color spectrum).

The multiplexing provided by the instant disclosure can also be accomplished using a probe for a target, instead of using a primer pair, at an early step. An example of a probe that can be used is a linear oligonucleotide with two ends specifically designed to hybridize to adjacent, or neighboring, sequences within a target polynucleotide. A non-limiting example of such a probe is a padlock probe, which is a linear oligonucleotide with two ends specifically designed to hybridize to adjacent target sequences. Once hybridized, the two ends can be joined by ligation and the padlock probe becomes circularized. Padlock probes are disclosed in, e.g., Lizardi et al. (1998) Nat Genetics 19:225-232; U.S. patent 5,871,921; 6,235,472; and 5,866,337. In some cases, the probe (e.g., oligonucleotide) binds to adjacent sequences of genomic DNA and the ends can then be directly ligated via a ligase reaction. In other cases, there is a gap of one or more bases between the two ends. In such cases, an extension, or gap fill, reaction can be performed. For the gap fill reaction, any known method in the art will suffice. For example, a mix of nucleotides (dATP, dCTP, dGTP, dTTP, dUTP) can be added to a reaction mix, as well as a polymerase, ligase and other reaction components and incubating at about 60°C for about 10 minutes, followed by incubation at 37°C for about 1 minute. Following binding to a target polynucleotide, and ligation, a ligation probe (e.g., molecular inversion probe, padlock probe, etc.) may become circularized.

In some cases, the probe is an oligonucleotide probe that binds to a genetic target, as described herein. In other cases, the probe is an oligonucleotide probe that binds to a reference target. An example of a reference target is Chromosome 1, or other Chromosome unlikely to be associated with fetal aneuploidy. In some cases, the oligonucleotide or reference oligonucleotide comprises a site cleavable by an enzyme. For example, the oligonucleotide may be a DNA oligonucleotide that comprises a series of one or more uracil residues, e.g., at least 1, 2, 3, 4, 5, 6, 7, 10, 15, or 20 uracil residues, and may be cleavable by an enzyme such as uracil-N-glycosylase (UNG). In other cases, the oligonucleotide may comprise one or more restriction sites. The oligonucleotide may comprise one or more of the same restriction sites, or one or more different restriction sites. Examples of restriction sites are well known in the literature. In general, a site cleavable by a restriction enzyme may be used. The restriction enzymes may be any restriction enzyme (or endonuclease) that can cut at a specific site. In some cases, the restriction enzymes are blunt cutters; in others, the restriction enzymes cut at an asymmetrical site to create an overhang. Non-limiting examples of restriction enzymes are provided herein.

The oligonucleotide probe may further comprise sites that hybridize to forward and reverse primers, e.g., universal primers. As used herein, universal primers include one or more pairs of 5' and 3' primers that recognize and hybridize to sequences flanking a region to be amplified. The region to be amplified may be within a genetic target such as a suspected fetal aneuploid chromosome, with non-limiting examples of such chromosomes including chromosome 21, chromosome 13, chromosome 18, and the X chromosome. In some cases, the region to be amplified is within a genetic target of a presumed diploid chromosome.

In some cases, the region to be amplified is not within a genetic target, but within a probe to a genetic target, such as a molecular inversion probe. Primer pairs may be directed to a genetic target, or they may be universal primers that recognize sequences flanking a multitude of amplification targets. For example, probes to a genetic target may comprise one or more segments that recognize and bind to a specific sequence in a genetic target, and the probes may additionally comprise a universal sequence common to all of a set of probes. A single pair of universal primers may therefore be employed to amplify any probes within such a set. In some cases, the universal pair of primers only produces a detectable PCR product when the molecular inversion probe has been inverted. Inversion of a molecular inversion probe can be induced by cleavage of a site within a circular molecular inversion probe that results in an inverse orientation of a primer with respect to its primer pair. In some cases, a universal pair of primers only produces a detectable PCR product when amplifying the product of a ligation reaction, such as in a ligation detection reaction.

The oligonucleotide probe may also comprise a sequence that is complementary to a probe attached to a marker, such as a dye or fluorescent dye (e.g., TaqMan probe). In some cases, the TaqMan probe is bound to one type of dye (e.g., FAM, VIC, TAMRA, ROX). In other cases, there are more than one TaqMan probe sites on the oligonucleotide, with each site capable of binding to a different TaqMan probe (e.g., a TaqMan probe with a different type of dye). There may also be multiple TaqMan probe sites with the same sequence of the oligonucleotide probe described herein. Often, the TaqMan probe may bind only to a site on the oligonucleotide probe described herein, and not to genomic DNA, but in some cases a TaqMan probe may bind genomic DNA.

The advantage of using the oligonucleotide probes described herein is that the signal-to-background noise is improved greater than 1-, 2-, 5-, 10-, 15-, 20-, 30-, 40-, 50-, 75-, or 100-fold over using conventional PCR techniques such as techniques that use a primer set. One reason is that, potentially, only one probe is needed for all the oligonucleotide probes to a specific target, e.g., a chromosome. For example, there may be a large number of oligonucleotide probes (e.g., greater than 50), wherein each binds to a separate site on a chromosome, but wherein each also comprisese a TaqMan site that is universal or the same, and therefore will fluoresce at the same wavelength when a TaqMan probe bound to a specific fluorescent dye is annealed to the probe.

The methods provided herein include methods with the following steps: a denaturation and annealing step in order to permit hybridization of one or more oligonucleotide probes with genomic DNA. An optional gap fill reaction, if the 5' and 3' ends of the probes do not target directly adjacent sequences of genomic DNA, followed by a ligase reaction to circularize the probe. The method may further comprise an exonuclease treatment step wherein the sample is treated with exonuclease enzymes, e.g., exonuclease I and/or III, that digest linear probes (in other words, probes that did not successfully hybridize) as well as ssDNA and dsDNA (e.g., genomic DNA), followed by an inversion step. The method may further comprise an amplification step wherein PCR reagents are added to the samples, e.g., Taq polymerase, universal primers, fluorescence probes (e.g., TaqMan probe), and other PCR reaction components, in order to amplify one or more sites on the oligonucleotide probe. The method may further comprise a partitioning step, wherein the sample is emulsified into monodisperse water-in-oil droplets, e.g., greater than 1,000, 10,000, 20,000, 50,000, 100,000, 200,000, 500,000 or more water-in-oil droplets (also referred to as reaction volumes, herein), followed by thermal cycling, and detecting the fluorescence of each droplet at a wavelength corresponding to the fluorescent probes that were used. In some cases, on average, about 1, 2, 3, 4, or 5 copies of DNA are present in each droplet. In some cases, an average of about .001, .005, .01, .05, .1, .5, 1, 2, 3, 4, or 5 oligonucleotide probes are present in each droplet. The methods described herein may function at high multiplex depths. When a high multiplex depth is coupled with ddPCR counting it can provide a large number of target counts to enable high resolution for relative chromosome dosage. This multiplex approach may be coupled with ddPCR fetal load quantification using paternally inherited SNPs, Y chromosome targets or fetal- specific methylation markers, to protect against false negatives. The fetal load measurement may be performed separately on an aliquot of the extracted sample, or may be conducted after the inversion step of the assay by multiplexing the two orthogonal assays (universal MIP PCR+fetal specific quantitation assay).

In some cases, ligation is coupled with universal PCR methods in order to achieve multiplexing. Examples include but are not limited to: a Molecular Inversion Probe (MIP) strategy *(see* Hardenbol et al., (2003) Nature Biotechnology, 21(6): 673-78);U.S. Patent Application Pubication No. 2004/0101835; Multiplex Ligation-dependent Probe Amplification (MLPA) (see Schouten JP, McElgunn CJ, Waaijer R, Zwijnenburg D, Diepvens F, Pals G (2002), Nucleic Acids Res. 30 (12)*;* Ligation Detection Reaction (LDR); and Ligase Chain Reaction. The Figures of the instant specification provide a summary of different multiplex strategies using different types of probes or probe/primer combinations.

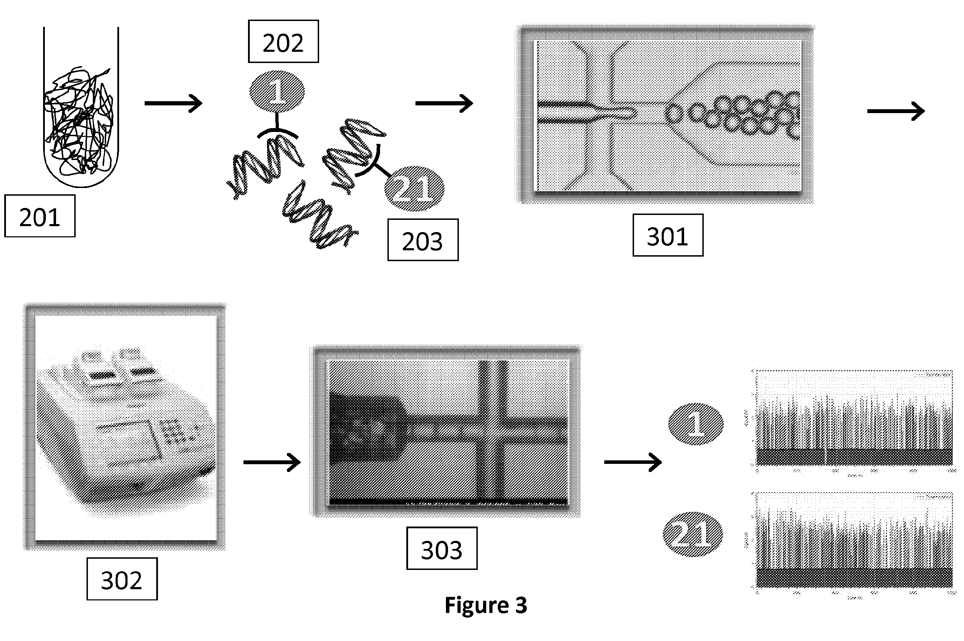

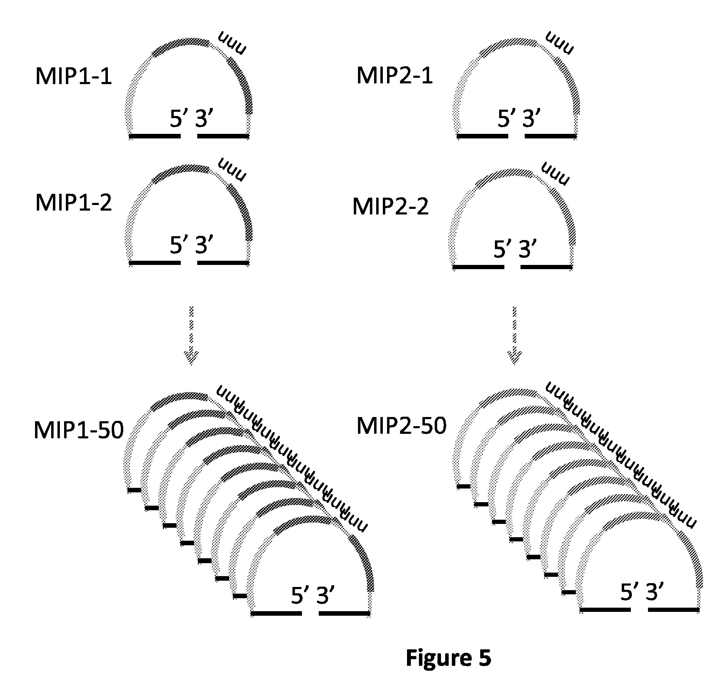

FIG. 5 shows multiplexing of the MIP approach to increase sensitivity of detection of genetic targets. A MIP recognizing a particular genetic target (MIP1-1) can be combined with a second MIP recognizing a different portion of the same genetic target (MIP1-2). This process can be repeated, generating many MIPs (MIP1-50 shown) to recognize the same genetic target. Similarly, a collection of MIPs can be generated to recognize a second genetic target (MIP2-50). These MIPs can be employed in analysis such as that depicted in Figure 3, to compare two genetic targets.

The ligation, padlock or other oligonucleotide probe described herein may be mixed with genomic DNA. In some cases, a plurality of oligonucleotide probes are used, comprisomg greater than 1, 2, 3, 4, 5, 6, 7, 8, 9, 10, 15, 20, 25, 30, 35, 40, 45, 50, 100, 200, 500, 1000, 5000, or 10,000 oligonucleotide probes to a specific site on a chromosome or different chromosomes.

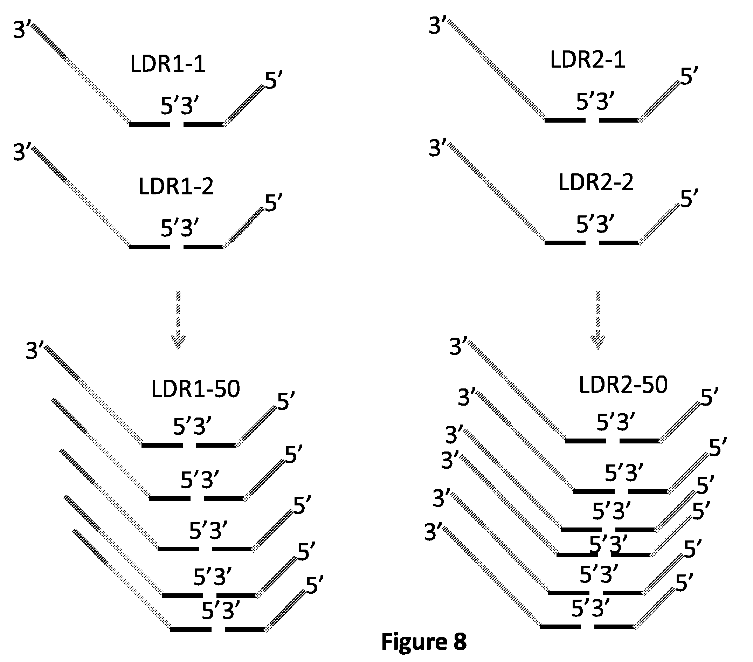

FIG. 8 depicts the use of multiplexed oligonucleotides for LDR-PCR in droplets to enhance sensitivity of this approach to detect genetic targets in droplets. As described in FIG. 7, a single pair of linear oligonucleotides (LDR1-1) is designed to recognize neighboring regions of a genetic target. A different pair (LDR2-1) recognizes a second genetic target. Multiple pairs of oligonucleotides (LDR1-50 and LDR2-50) may be designed to recognize different portions of a genetic target. These pairs of oligonucleotides bind a portion of the genetic target and undergo ligation as described in FIG. 7. Two different colors are used to detect the two different genetic targets depicted. For example, half the LDR probes may recognize a target sequence such as a suspected aneuploid chromosome, while the other half recognize a reference sequence such as a presumed diploid chromosome, allowing detection of aneuploidy with improved sensitivity.

In some embodiments, a target and reference sequence can be pre-amplified prior to analysis using digital droplet detection. Methods of amplification are known in the art, and include a self-sustained sequence reaction, ligase chain reaction, rapid amplification of cDNA ends, polymerase chain reaction and ligase chain reaction, Q-beta phage amplification, strand displacement amplification, isothermal amplification or splice overlap extension polymerase chain reaction. The pre-amplification product can then be used in the methods described herein.

### Genetic Targets

In some embodiments, extracted DNA or RNA may be processed to select, tag, capture and/or isolate target sequence polynucleotides, which may particularly include genetic targets described herein. In some cases, capture and isolation involves physical separation of target sequences from bulk genetic material, and removal of unwanted genetic material. In some cases, physical separation may be achieved by hybridizing desired sequences to complementary sequences immobilized on a solid structure such as a polymer surface, polymer beads, magnetic beads, or surface of a microfluidic channel. In other cases, physical separation is achieved by affinity methods, such as capturing a desired sequence using a probe of complementary sequence conjugated with an affinity tag, non-limiting examples of affinity interactions including streptavidin-biotin, antibody-antigen, enzyme-substrate, receptor-ligand, and protein-small molecule interactions having a binding affinity of greater than micromolar, nanomolar, picomolar, femtomolar, or greater than femtomolar strength. Following capture, desired sequences may in some cases be isolated from bulk genetic material using wash methods that are well-known in the arts, including washing with buffered saline solutions comprising mild ionic or non-ionic detergents, protease inhibitors, and DNase inhibitors. In some embodiments, the droplets described herein do not comprise beads , polymer beads, or magnetic beads.

The targets for the assays and probes described herein can be any genetic target associated with fetal genetic abnormalities, including aneuploidy as well as other genetic variations, such as mutations, insertions, additions, deletions, translocation, point mutation, trinucleotide repeat disorders and/or single nucleotide polymorphisms (SNPs), as well as control targets not associated with fetal genetic abnormalities. Other assays unrelated to fetal aneuploidy are also described herein.

Often the methods and compositions described herein can enable detection of extra or missing chromosomes, particularly those typically associated with birth defects or miscarriage. For example, the methods and compositions described herein enable detection of autosomal trisomies (e.g., Trisomy 13, 15, 16, 18, 21, or 22). In some cases the trisomy may be associated with an increased chance of miscarriage (e.g., Trisomy 15, 16, or 22). In other cases, the trisomy that is detected is a liveborn trisomy that may indicate that an infant will be born with birth defects (e.g., Trisomy 13 (Patau Syndrome), Trisomy 18 (Edwards Syndrome), and Trisomy 21 (Down Syndrome)). The abnormality may also be of a sex chromosome (e.g., XXY (Klinefelter's Syndrome), XYY (Jacobs Syndrome), or XXX (Trisomy X). In certain preferred embodiments, the genetic target is one or more targets on one or more of the following chromosomes: 13, 18, 21, X or Y. For example, the genetic target may be 50 sites on chromosome 21 and/or 50 sites on chromosome 18, and/or 50 sites on chromosome 13.

Further fetal conditions that can be determined based on the methods and systems herein include monosomy of one or more chromosomes (X chromosome monosomy, also known as Turner's syndrome), trisomy of one or more chromosomes (13, 18, 21, and X), tetrasomy and pentasomy of one or more chromosomes (which in humans is most commonly observed in the sex chromosomes, e.g. XXXX, XXYY, XXXY, XYYY, XXXXX, XXXXY, XXXYY, XYYYY and XXYYY), monoploidy, triploidy (three of every chromosome, e.g. 69 chromosomes in humans), tetraploidy (four of every chromosome, e.g. 92 chromosomes in humans), pentaploidy and multiploidy.

In some cases, the genetic target comprises more than 1, 2, 3, 4, 5, 6, 7, 8, 9, 10, 11, 12, 13, 14, 15, 16, 17, 18, 19, 20, 21, 22, 23,24, 25, 26, 27, 28, 29, 30, 31, 32, 33, 34, 35, 36, 37, 38, 39, 40, 41, 42, 43,44,45, 46, 47, 48, 49, 50, 75, 100, 125, 150, 175, 200, 225, 250, 300, 350, 400, 450, 500, 1,000, 5,000, 10,000, 20,000, 30,000, 40,000, 50,000, 60,000, 70,000, 80,000, 90,000 or 100,000 sites on a specific chromosome. In some cases, the genetic target comprises targets on more than 1, 2, 3, 4, 5, 6, 7, 8, 9, 10, 11, 12, 13, 14, 15, 16, 17, 18, 19, 20, 21, or 22 different chromosomes. In some cases the genetic target comprises targets on less than 2, 3, 4, 5, 6, 7, 8, 9, 10, 11, 12, 13, 14, 15, 16, 17, 18, 19, 20, 21, 22, or 23 chromosomes. In some cases, the genetic target comprises a gene that is known to be mutated in an inherited genetic disorder, including autosomal dominant and recessive disorders, and sex-linked dominant and recessive disorders. Non-limiting examples include genetic mutations that give rise to autoimmune diseases, neurodegenerative diseases, cancers, and metabolic disorders. In some embodiments, the method detects the presence of a genetic target associated with a genetic abnormality (such as trisomy), by comparing it in reference to a genetic target not associated with a genetic abnormality (such as a gene located on a normal diploid chromosome).

The methods or compositions herein may also comprise primer sets and/or probes targeting separate regions of a chromosome. For example, a plurality of probes (e.g.., MIP probes, ligation probes) may include at least one first probe that targets a first specific region of a chromosome and at least one second probe that targets a second specific region of a chromosome. In some cases, the first probe is tagged with a signaling molecule or agent (e.g., fluorophore), and the second probe is tagged with a second signaling molecule (e.g., a fluorophore of a color/wavelength distinguishable from that of the fluorophore conjugated to the first probe). The plurality of probes can then bind to the target polynucleotide. Following a selection protocol (e.g., ligation, circularization followed by exonuclease, etc.), the selected probes are partitioned into multiple partitions (e.g., droplets) followed by analysis of the number of partitions (e.g., droplets) containing a selected probe. The ratio between the number of first probes and the number of second probes may then be used to evaluate whether a target polynucleotide contains partial deletions, translocations, or amplifications. For example, such method may be used to detect a partial deletion of a chromosome, where probe 1 is directed to the intact chromosome and probe 2 is directed to a sequence within the deleted portion of the chromosome. In some embodiments, greater than 1, 2, 3, 4, 5, 6, 7, 8, 9, 10, 11, 12, 13, 14, 15, 16, 17, 18, 19, or 20 probes directed to different targets may be used. In some cases , this number may be greater than 20, 30, 40, 50, 100, 500, 1000, 5000, 10000, 50000, 100000, 500000, 1000000, or more.

In a nonlimiting example, a ligation probe (or primer set) targets the q arm of chromosome 21 and a second ligation probe (or primer set) targets the p arm. If both are giving answers that are reasonably close (e.g., within some pre-defined confidence interval) to each other, this may provide validation of the measurement of chromosome 21 concentration. If, on the other hand, the measurement made with the targets on the p arm is significantly different from the measurement made with those on the q arm, this may indicate a partial aneuploidy (fragment of a chromosome), or may indicate that the assay requires further optimization or validation.

The actual measurement of the target sets can be performed simultaneously by using one color for 21q targets and another color for 21p targets. Alternatively, the sample may be split so that the 21q measurements are made in one portion and the 21p measurement in the other. Also, chromosomes can be partitioned into more than two primer sets (or oligonucleotide probes) to have a more fine-grained assessment of the chromosomal copy number.

The term polynucleotide refers to any nucleic acid molecule containing more than one nucleotide, and can include, but is not limited to lengths of 2, 3, 5, 10, 20, 30, 50, 100, 200, 300, 400, 500, or 900 nucleotides, or 1, 2, 3, 5, 10, 20, 30, 50, 100, 200, 300, 400, 500, or 900 kilobases, or 1, 2, 3, 5, 10, 20, 30, 50, 100, 200, 300, 400, 500, or 900 megabases. A polynucleotide may also refer to the coding region of a gene, or non-coding regions of DNA, or a whole chromosome.

As used herein, an allele is one of several alternate forms of a gene or non-coding regions of DNA that occupy the same position on a chromosome. The term allele can be used to describe DNA from any organism including but not limited to bacteria, viruses, fungi, protozoa, molds, yeasts, plants, humans, non-humans, animals, and archeabacteria. For example, bacteria typically have one large strand of DNA. The term allele with respect to bacterial DNA refers to the form of a gene found in one cell as compared to the form of the same gene in a different bacterial cell of the same species.

Alternate forms of a gene (e.g., alleles) may include one or more single nucleotide polymorphisms (SNPs) in which a single nucleotide varies between alternate forms. Alternate forms of a gene or noncoding region may encompass short tandem repeats (STR), adjacent repeated patterns of two or more nucleotides.

Alleles can have the identical sequence or can vary by a single nucleotide or more than one nucleotide. With regard to organisms that have two copies of each chromosome, if both chromosomes have the same allele, the condition is referred to as homozygous. If the alleles at the two chromosomes are different, the condition is referred to as heterozygous.

Examples of diseases where the target sequence exist in one copy in the maternal DNA (heterozygous) disease in a fetus (homozygous), include sickle cell cystic fibrosis, hemophilia, and Tay Sachs disease. Accordingly, using the methods described here, one may distinguish genomes with one specific mutation at a certain site from genomes with two specific mutations at a certain site.

Sickle-cell anemia is an autosomal recessive disease. Nine-percent of US blacks are heterozygous, while 0.2% are homozygous recessive. The recessive allele causes amino acid substitution in the beta chains of hemoglobin.

Tay-Sachs Disease is an autosomal recessive resulting degeneration of the nervous system. Symptoms manifest after birth. Children homozygous recessive for this allele rarely survive past five years of age. Sufferers lack the ability to make the enzyme N-acetyl-hexosaminidase, which breaks down the GM2 ganglioside lipid.

Another example is phenylketonuria (PKU), a recessively inherited disorder whose sufferers lack the ability to synthesize an enzyme to convert the amino acid phenylalanine into tyrosine. Individuals homozygous recessive for this allele have a buildup of phenylalanine and abnormal breakdown products in the urine and blood.

Hemophilia is a group of diseases in which blood does not clot normally. Factors in blood are involved in clotting. Hemophiliacs lacking the normal Factor VIII are said to have Hemophilia A, and those who lack Factor IX have hemophilia B. These genes are carried on the X chromosome, so primers and probes may be used in the present method to detect whether or not a fetus inherited the mother's defective X chromosome, or the father's normal allele.

In some cases, the genetic target is a gene, or portion of a gene, e.g., CFTR, Factor VIII (F8 gene), beta globin, hemachromatosis, G6PD, neurofibromatosis, GAPDH, beta amyloid, or pyruvate kinase gene.

In some embodiments, the genetic target is any sequence whose copy number variation may be associated with a disease or disorder. Other diseases arising from genetic abnormalities include Achondroplasia, Adrenoleukodystrophy, X-Linked, Agammaglobulinemia, X-Linked, Alagille Syndrome, Alpha-Thalassemia X-Linked Mental Retardation Syndrome, Alzheimer Disease, Alzheimer Disease, Early-Onset Familial, Amyotrophic Lateral Sclerosis Overview, Androgen Insensitivity Syndrome, Angelman Syndrome, Ataxia Overview, Hereditary, Ataxia-Telangiectasia, Becker Muscular Dystrophy also The Dystrophinopathies), Beckwith-Wiedemann Syndrome, Beta-Thalassemia, Biotinidase Deficiency, Branchiootorenal Syndrome, BRCA1 and BRCA2 Hereditary Breast/Ovarian Cancer, Breast Cancer, CADASIL, Canavan Disease, Cancer, Charcot-Marie-Tooth Hereditary Neuropathy, Charcot-Marie-Tooth Neuropathy Type 1, Charcot-Marie-Tooth Neuropathy Type 2, Charcot-Marie-Tooth Neuropathy Type 4, Charcot-Marie-Tooth Neuropathy Type X, Cockayne Syndrome, Colon Cancer, Contractural Arachnodactyly, Congenital, Cranio synostosis Syndromes (FGFR-Related), Cystic Fibrosis, Cystinosis,, Deafness and Hereditary Hearing Loss, DRPLA (Dentatorubral-Pallidoluysian Atrophy), DiGeorge Syndrome (also 22q1 Deletion Syndrome), Dilated Cardiomyopathy, X-Linked, Down Syndrome (Trisomy 21), Duchenne Muscular Dystrophy (also The Dystrophinopathies), Dystonia, Early-Onset Primary (DYT1), Dystrophinopathies, The, Ehlers-Danlos Syndrome, Kyp ho scoliotic Form, Ehlers-Danlos Syndrome, Vascular Type, Epidermolysis Bullosa Simplex, Exostoses, Hereditary Multiple, Facioscapulohumeral Muscular Dystrophy, Factor V Leiden Thrombophilia, Familial Adenomatous Polyposis (FAP), Familial Mediterranean Fever, Fragile X Syndrome, Friedreich Ataxia, Frontotemporal Dementia with Parkinsonism-17, Galactosemia, Gaucher Disease, Hemochromatosis, Hereditary, Hemophilia A, Hemophilia B, Hemorrhagic Telangiectasia, Hereditary 55, Hearing Loss and Deafness, Nonsyndromic, DFNA (Connexin 26), Hearing Loss and Deafness, Nonsyndromic, DFNB 1 (Connexin 26), Hereditary Spastic Paraplegia, Hermansky-Pudlak Syndrome, Hexosaminidase A Deficiency (also Tay-Sachs), Huntington Disease, Hypochondroplasia, Ichthyosis, Congenital, Autosomal Recessive, Incontinentia Pigmenti, Kennedy Disease (also Spinal and Bulbar Muscular Atrophy), Krabbe Disease, Leber Hereditary Optic Neuropathy, Lesch-Nyhan Syndrome Leukemias, Li-Fraumeni Syndrome, Limb-Girdle Muscular Dystrophy, Lipoprotein Lipase Deficiency, Familial, Lissencephaly, Marfan Syndrome, MELAS (Mitochondrial Encephalomyopathy, Lactic Acidosis, and, Stroke-Like Episodes), Monosomies, Multiple Endocrine Neoplasia Type 2, Multiple Exostoses, Hereditary Muscular Dystrophy, Congenital, Myotonic Dystrophy, Nephrogenic Diabetes Insipidus, Neurofibromatosis 1, Neurofibromatosis 2" Neuropathy with Liability to Pressure Palsies, Hereditary, Niemann-Pick Disease Type C, Nijmegen Breakage Syndrome Norrie Disease, Oculocutaneous Albinism Type 1, Oculopharyngeal Muscular Dystrophy, Ovarian Cancer, Pallister-Hall Syndrome, Parkin Type of Juvenile Parkinson Disease, Pelizaeus-Merzbacher Disease, Pendred Syndrome, Peutz-Jeghers Syndrome Phenylalanine Hydroxylase Deficiency, Prader-Willi Syndrome, PROP 1-Related Combined Pituitary Hormone Deficiency (CPHD), Prostate Cancer, Retinitis Pigmentosa, Retinoblastoma, Rothmund-Thorns on Syndrome, Smith-Lemli-Opitz Syndrome, Spastic Paraplegia, Hereditary, Spinal and Bulbar Muscular Atrophy (also Kennedy Disease), Spinal Muscular Atrophy, Spinocerebellar Ataxia Type 1, Spinocerebellar Ataxia Type 2, Spinocerebellar Ataxia Type 3, Spinocerebellar Ataxia Type 6, Spinocerebellar Ataxia Type 7, Stickler Syndrome (Hereditary Arthroophthalmopathy), Tay-Sachs (also GM2 Gangliosidoses), Trisomies, Tuberous Sclerosis Complex, Usher Syndrome Type I. Usher Syndrome Type II, Velocardiofacial Syndrome (also 22q1 Deletion Syndrome), Von Hippel-Lindau Syndrome, Williams Syndrome, Wilson Disease, X-Linked Adreno leukodystrophy, X-Linked Agammaglobulinemia, X-Linked Dilated Cardiomyopathy (also The Dystrophinopathies), and X-Linked Hypotonic Facies Mental Retardation Syndrome.

### Droplet Generation

The present disclosure includes compositions and methods for the detection of fetal genetic material using droplet digital PCR. The droplets described herein include emulsion compositions (or mixtures of two or more immiscible fluids) described in US Patent No. 7,622,280, and droplets generated by devices described in International Application No. PCT/US2009/005317, filed 9-23-2009, first inventor: Colston. The term emulsion, as used herein, refers to a mixture of immiscible liquids (such as oil and water). Oil-phase and/or water-in-oil emulsions allow for the compartmentalization of reaction mixtures within aqueous droplets. In preferred embodiments, the emulsions comprise aqueous droplets within a continuous oil phase. In other cases, the emulsions provided herein are oil-in-water emulsions, wherein the droplets are oil droplets within a continuous aqueous phase. The droplets provided herein are designed to prevent mixing between compartments, with each compartment protecting its contents from evaporation and coalescing with the contents of other compartments.

The mixtures or emulsions described herein may be stable or unstable. In preferred embodiments, the emulsions are relatively stable and have minimal coalescence. Coalescence occurs when small droplets combine to form progressively larger ones. In some cases, less than .00001%,.00005%,.00010%,.00050%,.001%,, .005%, .01%, .05%, .1%, .5%, 1%, 2%. 2.5%, 3%, 3.5%, 4%, 4.5%, 5%, 6%, 7%, 8%, 9%, or 10% of droplets generated from a droplet generator coalesce with other droplets. The emulsions may also have limited flocculation, a process by which the dispersed phase comes out of suspension in flakes.

Splitting a sample into small reaction volumes as described herein, may enable the use of reduced amounts of reagents, thereby lowering the material cost of the analysis. Reducing sample complexity by partitioning also improves the dynamic range of detection, since higher-abundance molecules are separated from low-abundance molecules in different compartments, thereby allowing lower-abundance molecules greater proportional access to reaction reagents, which in turn enhances the detection of lower-abundance molecules.

In some cases, droplets may be generated having an average diameter of about .001, .01, .05, .1, 1, 5, 10, 20, 30, 40, 50, 60, 70, 80, 100, 120, 130, 140, 150, 160, 180, 200, 300, 400, or 500 microns. Microfluidic methods of producing emulsion droplets using microchannel cross-flow focusing or physical agitation are known to produce either monodisperse or polydisperse emulsions. In some embodiments, the droplets are monodisperse droplets. In some cases, the droplets are generated such that the size of said droplets does not vary by more than plus or minus 5% of the average size of said droplets. In some cases, the droplets are generated such that the size of said droplets does not vary by more than plus or minus 2% of the average size of said droplets. In some cases, a droplet generator will generate a population of droplets from a single sample, wherein none of the droplets vary in size by more than plus or minus .1%, .5%, 1%, 1.5%, 2%, 2.5%, 3%, 3.5%, 4%, 4.5%, 5%, 5.5%, 6%, 6.5%, 7%, 7.5%, 8%, 8.5%, 9%, 9.5%, or 10% of the average size of the total population of droplets.

Higher mechanical stability is useful for microfluidic manipulations and higher-shear fluidic processing (e.g. in microfluidic capillaries or through 90 degree turns, such as valves, in fluidic path). Pre- and post- thermally treated droplets or capsules are mechanically stable to standard pipet manipulations and centrifugation.

In some cases, the droplet is formed by flowing an oil phase through an aqueous sample. In some preferred embodiments, the aqueous phase comprises a buffered solution and reagents for performing a PCR reaction, including nucleotides, primers, probe(s) for fluorescent detection, template nucleic acids, DNA polymerase enzyme, and optionally, reverse transcriptase enzyme.

In some cases, the aqueous phase comprises a buffered solution and reagents for performing a PCR reaction without solid-state beads, such as magnetic-beads. In some cases, the buffered solution may comprise about 1, 5, 10, 15, 20, 30, 50, 100, or 200 mM Tris. In some cases, the concentration of potassium chloride may be about 10, 20, 30, 40, 50, 60, 80, 100, 200 mM. In one preferred embodiment, the buffered solution comprises 15 mM Tris and 50 mM KCl. In some cases, the nucleotides comprise deoxyribonucleotide triphosphate molecules, including dATP, dCTP, dGTP, dTTP, in concentrations of about 50, 100, 200, 300, 400, 500, 600, or 700 µM each. In some cases dUTP is added within the aqueous phase to a concentration of about 50, 100, 200, 300, 400, 500, 600, or 700, 800, 900, or 1000 µM. In some cases, magnesium chloride (MgCl2) is added to the aqueous phase at a concentration of about 1.0, 2.0, 3.0, 4.0, or 5.0 mM. In one preferred embodiment, the concentration of MgCl2 is 3.2mM

A non-specific blocking agent such as BSA or gelatin from bovine skin may be used, wherein the gelatin or BSA is present in a concentration range of approximately 0.1-0.9% w/v. Other possible blocking agents may include betalactoglobulin, casein, dry milk, or other common blocking agents. In some cases, preferred concentrations of BSA and gelatin are 0.1% w/v.

Primers for amplification within the aqueous phase may have a concentration of about 0.1, 0.2, 0.3, 0.4, 0.5, 0.6, 0.7, 0.8, 0.9, or 1.0 µM. In one preferred embodiment, the concentration of primers is 0.5 µM. In some cases, the aqueous phase comprises one or more probes for fluorescent detection, at a concentration of about 0.1, 0.2, 0.3, 0.4, or 0.5 µM. In one preferred embodiment, the concentration of probes for fluorescent detection is 0.25 µM. Amenable ranges for target nucleic acid concentrations in PCR are between about 1 pg and about 500 ng.

In some embodiments, the aqueous phase may also comprise additives including, but not limited to, non-specific background/blocking nucleic acids (e.g., salmon sperm DNA), biopreservatives (e.g. sodium azide), PCR enhancers (e.g. Betaine, Trehalose, etc.), and inhibitors (e.g. RNAse inhibitors).

In some cases, a non-ionic Ethylene Oxide/Propylene Oxide block copolymer is added to the aqueous phase in a concentration of about 0.1%, 0.2%, 0.3%, 0.4%, 0.5%, 0.6%, 0.7%, 0.8%, 0.9%, or 1.0%. Common biosurfactants include non-ionic surfactants such as Pluronic F-68, Tetronics, Zonyl FSN. In one preferred embodiment, Pluronic F-68 is present at a concentration of 0.5% w/v.

In some cases magnesium sulfate may be substituted for magnesium chloride, at similar concentrations. A wide range of common, commercial PCR buffers from varied vendors may be substituted for the buffered solution.

The oil phase may comprise a fluorinated base oil which may be additionally stabilized by combination with a fluorinated surfactant such as a perfluorinated polyether. In some cases, the base oil may be one or more of HFE 7500, FC-40, FC-43, FC-70, or another common fluorinated oil. In some cases, the anionic surfactant is Ammonium Krytox (Krytox-AM), the ammonium salt of Krytox FSH, or morpholino derivative of Krytox-FSH. Krytox-AS may be present at a concentration of about 0.1%, 0.2%, 0.3%, 0.4%, 0.5%, 0.6%, 0.7%, 0.8%, 0.9%, 1.0%, 2.0%, 3.0%, or 4.0% w/w. In some preferred embodiments, the concentration of Krytox-AS is 1.8%. In other preferred embodiments, the concentration of Krytox-AS is 1.62%. Morpholino derivative of Krytox-FSH may be present at a concentration of about 0.1%, 0.2%, 0.3%, 0.4%, 0.5%, 0.6%, 0.7%, 0.8%, 0.9%, 1.0%, 2.0%, 3.0%, or 4.0% w/w. In some preferred embodiments, the concentration of morpholino derivative of Krytox-FSH is 1.8%. In some preferred embodiments, the concentration of morpholino derivative of Krytox-FSH is 1.62%.

The oil phase may further comprise an additive for tuning the oil properties, such as vapor pressure or viscosity or surface tension. Nonlimiting examples include perfluoro-octanol and 1H,1H,2H,2H-Perfluorodecanol. In some preferred embodiments, 1H,1H,2H,2H-Perfluorodecanol is added to a concentration of about 0.05%, 0.06%, 0.07%, 0.08%, 0.09%, 1.00%, 1.25%, 1.50%, 1.75%, 2.00%, 2.25%, 2.50%, 2.75%, or 3.00% w/w. In some preferred embodiments, 1H,1H,2H,2H-Perfluorodecanol is added to a concentration of 0.18% w/w.

In some embodiments, the emulsion is formulated to produce highly monodisperse droplets having a liquid-like interfacial film that can be converted by heating into microcapsules having a solid-like interfacial film; such microcapsules may behave as bioreactors able to retain their contents through a reaction process such as PCR ampflication. The conversion to microcapsule form may occur upon heating. For example, such conversion may occur at a temperature of greater than about 50, 60, 70, 80, 90, or 95 degrees Celsius. In some cases this heating occurs using a thermocycler. During the heating process, a fluid or mineral oil overlay may be used to prevent evaporation. Excess continuous phase oil may or may not be removed prior to heating. The biocompatible capsules may be resistant to coalescence and/or flocculation across a wide range of thermal and mechanical processing.

Following conversion, the capsules may be stored at about 3, 4, 5, 6, 7, 8, 9, 10, 15, 20, 25, 30, 35, or 40 degrees, with one preferred embodiment comprising storage of capsules at less than about 25 degrees. In some embodiments, these capsules are useful in biomedical applications, such as stable, digitized encapsulation of macromolecules, particularly aqueous biological fluids containing a mix of nucleic acids or protein, or both together; drug and vaccine delivery; biomolecular libraries; clinical imaging applications, and others.

The microcapsules may contain one or more nucleic acid probes (e.g., molecular inversion probe, ligation probe, etc.) and may resist coalescence, particularly at high temperatures. Accordingly, PCR amplification reactions may occur at a very high density (e.g., number of reactions per unit volume). In some cases, greater than 100,000, 500,000, 1,000,000, 1,500,000, 2,000,000, 2,500,000, 5,000,000, or 10,000,000 separate reactions may occur per ml. In some cases, the reactions occur in a single well, e.g.., a well of a microtiter plate, without intermixing between reaction volumes. The microcapsules may also contain other components necessary to enable a PCR reaction to occur, e.g., primers, probes, dNTPs, DNA or RNA polymerases, etc. These capsules exhibit resistance to coalescence and flocculation across a wide range of thermal and mechanical processing.

### Role of Devices

A variety of devices may be used to effectuate the methods described herein. FIG. 3 depicts a workflow of an exemplary method for diagnosing fetal aneuploidy and highlights some devices that may be used in the methods herein. A maternal tissue sample containing maternal and fetal genetic material (201) is obtained. DNA is extracted from the sample, and bound to probes recognizing chromosome 1 (202) and 21 (203), which then undergo a ligation reaction. A sample comprising ligated probes (as well as components necessary for a PCR reaction) is introduced into a droplet generator (301), which partitions the probes into multiple droplets within a water-in-oil emulsion. Examples of some droplet generators useful in the present disclosure are provided in International Application No. PCT/US2009/005317, filed 9-23-2009, first inventor: Colston. Droplets are then incubated in a thermocycler (302) to allow amplification of the probes. During the amplification reaction, a droplet comprising an amplified probe experiences an increase in fluorescence relative to droplets that do not contain amplified probe. The droplets are then processed individually through a droplet reader (303), and data is collected to detect fluorescence. Examples of some droplet readers useful in the present disclosure are provided in International Application No. PCT/US2009/005317, filed 9-23-2009, first inventor: Colston.

As depicted in FIG. 3, data relating to the copy number of chromosome 1 and 21 is then compared in order to detect fetal aneuploidy. Often, the data is analyzed using an algorithm applied by a device such as a computer. In some cases, the droplet generator, thermocycler, droplet reader, and computer are each a separate device. In other cases, one device comprises two or more of such devices, in any combination. For example, one device may comprise a droplet generator in communication with a thermocycler. In other cases, a device may comprise a droplet generator, thermocycler, and droplet reader.

The present disclosure provides means for rapid, efficient and sensitive detection of copy number and/or detection of copy number variations (e.g., fetal aneuploidy). The present disclosure is particularly useful for identifying changes in copy number of polynucleotides present in rare amounts within a genetic sample (e.g., fetal polynucleotides within a sample of maternal blood). In some cases, less than .00001,.00005,.00010,.00050,.001,, .005, .01, .05, .1, .5, 1, 2, 2.5, 3, 3.5, 4, 4.5, 5, 6, 7, 8, 9, or 10 copies of target polynucleotide are detected. In some cases, less than 1, 2, 3, 4, 5, 6, 7, 8, 9, 10, 11, 12, 13, 14, 15, 20, 25, 30, 35, 40, 45, 50, 55, 60, 65, 70, 75, 80, 85, 90, 95, 100, 150, 200, 250, 300, 350, 400, 450, or 500 copies of a target polynucleotide are detected.. In some cases, the droplets described herein are generated at a rate of greater than 1, 2, 3, 4, 5, 10, 50, 100, 200, 300, 400, 500, 600, 700, 800, 900, or 1000 droplets/second.

### Amplification

Techniques for amplification of target and reference sequences (as well as sequences within ligation probes) are known in the art, and include the methods described in US Patent No. 7,048,481. Briefly, the techniques include methods and compositions that separate samples into small droplets, in some instances with each containing on average less than one nucleic acid molecule per droplet, amplifying the nucleic acid sequence in each droplet and detecting the presence of a particular target sequence. In some cases, the sequence that is amplified is present on a probe to the genomic DNA, rather than the genomic DNA itself.

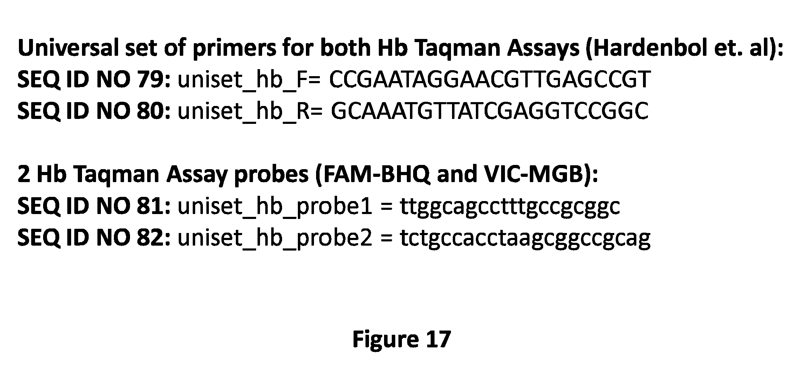

Primers are designed according to known parameters for avoiding secondary structures and self-hybridization. In some embodiments, different primer pairs will anneal and melt at about the same temperatures, for example, within 1, 2, 3, 4, 5, 6, 7, 8, 9 or 10°C of another primer pair. In some cases, only ligatable probes, and no primers, are initially added to genomic DNA, followed by partitioning the ligated probes, followed by amplification of one or more sequences on the probe within each partition using, for example, universal primers. In some cases, greater than 1, 2, 3, 4, 5, 6, 7, 8, 9, 10, 15, 20, 25, 30, 35, 40, 45, 50, 100, 200, 500, 1000, 5000, 10,000 or more probes are initially used. Such probes may be able to hybridize to the genetic targets described herein. For example, a mixture of probes can be used, wherein at least one probe targets a specific site on a chromosome and a second probe targets a different site on the same chromosome or a different chromosome. Each set of ligatable probes can have its own universal probe set and be distinguished by the corresponding TaqMan probe for each set. Or, all ligatable probe sets can use the same universal primer set and be distinguished by the corresponding TaqMan probe for each set. Exemplary sequences for universal primers bearing no homology to human genomic DNA include SEQ ID NOS: 79 and 80 (FIG. 17).

While the preferred embodiment of the disclosure is described in terms of PCR, the disclosure is primarily directed to the use of multiple individual genetic sequence detections. In some embodiments, the method of amplification can be, for example, a self-sustained sequence reaction, ligase chain reaction, rapid amplification of cDNA ends, and polymerase chain reaction, Q-beta phage amplification, strand displacement amplification, isothermal amplification or splice overlap extension polymerase chain reaction.

Primers can be prepared by a variety of methods including but not limited to cloning of appropriate sequences and direct chemical synthesis using methods well known in the art (Narang et al., Methods Enzymol. 68:90 (1979); Brown et al., Methods Enzymol. 68:109 (1979)). Primers can also be obtained from commercial sources such as Operon Technologies, Amersham Pharmacia Biotech, Sigma, and Life Technologies. The primers can have an identical melting temperature. The lengths of the primers can be extended or shortened at the 5' end or the 3' end to produce primers with desired melting temperatures. In a preferred embodiment, one of the primers of the prime pair is longer than the other primer. In a preferred embodiment, the 3' annealing lengths of the primers, within a primer pair, differ. Also, the annealing position of each primer pair can be designed such that the sequence and length of the primer pairs yield the desired melting temperature. The simplest equation for determining the melting temperature of primers smaller than 25 base pairs is the Wallace Rule (Td=2(A+T)+4(G+C)). Computer programs can also be used to design primers, including but not limited to Array Designer Software (Arrayit Inc.), Oligonucleotide Probe Sequence Design Software for Genetic Analysis (Olympus Optical Co.), NetPrimer, and DNAsis from Hitachi Software Engineering. The TM (melting or annealing temperature) of each primer is calculated using software programs such as Net Primer (free web based program at http://premierbio soft. com/netprimer/netprlaunch/netprlaunch.html; internet address as of Apr. 17, 2002). In another embodiment, the annealing temperature of the primers can be recalculated and increased after any cycle of amplification, including but not limited to cycle 1, 2, 3, 4, 5, cycles 6-10, cycles 10-15, cycles 15-20, cycles 20-25, cycles 25-30, cycles 30-35, or cycles 35-40. After the initial cycles of amplification, the 5' half of the primers is incorporated into the products from each loci of interest, thus the TM can be recalculated based on both the sequences of the 5' half and the 3' half of each primer.

In some preferred embodiments, desired sequences that may include target and reference sequences are represented by template MIPs, which are formerly-circularized MIPs that have been isolated and linearized as described above. Template MIPs serve as template molecules in PCR. In some cases, template MIPs are produced prior to droplet generation, and in other cases, template MIPs are produced during or following droplet generation. In an example of the last case, a circular MIP containing abasic sites resulting from uracil-N-deglycosylase treatment of uracil bases undergoes a spontaneous ring-opening reaction upon heating in a melting step of a PCR reaction in a thermocycler. In some cases, template MIPs serve as DNA templates for droplet digital PCR, wherein amplification of the template MIP corresponds to detection of the desired sequence that the MIP represents (e.g. a target or reference sequence). In some embodiments, the method involves producing a droplet for a droplet digital PCR reaction by flowing an immiscible liquid in a sample fluid, wherein the sample fluid comprises one or more MIPs or one or more template MIPs, and a master mix containing reagents necessary for PCR. In some preferred embodiments, a master mix for PCR comprises a thermostable polymerase enzyme, universal primers for template MIP amplification, free DNA nucleotides for incorporation, and buffer components for the reaction. The thermostable polymerase enzyme may retain activity when exposed to temperatures greater than 99, 98, 97, 96, 95, 94, 93, 92, 91, 90, 80, 70 degrees or less. In some cases, the sample fluid additionally comprises digested genomic DNA or inactivated enzymes such as endonucleases and/or deglycosylases retained from MIP template generation. In some embodiments, the method involves generating droplets comprising less than one, one, or more than one genome equivalents of DNA represented by MIPs or MIP templates.

In some embodiments, desired sequences that may include target and reference sequences are present as part of a mixture containing unwanted background genomic DNA. In some cases, only desired sequences, and not background genomic DNA sequences, are detected using ligation detection reaction and droplet digital PCR (e.g., in cases where only ligation products are competent to form detectable products in PCR using a master mix comprising universal primers). In other cases, desired sequences are detected in droplet digital PCR using sequence-specific primers.

In some preferred embodiments, the present disclosure involves compositions comprising emulsions comprising an average of about one genome equivalent of DNA that can be used to detect fetal genetic material. In some cases, one or more MIPs or MIP templates represent a sequence of interest (such as a region of chromosome 21) whose detection can enable determination of fetal aneuploidy. In some cases, a composition containing a sequence of interest representing a genetic target that may be associated with a genetic abnormality (such as trisomy) can be compared to a composition containing a sequence representing a reference sequence that may not be associated with a genetic abnormality. In some cases, sensitivity of detection may be enhanced through multiplexing of probes directed to a genetic target. Furthermore, multiple genetic targets may be examined in parallel using multiple simultaneous detection modes, such as different colors in the fluorescence detection methods detailed below.

In some embodiments, genetic targets may include any nucleic acid molecules that can be represented by ligation products such as MIPs, MIP templates, or ligated probes. These ligation products are present in a sample fluid in which an immiscible liquid is flowed to generate a droplet. Reagents necessary for PCR may also be contained in the droplet, for subsequent droplet digital PCR. Examples of genetic targets that may be analyzed herein include genetic variations, such as aneuploidy, mutations, insertions, additions, deletions, translocation, point mutation, trinucleotide repeat disorders and/or single nucleotide polymorphisms (SNPs), that may not be associated with fetal genetic abnormalities.

The annealing temperature of the primers can be recalculated and increased after any cycle of amplification, including but not limited to cycle 1, 2, 3, 4, 5, cycles 6-10, cycles 10-15, cycles 15-20, cycles 20-25, cycles 25-30, cycles 30-35, or cycles 35-40. After the initial cycles of amplification, the 5' half of the primers is incorporated into the products from each loci of interest, thus the TM can be recalculated based on both the sequences of the 5' half and the 3' half of each primer. Any DNA polymerase that catalyzes primer extension can be used including but not limited to E. coli DNA polymerase, Klenow fragment of E. coli DNA polymerase 1, T7 DNA polymerase, T4 DNA polymerase, Taq polymerase, Pfu DNA polymerase, Vent DNA polymerase, bacteriophage 29, REDTaq™. Genomic DNA polymerase, or sequenase. Preferably, a thermostable DNA polymerase is used. A hot start PCR can also be performed wherein the reaction is heated to 95°C. for two minutes prior to addition of the polymerase or the polymerase can be kept inactive until the first heating step in cycle 1. Hot start PCR can be used to minimize nonspecific amplification. Any number of PCR cycles can be used to amplify the DNA, including but not limited to 2, 5, 10, 15, 20, 25, 30, 35, 40, or 45 cycles.

Amplification of target nucleic acids (e.g., ligation probes, MIP probes) can be performed by any means known in the art. In some cases, target nucleic acids are amplified by polymerase chain reaction (PCR). Examples of PCR techniques that can be used include, but are not limited to, quantitative PCR, quantitative fluorescent PCR (QF-PCR), multiplex fluorescent PCR (MF-PCR), real time PCR(RT-PCR), single cell PCR, restriction fragment length polymorphism PCR (PCR-RFLP), PCR-RFLP/RT-PCR-RFLP, hot start PCR, nested PCR, in situ polonony PCR, in situ rolling circle amplification (RCA), bridge PCR, picotiter PCR and emulsion PCR. Other suitable amplification methods include the ligase chain reaction (LCR), transcription amplification, self-sustained sequence replication, selective amplification of target polynucleotide sequences, consensus sequence primed polymerase chain reaction (CP-PCR), arbitrarily primed polymerase chain reaction (AP-PCR), degenerate oligonucleotide-primed PCR (DOP-PCR) and nucleic acid based sequence amplification (NABSA). Other amplification methods that can be used herein include those described in U.S. Pat. Nos. 5,242,794; 5,494,810; 4,988,617; and 6,582,938. In some embodiments, amplification of target nucleic acids may occur on a bead. In other embodiments, amplification does not occur on a bead.
In some cases, thermocycling reactions are performed on samples contained in droplets. In some preferred cases, the droplets remain intact during thermocycling. Droplets may remain intact during thermocycling at densities of greater than about 10,000 droplets/mL, 100,000droplets/mL, 200,000droplets/mL, 300,000droplets/mL, 400,000droplets/mL, 500,000droplets/mL, 600,000droplets/mL, 700,000droplets/mL, 800,000droplets/mL, 900.000droplets/mL or 1,000,000droplets/mL.In other cases, two or more droplets may coalesce during thermocycling. In other cases, greater than 100 or greater than 1,000 droplets may coalesce during thermocycling.

### Detection and Analysis

Detection of PCR products can be accomplished using fluorescence techniques. DNA-intercalating dyes such as ethidium bromide or SYBR green that increases fluorescence upon binding DNA can provide a quantitative readout of the amount of DNA present in a reaction volume. As this amount of DNA increases over the course of a reaction, the fluorescence intensity increases. Methods involving DNA-intercalating dyes are susceptible to background fluorescence since they do not measure DNA in a sequence-specific manner, and do not distinguish between reaction products and other molecules such as primer dimers. A method for detecting PCR products that provides sequence specificity involves probes that contain a fluorescer-quencher pair and hybridize to a specific sequence. The fluorescer may be any molecule emitting detectable light such as a fluorophore, and the quencher may be any molecule that absorbs this emission, reducing the intensity of emission by the fluorescer. When present in a solution containing a complementary sequence, the fluorescer-quencher probe binds to the sequence. During a PCR reaction, a polymerase such as Taq can use this probe as a primer, and the probe is cleaved by a 5'-->3' exonuclease activity that functions in cells to excise RNA primers. In the case of PCR reactions using synthetic fluorescer-quencher probes as primers, the 5'→3' exonuclease activity causes the probes to be cleaved, resulting in separation of the fluorescer from the quencher. Once it is no longer covalently attached to the quencher, the fluorescence emission from the fluorescer can be detected.

A preferred embodiment of the present disclosure involves detecting droplet digital PCR products produced using MIP templates. In some cases, detection occurs via cleavage of a fluorescer-quencher probe that binds a sequence that is specific to the MIP, distinct from the genetic target. This strategy allows the use of universal fluorescer-quencher probes that detect MIPs without requiring sequence specificity to the genetic target represented by the MIP.

In some embodiments, molecular beacon (MB) probes, which become fluorescent on binding to the target sequence(s) may be used. MB probes are oligonucleotides with stem-loop structures that contain a fluorescer at the 5' end and a quencher at the 3' end. The degree of quenching via fluorescence energy resonance transfer is inversely proportional to the 6th power of the distance between the quencher and the fluorescer. After heating and cooling, MB probes reform a stem-loop structure, which quenches the fluorescent signal from the fluorescer. If a PCR product whose sequence is complementary to the loop sequence is present during the heating/cooling cycle, hybridization of the MB to one strand of the PCR product will increase the distance between the quencher and the fluorescer, resulting in increased fluorescence.

In some embodiments, detection occurs through the use of universal probes. A universal fluorescer probe (UFP) contains a fluorescent molecule that emits a detectable electromagnetic radiation upon absorbing electromagnetic radiation in a range of wavelengths. A universal quencher probe (UQP) contains a quencher molecule that reduces the intensity of fluorescent emission of a proximal fluorescer probe. In one case, a universal fluorescer probe contains a nucleic acid segment that hybridizes to a complementary nucleic acid segment on a universal quencher probe or a complementary nucleic acid segment within a target sequence, such as a MIP. During PCR, amplification of such a target sequence results in increased binding of a universal fluorescer probe to a target sequence, compared to a quencher probe, which results in increased detectable fluorescence. In some cases, the length of complementary sequence between a universal fluorescer probe and a universal quencher probe may be varied to modulate the melting temperature of the complex of universal fluorescer probe bound to universal quencher probe. In some cases, the length of the complementary sequence may be 15 base pairs. In some cases, the length of the complementary sequence may be more than about 5, 10, 15, 20, 25, 30, 35, 40, 45, 50, 55, 60, 70, or 80 base pairs. The melting temperature of the complex of universal fluorescer probe bound to universal quencher probe may be greater than about 40, 45, 50, 55, 60, 65, 70, 75, 80, 85, 90, or 95 degrees Celsius.

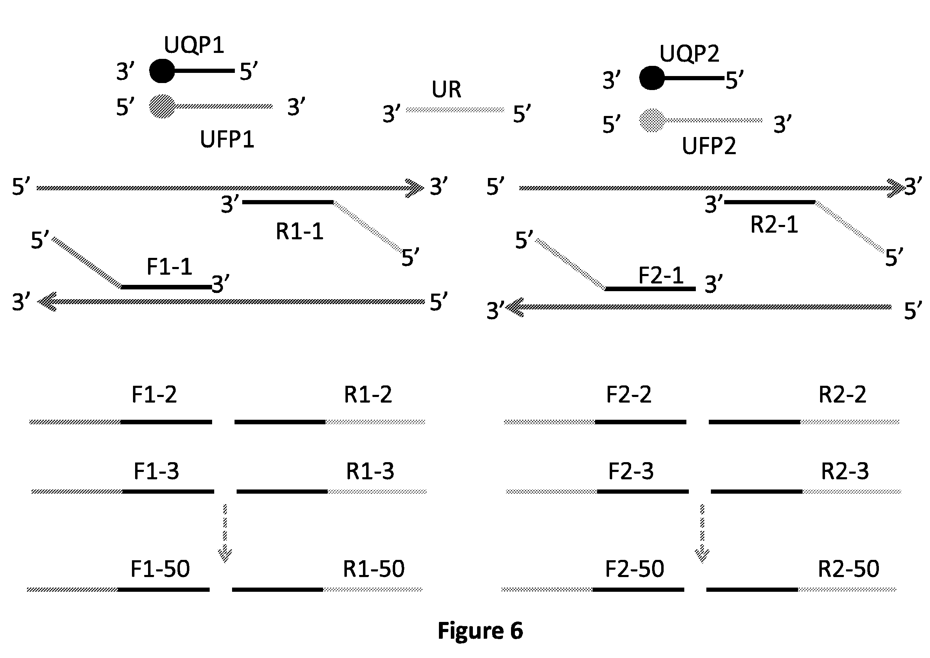

FIG. 6 shows a two-color system for detection of nucleic acids in droplets using universal primers and universal probes without cleavage. A universal probe comprises two complementary oligonucleotides, one fluorescer probe containing a fluorescent molecule (UFP1 or UFP2) and one quencher probe containing a quenching molecule (UQP1 or UQP2). UFP1 and UFP2 fluoresce at different colors and are distinguishable in detection. When bound to the quencher probe, the fluorescence intensity of the fluorescer probe is substantially reduced. Additionally, two pairs of universal forward and reverse primers contain regions that are complementary to the fluorescer probe and promote PCR amplification of a target sequence. In the first round of amplification, the region complementary to the fluorescer probe is incorporated via the universal primers into the template. In subsequent rounds of amplification, the fluorescer probes UFP1 or UFP2 can therefore hybridize to this template, rather than to their respective quencher probes. As more of these templates are generated exponentially by amplification reactions, UFP1-UQP1 and UFP2-UQP2 complexes are replaced by UFP1-template and UFP2-template complexes through competitive binding. As a result of this separation between fluorescer probe and quencher probe, fluorescence intensity will increase in the reaction, and can be detected in following steps.

Universal probes may be designed by methods known in the art. In some embodiments, the probe is a random sequence. The universal probe may be selected to ensure that it does not bind the target polynucleotide in an assay, or to other non-target polynucleotides likely to be in a sample (e.g., genomic DNA outside the region occupied by the target polynucleotide). Exemplary sequences for universal probes include SEQ ID NOS: 81 and 82.

Fluorescence detection can be achieved using a variety of detector devices equipped with a module to generate excitation light that can be absorbed by a fluorescer, as well as a module to detect light emitted by the fluorescer. In some cases, samples (such as droplets) may be detected in bulk. For example, samples may be allocated in plastic tubes that are placed in a detector that measures bulk fluorescence from plastic tubes. In some cases, one or more samples (such as droplets) may be partitioned into one or more wells of a plate, such as a 96-well or 384-well plate, and fluorescence of individual wells may be detected using a fluorescence plate reader.

In some cases, the detector further comprises handling capabilities for droplet samples, with individual droplets entering the detector, undergoing detection, and then exiting the detector. For example, a flow cytometry device can be adapted for use in detecting fluorescence from droplet samples. In some cases, a microfluidic device equipped with pumps to control droplet movement is used to detect fluorescence from droplets in single file. In some cases, droplets are arrayed on a two-dimensional surface and a detector moves relative to the surface, detecting fluorescence at each position containing a single droplet.

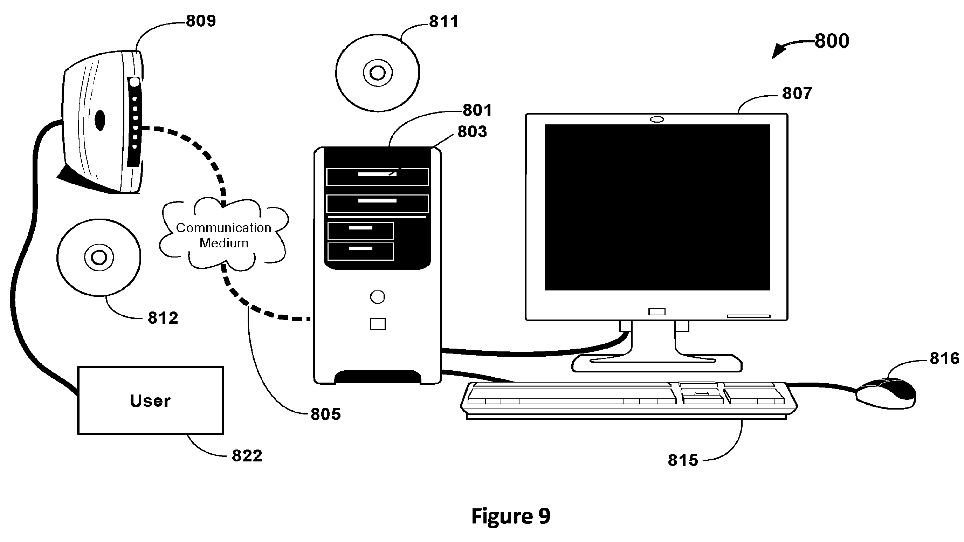

Following acquisition of fluorescence detection data, a computer is used in some cases to store and process the data. A computer-executable logic may be employed to perform such functions as subtraction of background fluorescence, assignment of target and/or reference sequences, and quantification of the data. For example, the number of droplets containing fluorescence corresponding to the presence of an suspected aneuploid chromosome (such as chromosome 21) in the sample may be counted and compared to the number of droplets containing fluorescence corresponding to the presence of chromosome not suspected to be aneuploidy (such as chromosome 1). FIG. 9 depicts a computer useful for displaying, storing, retrieving, or calculating diagnostic results from the molecular profiling; displaying, storing, retrieving, or calculating raw data from genomic or nucleic acid expression analysis; or displaying, storing, retrieving, or calculating any sample or patient information useful in the methods described herein.

Following digital PCR of samples having primers to amplify a target and a reference sequence, the number of positive samples having a target sequence and the number of positive samples having a reference sequence can be compared. Since this is a comparison of sequences present in the maternal tissue, there is no need to differentiate between maternal and fetal DNA. When a target sequence contains the same number of copies as a reference sequence known to be diploid, then the sample can be determined to be diploid as well. When the target sequence differs from the reference sequence, then the sample possibly contains an aneuploidy.

In some embodiments, the genomic DNA obtained from a maternal tissue as described above is partitioned into multiple reaction volumes (e.g. droplets), so that there is, on average, less than one genome equivalent (GE) per droplet. In some cases, the droplets contain much more than, on average, one GE per droplet, such as, on average, 1, 2, 3, 4, 5, 6, 7, 8, 9, 10, 15, 20, 25, 30, 35, 30, 45, or 50 GE/droplet. In some cases, a sample will produce greater than on average 1, 5, or 10 GE/droplet, but, nonetheless some of the droplets will contain no GE, or no target polynucleotide. In such cases, it may be necessary to apply an algorithm to calculate the average number of copies/droplet of a particular genetic target. In some cases, the genetic target is actually an entire chromosome (or fragment), that is then fragmented and therefore one copy may appear in multiple droplets.

Often, when individual discrete reaction volumes are analyzed for the presence of a genetic abnormality to be tested, the DNA (chromosomal) to be analyzed may on average, either be present or absent, permitting so-called digital analysis. The collective number of reaction volumes containing a particular target sequence can be compared to a reference sequence for differences in number. A ratio other than normal (e.g., 1:1) between a target sequence and a reference sequence known to be a diploid sequence is indicative of an aneuploidy. For example, a sample can be partitioned into reaction volumes, such as droplets, such that each droplet contains less than a nominal single genome equivalent of DNA. The relative ratio of the target of interest (e.g., a genetic marker for chromosome 21 trisomy, or related probe) to a reference sequence (e.g. known diploid sequence on chromosome 1, or related probe) can be determined by examining a large number of reaction volumes (e.g., droplets), such as 10,000, 20,000, 50,000, 100,000, 200,000, 500,000 or more. In other cases, the reaction volumes, such as droplets, comprise on average one or more target nucleotides (or genomic equivalents) per droplet. In such cases, the average copy number of the target nucleotide may be calculated by applying an algorithm, such as that described in Dube et al. (2008) Plos One 3(8): e2876.

By analyzing a large number of reaction volumes, a change in the relative ratio from 1:1 resulting from the fetal aneuploidy can be measured from a mixture of fetal and maternal DNA in the starting sample, where the relative concentration of fetal DNA is low compared to the maternal DNA. This is termed a digital analysis, because each reaction volume will have, on average, one genome equivalent per reaction volume, and furthermore, the dilution can be read as a binary "yes-no" result as to the presence of the sequence (e.g. target or reference) to be counted.

The methods and compositions described herein can be used in a wide range of applications. In some embodiments, the methods and compositions related to methods for diagnosing, detecting, identifying, predicting, evaluating, or prognosing a condition associated with a genetic disorder. Such condition may due to genetic causes, including genetic disorders, variations, mutations, SNPs, deletions, amplifications, translocations, inversions, or any other abnormality within a specific genetic locus (including any locus provided herein).

The methods and compositions provided herein may be used to diagnose, detect, predict, identify, or otherwise evaluate the risk that a fetus has a genetic abnormality (e.g., Down's Syndrome, fetal aneuploidy, etc.). The methods may also be used to identify, quantify, diagnose, prognose, evaluate, or analyze the risk that an expectant mother will experience issues in pregnancy including miscarriage within the first trimester, second trimester, or third trimester; still birth; birth defects in her infant; pre-term labor, or other issues with labor; and any other condition associated with pregnancy, labor, or the birth of a child.

The methods and compositions provided herein may be used to evaluate the relative copy number of a first polynucleotide (e.g., DNA, RNA, genomic DNA, mRNA, siRNA, miRNA, cRNA, single-stranded DNA, double-stranded DNA, single-stranded RNA, double-stranded RNA, tRNA, rRNA, cDNA, etc.) compared to a second polynucleotide. The methods may be used to analyze the quantity of synthetic plasmids in a solution; to detect the sequence of a pathogenic organism (e.g., bacteria, virus, retrovirus. lentivirus, HIV-1, HIV-2, influenza virus, etc.) within a sample obtained from a subject. The methods also may be used in other applications wherein a rare population of polynucleotides exists within a larger population of polynucleotides.

### Some examples of methods

In some embodiments, the present method comprises generally the following steps:
1. Obtaining a tissue containing DNA from a pregnant subject. In some embodiments, the tissue can be maternal blood (whole blood or peripheral blood), plasma or serum. This material can be drawn blood, and the circulating DNA can be found in the blood plasma, rather than in cells. In some embodiments, the maternal tissue (such as blood or plasma) can be enriched for fetal DNA by known methods, such as size fractionation to select for DNA fragments less than about 300 nucleotides. In some embodiments, maternal DNA, which tends to be larger than about 500 nucleotides can be excluded. In other embodiments, another enrichment step can be used to treat the blood sample with formaldehyde, as described in Dhallan et al. "Methods to Increase the Percentage of Free Fetal DNA Recovered From the Maternal Circulation," (2004) J. Am. Med. Soc. 291:1114-1119. In still other embodiments, the DNA is purified from other material in the sample using methods well-known in the art, such as ethanol precipitation.
   Optionally, sequences of interest in genomic DNA can be captured by specific binding to one or more oligonucleotides, or probes. In some cases, the mixture of genomic DNA and probes is subjected to a ligase reaction that results in selective ligation of probes that are bound to genomic DNA. In some cases, binding to the DNA results in inversion and circularization of a linear probe such as a MIP, and the ligation reaction produces a circular product. In some of these cases, genomic DNA and unbound probe can be removed from the sample using exonuclease treatment.
2. Distributing single DNA molecules from this sample to a number of discrete reaction volumes (e.g. aqueous phase droplets) that are partitioned from the starting sample. In some embodiments, the number of reaction volumes can be selected to give a statistically significant result for the number of copies of a target in the starting sample DNA molecules. The reaction volume can be confined to a small volume to bring the reaction molecules into close proximity which can decrease reaction times. The amount of DNA molecules per reaction volume can be on the order of 0.5, 1, 2, 3, 4, or more DNA molecules per reaction volume. In some cases, the number of DNA molecules per reaction volume is on average about 1 DNA molecule per reaction volume. In some cases, the reaction volume of a single droplet can be up to 100pL, 500pL, 1nL, 10nL or 100nL.
3. Detecting the presence of the target sequence in the DNA with a nucleic acid amplification technique such as a PCR reaction. Each reaction volume (e.g. droplet) can contain all the necessary reagents for performing PCR which are well known in the art. In one embodiment, the maternal tissue sample is partitioned into droplets that are amplified in a continuous flow PCR amplification, such as described in US Patent No. 7,048,481. In some cases, the amplification is of a sequence within a probe that binds to genomic DNA, rather than of the genomic DNA itself. In other embodiments, the maternal tissue sample is partitioned into droplets that are amplified in wells of a thermocycler. In some embodiments, the PCR product can be probed or labeled to give a convenient quantitative read out. For example, a fluorescence signal can be read for one or more sequences in each reaction volume, such as through the use of fluorescence labels, probes or intercalating dyes. The detection step is referred to here as digital PCR and can be carried out by a variety of methods, such as by measuring fluorescence of (a) individual droplets containing PCR products in a flowing stream or in a stopped flow; (b) PCR products from samples diluted into individual wells of a microtiter plate; (c) PCR products from samples diluted into emulsions; or (d) PCR products from samples trapped in a microfluidic chamber; and
4. Quantitative analysis of the detection of the maternal and fetal target sequences. In some cases this may include targets to different regions, such as probes to a target on a chromosome suspected of being present in an abnormal copy number (such as trisomy) compared to a sequence on a normal diploid chromosome, which is used as a reference. The analysis may also involve the detection of ligation products, such as circular MIPs which have been isolated from genomic DNA and unbound probe (for example, using exonuclease treatment) and linearized using enzymatic treatment. In some cases, quantitative determinations are made by measuring the fluorescence intensity of individual partitions, while in other cases, measurements are made by counting the number of partitions containing detectable signal. In some embodiments, control samples can be included to provide background measurements that can be subtracted from all the measurements to account for background fluorescence. In other embodiments, 1, 2, 3, 4, or more than 4 different colors can be used to measure different sequences, such as by using fluorophores of different colors on different PCR primers matched to probes recognizing different sequences.

### EXAMPLES

### Example 1. Detection of fetal DNA using a two-color detection scheme

In this example, detection of a trisomy 21 fetal aneuploidy is described, where there is 3% fetal DNA in a maternal plasma sample. There are 1000 genome equivalents (GE) per mL in the maternal plasma, and a maternal blood volume of 20 mL is collected.

Plasma is isolated from the maternal blood sample by centrifugation, and the nucleic acids are purified and concentrated to a volume of 50 µL. The sample is mixed with an equal volume of PCR reagent containing the multiplexed assay components. The entire 100 µL sample is partitioned into 100,000 aqueous droplets having a volume of 1 nL per droplet. For an ideal positive droplet percentage for quantitation of 75%, this would mean 1.47 copies of target sequence per droplet, based on Poisson distribution, which translates to 147,000 targets that need to be compartmentalized into 100,000 1nL droplets. The number of primer sets required to reach this is 147,000GE/10,000GE, which is a 15 plex. Thus, in each droplet, there would be a 15 plex for each target and reference sequence, or a total of 30 primer sets per droplet. The samples are analyzed using a two-color detection scheme, where the target sequence probes fluoresce using a green emitter and the reference sequence probes fluoresce using a yellow, orange or red emitter. Detection is performed over the 100,000 droplets and the ratio of target (green) to reference (yellow, orange or red) sequence is calculated.

### Example 2. Detection of fetal DNA using a one-color detection scheme.

The conditions for Example 1 are used here, except that rather than using different colored target and reference probes, the sample is split (e.g. in half), then two set of droplets are generated, amplified and separately analyzed, with one half using a target probe and the other half using a reference probe.

### Example 3. Detecting fetal DNA using MIP-ddPCR

Cell-free plasma is isolated from a maternal blood sample by centrifugation. The nucleic acids are then purified and concentrated using a cell free DNA kit (Qiagen). The purified genomic DNA is then mixed with 1000 chromosome-sequence specific oligonucleotide probes (e.g., MIP probe) to Chromosome 21 (MIP-21Chr), and 1000 chromosome-sequence specific oligonucleotide probes (e.g., MIP probe) to Chromosome 1 (MIP-1Chr). Ligase, polymerase and other reaction components are added to the mix. The sample is incubated at 20°C for 4 minutes. The sample is then incubated at 95°C for 5 minutes to promote denaturation, and then at 60°C for 15 minutes in order to promote annealing of the MIP probes to the genomic DNA. A gap fill reaction is then performed in order to circularize the MIP probes. (In some cases, the ends may be directly ligated without a gap fill reaction). Nucleotides are added to the sample, which is then incubated at 60°C for 10 minutes in order to allow binding of the ligase and polymerase to the gap in the MIP probes. The sample is then incubated at 37°C for 1 minute. Next, the sample is treated with Exonuclease I and III in order to digest remaining linear probes and ssDNA such as genomic DNA that is not hybridized to a probe, followed by incubation at 37°C for 14 minutes to promote exonuclease activity, an incubation at 95°C for 2 minutes to inactivate the exonucleases, and, finally, an incubation at 37°C for 1 minute. Uracil-N-glycosylase is next added to the sample, which is incubated at 37°C for 10 minutes in order to promote enzymatic depurination, followed by incubation at 95°C for 20 minutes in order to allow cleavage of abasic depurinated uracil residues in the MIP probes. The linearized probes now have an inverted primer orientation.

Next, droplet digital PCR is performed on the sample. Taq polymerase, universal primers, Taqman fluorescence probes, and PCR reaction components are added to the sample. The Taqman fluorescent probes complementary to the universal probe binding sequence on the MIP-21Chr probe are tagged with a FAM dye; and the Taqman fluorescent probes complementary to the universal probe binding sequence on the MIP-1Chr probe are tagged with a VIC dye. The sample is then emulsified into 100,000 monodisperse-water-in-oil droplets stabilized by surfactant additives into emulsification oil phase and/or aqueous PCR reaction phase. As a result, the sample is partitioned into 100,000 droplets. The sample then undergoes 15-50 thermal cycles under conditions to drive each PCR reaction in each droplet to end-point. The droplets are then analyzed by using a two-color detection scheme to detect the emission of the FAM and VIC dyes. The number of targets counted for Ch21 is determined by identifying the fraction of positive and negative droplets for FAM fluorescence. Similarly, the number of targets counted for the reference sample (Ch1) is determined by identifying the fraction of positive and negative droplets for VIC fluorescence. The number of positive and negative droplets are then used as input in a Poisson distribution to determine the number of copies per droplet (lambda) for both the target and reference chromosomes. The relative copy number of Ch21 is then determined using equations known in the art, e.g., as described in Dube et al. (2008) Plos ONE 3(8):e2876. doi:10.1371/journal.pone.0002876. The confidence of the estimate is also determined using such equations.

### Example 4. Separation of positive and negative droplet signals and sensitivity of ddPCR to Template Copy number in MIP reaction.

### Circularization Reactions

Multiplexed MIP circularization products were generated using either 3-plex or 12-plex probe pools containing 100 attomoles (amol) of each MIP species in the multiplex per 10 µL annealing mixture. One attomole is equivalent to 10⁻¹⁸ mole. 100 amol equals approximately ∼60M copies of each MIP probe sequence. The volume of the annealing reactions was 20 ul.

(Note that in the current experiment, all volumes cited in this protocol were doubled, beginning with a 20ul annealing reaction; however, all DNA, buffer and enzyme concentrations were maintained the same as in the standard 10ul annealing reaction protocol). The probe pools were formulated from mass-dilutions of selected MIP probes (the IDT Ultramers, purified by PAGE) from among either the Chromosome 1 Reference set of 24 nucleic acids (SEQ ID NOS: 1-24); detected by SEQ ID NO: 81, or from the Chromosome 21 Test set of 24 nucleic acids (SEQ ID NOS: 25-48); detected by the SEQ ID NO: 82.

MIP probes were combined with varying numbers of copies of Raji human gDNA (0; 100; 1,000; or 10,000 copies, 3pg gDNA/copy) in 1X Ampligase buffer in 96-well PCR plates, denatured for 5 minutes at 95 °C in a thermocycler (Eppendorf Mastercycler Pro.S or ABI 9700), then cooled to 58 °C and allowed to incubate and anneal at this temperature for >12h.

After annealing, while remaining in the thermocycler at 58 °C, 0.75U of Ampligase was added to each reaction in 5 µL of 1X Ampligase buffer with mixing to provide mixtures with a total volume of 15 µL, and the plates were resealed and allowed to incubate for 15 additional minutes at 58 °C.

### Digestion of Uncircularized Materials

Immediately following the circularization reaction, the temperature of the thermocycler was ramped down to 4 °C, and exonuclease digestion of uncircularized excess MIP probes and gDNA was carried out by adding to each reaction well a 5 µL mixture of 6U Exo I & 30U Exo III in 1X Exo III buffer (EpiCentre) with mixing and plate resealing (total reaction volume = 20 µL). Digestion proceeded for 20 minutes at 37 °C on the thermocycler, followed by heat denaturation at 95 °C for 10 minutes.

MIP reaction products were analyzed by qPCR (4 µL of circularization reaction mixture per 20 µL qPCR reaction) and subsequently frozen at -20 °C and stored for use in droplet digital PCR (ddPCR) experiments.

### Preparation of a general 2X stock solution

The general stock solution (10 mL) was formulated as follows.

| Component | Volume per 10 µL aliquot (µL) | Volume per 10 mL solution (µL) |
|---|---|---|
| FastStart Taq polymerase (Roche) (5U/µL) | 0.16 | 160 |
| 10X Buffer | 2 | 2000 |
| 10 mM dNTP / 20 mM dUTP | 0.4 | 400 |
| Glycerol (50% w/v) | 3.2 | 3200 |
| BSA (20 mg/mL) | 1 | 1000 |
| Pluronic® 10% | 1 | 1000 |
| Water | 2.24 | 2240 |
| Total Volume | 10.0 | 10,000 |

The general stock solution was stored at 4 °C, and was used for multiple experiments.

### Preparation of 2x Hb pr1 ddPCR stock solution

The 2x Hb_pr1 ddPCR stock solution (520.5 µL) was formulated as follows.

| Component | Volume per 52.05 µL aliquot (µL) | Volume per 520.5 µL solution (µL) |
|---|---|---|
| General stock solution | 50 | 500 |
| Primer Hb_Fwd (100 µM) | 0.9 | 9 |
| CCGAATAGGAACGTTGAGCCGT (SEQ ID NO: 79) | | |
| Primer Hb_Rev (100 µM) | 0.9 | 9 |
| GCAAATGTTATCGAGGTCCGGC (SEQ ID NO: 80) | | |
| Taqman Hb_pr1 (FAM-BHQ) (100 µM) | 0.25 | 2.5 |
| ttggcagcctttgccgcggc | | |
| (SEQ ID NO: 81) | | |
| Total Volume | 52.05 | 520.5 |

### Preparation of 1.25x Hb pr1 ddPCR stock solution

The 1.25x HB_PR1 ddPCR stock solution (800 µL) was formulated as follows.

| Component | Volume per 800 µL solution (µL) |
|---|---|
| 2x Hb_pr1 ddPCR stock solution | 520.5 |
| Aqueous MgCl₂ (25 mM) | 80 |
| Water | 199.5 |
| Total Volume | 800 |

The 1.25x Hb_pr1 ddPCR stock solution was partitioned among 4 centrifuge tubes (1.5 mL capacity) in 160 µL aliquots.

### Preparation of 2x Hb pr2 ddPCR stock solution

The 2x Hb_pr2 ddPCR stock solution (936.9 µL) was formulated as follows.

| Component | Volume per 52.05 µL aliquot (µL) | Volume per 936.9 µL solution (µL) |
|---|---|---|
| General stock solution | 50 | 900 |
| Primer Hb_Fwd (100 µM) | 0.9 | 16.2 |
| CCGAATAGGAACGTTGAGCCGT (SEQ ID NO: 79) | | |
| Primer Hb_Rev (100 µM) | 0.9 | 16.2 |
| GCAAATGTTATCGAGGTCCGGC (SEQ ID NO: 80) | | |
| Taqman Hb_pr2(FAM-BHQ) (100 µM) | 0.25 | 4.5 |
| tctgccacctaagcggccgcag (SEQ ID NO: 82) | | |
| Total Volume | 52.05 | 936.9 |

### Preparation of 1.25x Hb pr2 ddPCR stock solution

The 1.25x Hb_pr2 ddPCR stock solution (1440 µL) was formulated as follows.

| Component | Volume per 1440 µL solution (µL) |
|---|---|
| 2x Hb2 ddPCR stock solution | 936.9 |
| Aqueous MgCl₂ (25 mM) | 144 |
| Water | 359.1 |
| Total Volume | 1440 |

The 1.25x Hb_pr2 ddPCR stock solution was partitioned among 8 centrifuge tubes (1.5 mL capacity) in 160 µL aliquots.

### ddPCR Procedure

The products of the MIP circularization experiments were thawed and centrifuged (2,000 rpm for 2 min). 40 µL aliquots of MIP products, i.e. 2x 20 µL aliquots from duplicate assay reactions, were combined with 160 µL of either 1.25x Hb_pr1 ddPCR stock solution for MIPs designed to contain the Taqman Assay Hb_pr1, or 1.25x Hb2 ddPCR stock solution for MIPs designed to contain the Taqman Assay Hb_pr2. The reaction mixtures were partitioned into 1 nL droplets using a ChipShop droplet generation system with a syringe pump system.

Droplet samples were transferred to thermocycler plates (3 x 30 µL aliquots per droplet sample), sealed with a foil seal, then thermocycled for about 1.25 h. Thermocycling began by holding the plates at 94 °C for 10 minutes, subsequently cycling the plates through 35 or 40 cycles of (94 °C, 20 s / 65 °C, 60s), and finally cooling and holding the plates at 4 °C. Thermocycled plates were stored at that temperature.

Leftover droplet aliquots were visualized under a Nikon light microscope to assess uniformity and proper size.

Thermocycled samples were placed on a QuantaLife Box 2 Alpha detector system, where droplet samples were automatically withdrawn from one well at-a-time, and passed single-file by a detector, which was used to assess both droplet size and fluorescence intensity from reacted FAM Taqman probes.

Droplets in each well of the appropriate size were scored as either positive or negative droplets, depending upon their fluorescence amplitude, and these distributions were used to compute the concentration of the assayed sample target according to Poisson statistics.

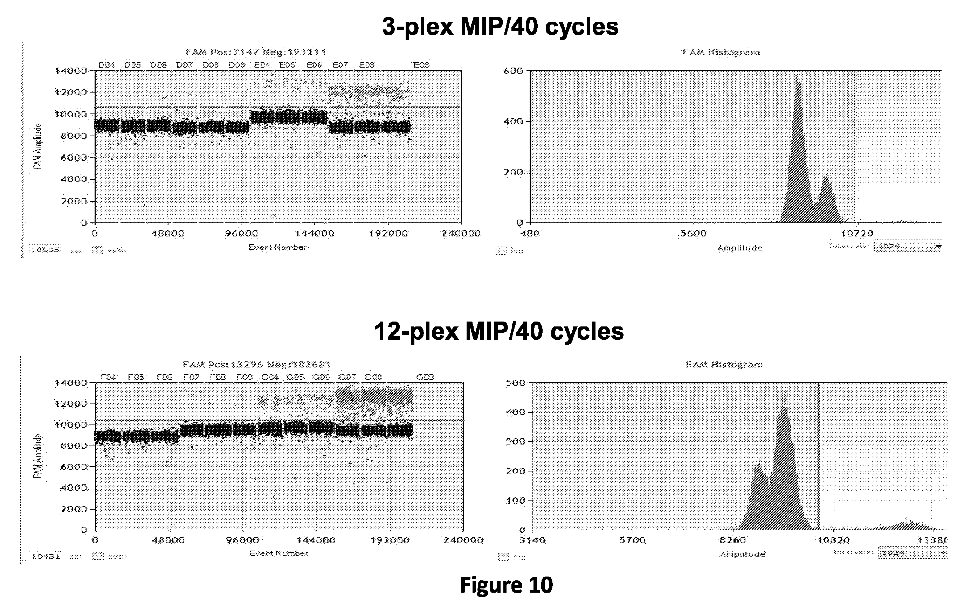

The upper panel of Fig. 10 shows that increasing numbers of positive droplets (or counts) is correlated with increasing input copies of template DNA. Here, Raji genomic DNA was used (derived from Raji cancer cells) for the experiments. For these experiments, 0 copies (or no template control "NTC") of input copies of DNA were used in the sample as indicated in the first three column (D4-6 in upper panel, F4-6 in lower panel); 100 copies in the next set of three (D7-9 upper , F7-9 lower); 1000 copies in the next set of three columns (E4-6 upper, G4-6 lower); and for the last three, 10,000 copies were used (E7-E9 upper, and G7-G9 lower). In the upper panel, all MIP reactions were carried out with a MIP three-plex, using three different MIP probes, each directed to a different site on the test chromosome (which is Chromosome 21, also corresponding to hb_pr2). For the upper panel, the horizontal line at 10605 RFUs (relative fluorescent units) on the left panel, and the vertical line at 10605 RFUs on the right panel demarcate the threshold between positive and negative droplets. Experiments as shown are conducted in triplicate.

The lower panel is the identical experiment conducted with a larger set of MIP probes. In the lower panel, a MIP 12-plex was used, wherein each of 12 MIP probes is directed to a different region within chromosome 21. As depicted in Fig. 10, the lower panel exhibits a roughly 4-fold greater number of positive droplets at a given input number (e.g., NTC, 100, 1000, 10000) of DNA template. The y-axis shows the relative fluorescent units for the FAM signal (or Taqman probe) emitted from each droplet. For both upper and lower panels, the right panel provides a frequency histogram showing the varying fluorescence amplitudes of the droplets and combines data from all 12 lanes presented in the left panel. Here, the X-axis provides the relative fluorescent units for the FAM signal. For the lower panel, the horizontal line at 10,431 RFUs (relative fluorescent units) on the left panel, and the vertical line at 10,431 RFUs on the right panel demarcate the threshold between positive and negative droplets.

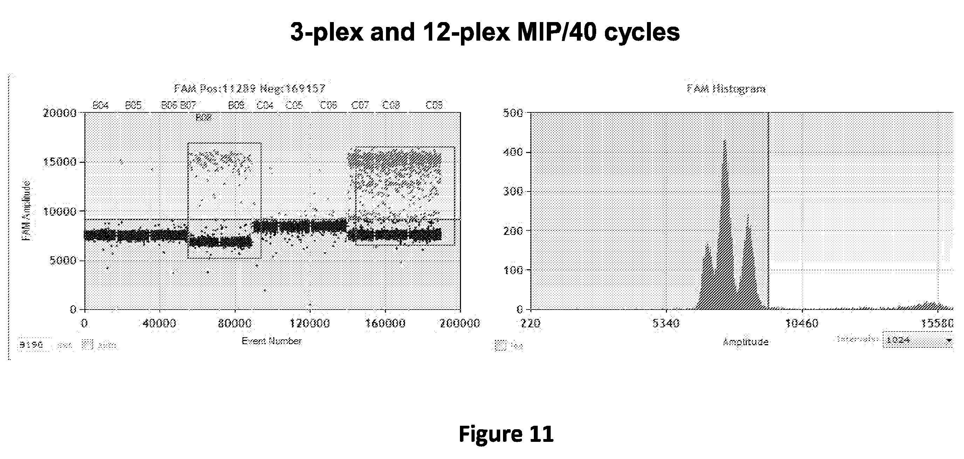

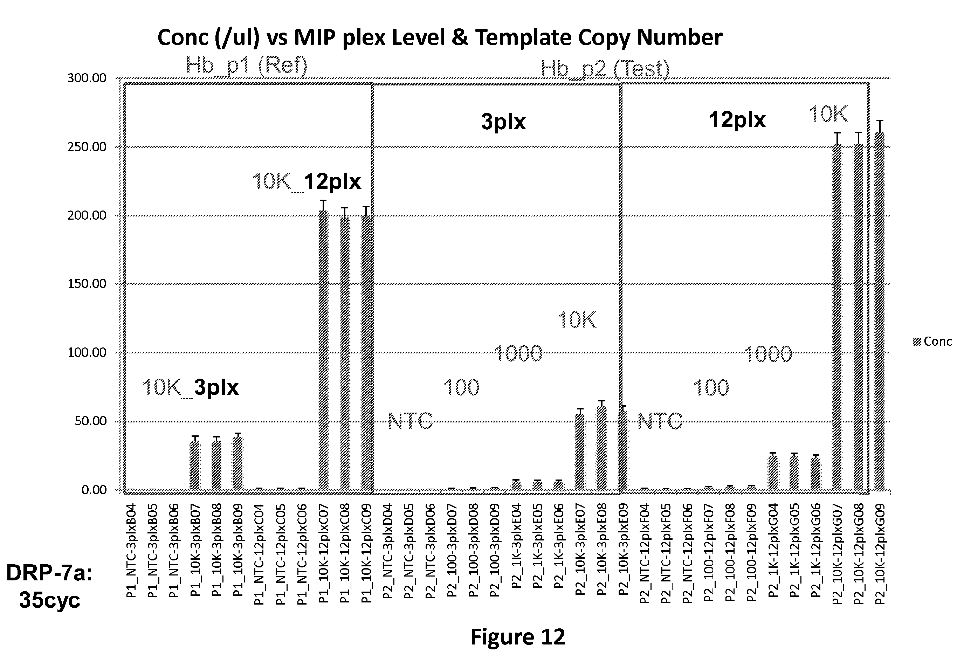

Fig. 11 shows that results similar to those shown in Fig. 10 are obtained when MIP probe pools are derived from probes to the reference polynucleotide (hb_pr1, or chromosome 1). The boxed counts on the left side of the panel reflect the number of counts obtained by using a three-plex MIP probe pool with 10,000 copies of template DNA, while the boxed counts on the right side of the graph reflect the number of counts obtained by using a 12-plex MIP probe pool with 10,000 copies of template DNA. Experiments as shown are conducted in triplicate. Fig. 12 illustrates that the hybridization efficiency is similar whether a thousand copies or 10,000 copies of template are present in the reaction, as shown by the 10-fold increase in counts when going from 1,000 to 10,000 copies of template. Fig. 12 also shows that for a given number of copies of genomic DNA, the number of counts can be increased by increasing the degree of multiplexing of the MIP probes. MIP probes enable multiplexing across a given chromosome, providing a large number of counts from a small number of genomic equivalents, that is important for differentiation of small copy number changes between a target and reference. Experiments as shown are conducted in triplicate.

## Claims

1. A method comprising:
a. binding a first ligation probe to a first target polynucleotide;
b. binding a second ligation probe to a second target polynucleotide;
c. subjecting each of said first and second ligation probes to a ligation reaction to obtain one or more ligated products, wherein said ligation reaction joins a 5' terminus of the first ligation probe to a 3' terminus of the first ligation probe and joins a 5' terminus of the second ligation probe to a 3' terminus of the second ligation probe;
d. partitioning said one or more ligated products into two or more partitions;
e. amplifying a region within said one or more ligated products to obtain amplified products;
f. determining a number of said partitions that contain said amplified products; and
g. calculating a relative copy number of said first and second target polynucleotide based on said number of said partitions.

2. The method of claim 1, wherein said amplification process occurs within said two or more partitions.

3. The method of claim 1, wherein said determining of step (f) comprises measuring a detectable signal from said partitions.

4. The method of claim 1, wherein said first ligation probe is designed to bind to a polynucleotide sequence that is conserved between individuals within a species.

5. The method of claim 1, wherein said two or more partitions are aqueous droplets present within a mixture of at least two immiscible fluids.

6. The method of claim 5, wherein a continuous oil phase comprises said aqueous droplets.

7. The method of claim 1, further comprising binding at least four ligation probes to said first target polynucleotide and at least four ligation probes to said second target polynucleotide.

8. The method of claim 1, wherein said first ligation probe is designed to bind to a first region within said first target polynucleotide and said second ligation probe is designed to bind to a second region within said first target polynucleotide, wherein said first and second regions do not have identical sequences.

9. The method of claim 1, wherein said first target polynucleotide is not identical to said second target polynucleotide.

10. The method of claim 9, wherein said first target polynucleotide is a test chromosome and said second target polynucleotide is a reference chromosome.

11. The method of claim 10, wherein said test chromosome is selected from the group consisting of: chromosome 21, chromosome 13, chromosome 18, and the X chromosome.

12. The method of claim 1, wherein said first target polynucleotide and said second target polynucleotide are located on the same molecule.

13. The method of claim 12, wherein said first target polynucleotide and said second target polynucleotide are located on the same chromosome.

## Patentansprüche

1. Verfahren, umfassend:
a. Binden einer ersten Ligationssonde an ein erstes Ziel-Polynukleotid;
b. Binden einer zweiten Ligationssonde an ein zweites Ziel-Polynukleotid;
c. Unterziehen der jeweils ersten und zweiten Ligationssonde einer Ligationsreaktion, um ein oder mehrere ligierte Produkte zu erhalten, wobei durch die Ligationsreaktion ein 5'-Terminus der ersten Ligationssonde mit einem 3'-Terminus der ersten Ligationssonde und ein 5'-Terminus der zweiten Ligationssonde mit einem 3'-Terminus der zweiten Ligationssonde verbunden wird;
d. Teilen von einem oder mehreren ligierten Produkten in zwei oder mehrere Teilstücke;
e. Amplifizieren einer Region in einem oder in mehreren ligierten Produkten, um amplifizierte Produkte zu erhalten;
f. Bestimmen einer Anzahl der Teilstücke, welche die amplifizierten Produkte enthalten; und
g. Berechnen einer relativen Kopienzahl des ersten und zweiten Ziel-Polynukleotids, basierend auf der Anzahl der Teilstücke.

2. Verfahren nach Anspruch 1, wobei der Amplifikationsprozess in den zwei oder in mehreren Teilstücken erfolgt.

3. Verfahren nach Anspruch 1, wobei das Bestimmen von Schritt (f) das Messen eines detektierbaren Signals von den Teilstücken umfasst.

4. Verfahren nach Anspruch 1, wobei die erste Ligationssonde konzipiert ist, um an eine Polynukleotidsequenz zu binden, die zwischen Individuen innerhalb einer Spezies konserviert ist.

5. Verfahren nach Anspruch 1, wobei zwei oder mehrere Teilstücke wässrige Tröpfchen sind, die innerhalb einer Mischung aus mindestens zwei nicht mischbaren Fluids vorliegen.

6. Verfahren nach Anspruch 5, wobei eine kontinuierliche ölige Phase die wässrigen Tröpfchen umfasst.

7. Verfahren nach Anspruch 1, ferner umfassend das Binden von mindestens vier Ligationssonden an das erste Ziel-Polynukleotid und von mindestens vier Ligationssonden an das zweite Ziel-Polynukleotid.

8. Verfahren nach Anspruch 1, wobei die erste Ligationssonde konzipiert ist, um an eine erste Region innerhalb des ersten Ziel-Polynukleotids zu binden, und die zweite Ligationssonde konzipiert ist, um an eine zweite Region innerhalb des ersten Ziel-Polynukleotids zu binden, wobei die erste und zweite Region keine identischen Sequenzen aufweisen.

9. Verfahren nach Anspruch 1, wobei das erste Ziel-Polynukleotid nicht mit dem zweiten Ziel-Polynukleotid identisch ist.

10. Verfahren nach Anspruch 9, wobei das erste Ziel-Polynukleotid ein Testchromosom ist und das zweite Ziel-Polynukleotid ein Referenzchromosom ist.

11. Verfahren nach Anspruch 10, wobei das Testchromosom aus der Gruppe ausgewählt ist, bestehend aus: Chromosom 21, Chromosom 13, Chromosom 18 und dem X-Chromosom.

12. Verfahren nach Anspruch 1, wobei das erste Ziel-Polynukleotid und das zweite Ziel-Polynukleotid an dem gleichen Molekül lokalisiert sind.

13. Verfahren nach Anspruch 12, wobei das erste Ziel-Polynukleotid und das zweite Ziel-Polynukleotid an dem gleichen Chromosom lokalisiert sind.

## Revendications

1. Procédé comprenant :
a. la liaison d'une première sonde de ligature à un premier polynucléotide cible ;
b. la liaison d'une deuxième sonde de ligature à un deuxième polynucléotide cible ;
c. la soumission de chacune desdites première et deuxième sondes de ligature à une réaction de ligature pour obtenir un ou plusieurs produits ligaturés, dans lequel ladite réaction de ligature joint une extrémité 5' de la première sonde de ligature à une extrémité 3' de la première sonde de ligature et joint une extrémité 5' de la deuxième sonde de ligature à une extrémité 3' de la deuxième sonde de ligature ;
d. le partitionnement dudit ou desdits produit(s) ligaturé(s) en deux partitions ou plus ;
e. l'amplification d'une région au sein dudit ou desdits produit(s) ligaturé(s) pour obtenir des produits amplifiés ;
f. la détermination d'un nombre desdites partitions qui contiennent lesdits produits amplifiés ; et
g. le calcul d'un nombre relatif de copies desdits premier et deuxième polynucléotides cibles sur la base dudit nombre desdites partitions.

2. Procédé selon la revendication 1, dans lequel ledit procédé d'amplification a lieu au sein desdites deux partitions ou plus.

3. Procédé selon la revendication 1, dans lequel ladite détermination de l'étape (f) comprend la mesure d'un signal détectable à partir desdites partitions.

4. Procédé selon la revendication 1, dans lequel ladite première sonde de ligature est conçue pour se lier à une séquence polynucléotidique qui est conservée entre les individus au sein d'une espèce.

5. Procédé selon la revendication 1, dans lequel lesdites deux partitions ou plus sont des gouttelettes aqueuses présentes dans un mélange d'au moins deux fluides non miscibles.

6. Procédé selon la revendication 5, dans lequel une phase huileuse continue comprend lesdites gouttelettes aqueuses.

7. Procédé selon la revendication 1, comprenant en outre la liaison d'au moins quatre sondes de ligature audit premier polynucléotide cible et au moins quatre sondes de ligature audit deuxième polynucléotide cible.

8. Procédé selon la revendication 1, dans lequel ladite première sonde de ligature est conçue pour se lier à une première région au sein dudit premier polynucléotide cible et ladite sonde de ligature est conçue pour se lier à une deuxième région au sein dudit premier polynucléotide cible, dans lequel lesdites première et deuxième régions n'ont pas de séquences identiques.

9. Procédé selon la revendication 1, dans lequel ledit premier polynucléotide cible n'est pas identique audit deuxième polynucléotide cible.

10. Procédé selon la revendication 9, dans lequel ledit premier polynucléotide cible est un chromosome d'essai et ledit deuxième polynucléotide cible est un chromosome de référence.

11. Procédé selon la revendication 10, dans lequel ledit chromosome d'essai est choisi dans le groupe constitué par: le chromosome 21, le chromosome 13, le chromosome 18 et le chromosome X.

12. Procédé selon la revendication 1, dans lequel ledit premier polynucléotide cible et ledit deuxième polynucléotide cible sont situés sur la même molécule.

13. Procédé selon la revendication 12, dans lequel ledit premier polynucléotide cible et ledit deuxième polynucléotide cible sont situés sur le même chromosome.
